# EUROPEAN PATENT APPLICATION

(11) **EP 1 586 660 A1**
(43) Date of publication of application: **19.10.2005**
(21) Application number: 05010573.3
(22) Date of filing: 17.04.1998
(51) Int. Cl.: C12Q 1/68

(54) **Novel molecules of the T85-related protein family and uses thereof**

(30) Priority: 18.04.1997 US 44746 P; 10.10.1997 US 62017 P
(62) Divisional of application: 98918329.8
(71) Applicant: MILLENNIUM PHARMACEUTICALS, INC., Cambridge, MA 02139 (US)
(72) Inventor: McCarthy, Sean A., Boston, MA02114 (US); Holtzmann, Douglas, Sherborn, MA 01770 (US)
(74) Representative: Lock, Graham James

(57) **Abstract**

Nucleotide and amino acid sequence of the T85-related protein family are useful in the preparation of medicaments for the treatment of a disorder characterised by aberrant T85 protein or nucleic acid expression or activity. They are also useful in diagnostic assays for identifying the presence or absence of a genetic lesion or mutation characterised by at least one of: aberrant modification or mutation of a gene encoding a T85 protein, mis-regulation of a gene encoding a T85 protein, and aberrant post-translational modification of a T85 protein, wherein a wild-type form of the gene encodes a protein with a T85 activity.

### Annex to the application documents-subsequently filed sequences listing

SEQUENCE LISTING

(1) GENERAL INFORMATION

(i) APPLICANT: McCarthy, Sean A.
Holtzman, Douglas

(ii) TITLE OF THE INVENTION: NOVEL MOLECULES OF THE FTHMA-070-
RELATED PROTEIN FAMILY AND THE T85-RELATED PROTEIN
FAMILY AND USES THEREOF

(iii) NUMBER OF SEQUENCES: 18

(iv) CORRESPONDENCE ADDRESS:
(A) ADDRESSEE: Fish & Richardson P.C.
(B) STREET: 225 Franklin Street
(C) CITY: Boston
(D) STATE: MA
(E) COUNTRY: USA
(F) ZIP: 02110-2804

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: 09/062,389
(B) FILING DATE: 17-APR-1998

(vii) PRIOR APPLICATION DATA:
(A) APPLICATION NUMBER: 60/062,017
(B) FILING DATE: 10-OCT-1997

(A) APPLICATION NUMBER: 60/044,746
(B) FILING DATE: 18-APR-1997

(viii) ATTORNEY/AGENT INFORMATION:
(A) NAME: Meiklejohn, Anita L.
(B) REGISTRATION NUMBER: 35,283
(C) REFERENCE/DOCKET NUMBER: 09404/051001

(ix) TELECOMMUNICATION INFORMATION:
(A) TELEPHONE: 617/542-5070
(B) TELEFAX: 617/542-8906
(C) TELEX: 200154

(2) INFORMATION FOR SEQ ID NO:1:

(i) SEQUENCE CHARACTERISTICS:
(A) LENGTH: 2133 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: Coding Sequence
(B) LOCATION: 73...1275

(xi) SEQUENCE DESCRIPTION: SEQ ID NO:1:

GTCGACCCAC GCGTCCGCCG GAGCGCTCGC CCAGCCGCCG CCTCCAAGCC CCTGAGGTTT 60
CCGGGGACCA CA ATG AAC AAG TTG CTG TGC TGC GCG CTC GTG TTT CTG GAC 111
Met Asn Lys Leu Leu Cys Cys Ala Leu Val Phe Leu Asp
1               5                   10

ATC TCC ATT AAG TGG ACC ACC CAG GAA ACG TTT CCT CCA AAG TAC CTT 159
Ile Ser Ile Lys Trp Thr Thr Gln Glu Thr Phe Pro Pro Lys Tyr Leu
    15                  20                  25

CAT TAT GAC GAA GAA ACT TCT CAT CAG CTG TTG TGT GAC AAA TGT CCT 207
His Tyr Asp Glu Glu Thr Ser His Gln Leu Leu Cys Asp Lys Cys Pro
30                  35                  40                  45

CCT GGT ACC TAC CTA AAA CAA CAC TGT ACA GCA AAG TGG AAG ACC GTG 255
Pro Gly Thr Tyr Leu Lys Gln His Cys Thr Ala Lys Trp Lys Thr Val
                50                  55                  60

TGC GCC CCT TGC CCT GAC CAC TAC TAC ACA GAC AGC TGG CAC ACC AGT 303
Cys Ala Pro Cys Pro Asp His Tyr Tyr Thr Asp Ser Trp His Thr Ser
            65                  70                  75

GAC GAG TGT CTA TAC TGC AGC CCC GTG TGC AAG GAG CTG CAG TAC GTC 351
Asp Glu Cys Leu Tyr Cys Ser Pro Val Cys Lys Glu Leu Gln Tyr Val
        80                  85                  90

AAG CAG GAG TGC AAT CGC ACC CAC AAC CGC GTG TGC GAA TGC AAG GAA 399
Lys Gln Glu Cys Asn Arg Thr His Asn Arg Val Cys Glu Cys Lys Glu
    95                  100                 105

GGG CGC TAC CTT GAG ATA GAG TTC TGC TTG AAA CAT AGG AGC TGC CCT 447
Gly Arg Tyr Leu Glu Ile Glu Phe Cys Leu Lys His Arg Ser Cys Pro
110                 115                 120                 125

CCT GGA TTT GGA GTG GTG CAA GCT GGA ACC CCA GAG CGA AAT ACA GTT 495
Pro Gly Phe Gly Val Val Gln Ala Gly Thr Pro Glu Arg Asn Thr Val
                130                 135                 140

TGC AAA AGA TGT CCA GAT GGG TTC TTC TCA AAT GAG ACG TCA TCT AAA 543
Cys Lys Arg Cys Pro Asp Gly Phe Phe Ser Asn Glu Thr Ser Ser Lys
            145                 150                 155

GCA CCC TGT AGA AAA CAC ACA AAT TGC AGT GTC TTT GGT CTC CTG CTA 591
Ala Pro Cys Arg Lys His Thr Asn Cys Ser Val Phe Gly Leu Leu Leu
        160                 165                 170

ACT CAG AAA GGA AAT GCA ACA CAC GAC AAC ATA TGT TCC GGA AAC AGT 639
Thr Gln Lys Gly Asn Ala Thr His Asp Asn Ile Cys Ser Gly Asn Ser
    175                 180                 185

GAA TCA ACT CAA AAA TGT GGA ATA GAT GTT ACC CTG TGT GAG GAG GCA 687
Glu Ser Thr Gln Lys Cys Gly Ile Asp Val Thr Leu Cys Glu Glu Ala
190                 195                 200                 205

TTC TTC AGG TTT GCT GTT CCT ACA AAG TTT ACG CCT AAC TGG CTT AGT 735
Phe Phe Arg Phe Ala Val Pro Thr Lys Phe Thr Pro Asn Trp Leu Ser
                210                 215                 220

GTC TTG GTA GAC AAT TTG CCT GGC ACC AAA GTA AAC GCA GAG AGT GTA 783
Val Leu Val Asp Asn Leu Pro Gly Thr Lys Val Asn Ala Glu Ser Val
            225                 230                 235

GAG AGG ATA AAA CGG CAA CAC AGC TCA CAA GAA CAG ACT TTC CAG CTG 831
Glu Arg Ile Lys Arg Gln His Ser Ser Gln Glu Gln Thr Phe Gln Leu
        240                 245                 250

CTG AAG TTA TGG AAA CAT CAA AAC AAA GAC CAA GAT ATA GTC AAG AAG 879
Leu Lys Leu Trp Lys His Gln Asn Lys Asp Gln Asp Ile Val Lys Lys
    255                 260                 265

ATC ATC CAA GAT ATT GAC CTC TGT GAA AAC AGC GTG CAG CGG CAC ATT 927
Ile Ile Gln Asp Ile Asp Leu Cys Glu Asn Ser Val Gln Arg His Ile
270                 275                 280                 285

GGA CAT GCT AAC CTC ACC TTC GAG CAG CTT CGT AGC TTG ATG GAA AGC 975
Gly His Ala Asn Leu Thr Phe Glu Gln Leu Arg Ser Leu Met Glu Ser
                290                 295                 300

TTA CCG GGA AAG AAA GTG GGA GCA GAA GAC ATT GAA AAA ACA ATA AAG 1023
Leu Pro Gly Lys Lys Val Gly Ala Glu Asp Ile Glu Lys Thr Ile Lys
            305                 310                 315

GCA TGC AAA CCC AGT GAC CAG ATC CTG AAG CTG CTC AGT TTG TGG CGA 1071
Ala Cys Lys Pro Ser Asp Gln Ile Leu Lys Leu Leu Ser Leu Trp Arg
        320                 325                 330

ATA AAA AAT GGC GAC CAA GAC ACC TTG AAG GGC CTA ATG CAC GCA CTA 1119
Ile Lys Asn Gly Asp Gln Asp Thr Leu Lys Gly Leu Met His Ala Leu
    335                 340                 345

AAG CAC TCA AAG ACG TAC CAC TTT CCC AAA ACT GTC ACT CAG AGT CTA 1167
Lys His Ser Lys Thr Tyr His Phe Pro Lys Thr Val Thr Gln Ser Leu
350                 355                 360                 365

AAG AAG ACC ATC AGG TTC CTT CAC AGC TTC ACA ATG TAC AAA TTG TAT 1215
Lys Lys Thr Ile Arg Phe Leu His Ser Phe Thr Met Tyr Lys Leu Tyr
                370                 375                 380

CAG AAG TTA TTT TTA GAA ATG ATA GGT AAC CAG GTC CAA TCA GTA AAA 1263
Gln Lys Leu Phe Leu Glu Met Ile Gly Asn Gln Val Gln Ser Val Lys
            385                 390                 395

ATA AGC TGC TTA TAACTGGAAA TGGCCATTGA GCTGTTTCCT CACAATTGGC GAGAT 1320
Ile Ser Cys Leu
        400

CCCCATGGAT GAGTAAACTG TTTCTCAGGC ACTTGAGGCT TTCAGTGATA TCTTTCTCAT 1380

TACCAGTGAC TAATTTTGCC ACAGGGTACT AAAAGAAACT ATGATGTGGA GAAAGGACTA 1440
ACATCTCCTC CAATAAACCC CAAATGGTTA ATCCAACTGT CAGATCTGGA TCGTTATCTA 1500
CTGACTATAT TTTCCCTTAT TACTGCTTGC AGTAATTCAA CTGGAAATTA AAAAAAAAAA 1560
ACTAGACTCC ATTGTGCCTT ACTAAATATG GGAATGTCTA ACTTAAATAG CTTTGAGATT 1620
TCAGCTAYGC TAGAGGCTTT TATTAGAAAG CCATATTTTT TTCTGTAAAA GTTACTAATA 1680
TATCTGTAAC ACTATTACAG TATTGCTATT TATATTCATT CAGATATAAG ATTTGTACAT 1740
ATTATCATCC TATAAAGAAA CGGTATGACT TAATTTTAGA AAGAAAATTA TATTCTGTTT 1800
ATTATGACAA ATGAAAGAGA AAATATATAT TTTTAATGGA AAGTTTGTAG CATTTTTCTA 1860
ATAGGTACTG CCATATTTTT CTGTGTGGAG TATTTTTATA ATTTTATCTG TATAAGCTGT 1920
AATATCATTT TATAGAAAAT GCATTATTTA GTCAATTGTT TAATGTTGGA AAACATATGA 1980
AATATAAATT ATCTGAATAT TAGATGCTCT GAGAAATTGA ATGTACCTTA TTTAAAAGAT 2040
TTTATGGTTT TATAACTATA TAAATGACAT TATTAAAGTT TTCAAATTAT TTTTAAAAAA 2100
AAAAAAAAAA AAAAAAAAAA AAAGGGCGGC CGC 2133

(2) INFORMATION FOR SEQ ID NO:2:

(i) SEQUENCE CHARACTERISTICS:
(A) LENGTH: 401 amino acids
(B) TYPE: amino acid
(D) TOPOLOGY: linear

(ii) MOLECULE TYPE: protein

(v) FRAGMENT TYPE: internal

(xi) SEQUENCE DESCRIPTION: SEQ ID NO:2:

Met Asn Lys Leu Leu Cys Cys Ala Leu Val Phe Leu Asp Ile Ser Ile
1               5                   10                  15
Lys Trp Thr Thr Gln Glu Thr Phe Pro Pro Lys Tyr Leu His Tyr Asp
            20                  25                  30
Glu Glu Thr Ser His Gln Leu Leu Cys Asp Lys Cys Pro Pro Gly Thr
        35                  40                  45
Tyr Leu Lys Gln His Cys Thr Ala Lys Trp Lys Thr Val Cys Ala Pro
    50                  55                  60
Cys Pro Asp His Tyr Tyr Thr Asp Ser Trp His Thr Ser Asp Glu Cys
65                  70                  75                  80
Leu Tyr Cys Ser Pro Val Cys Lys Glu Leu Gln Tyr Val Lys Gln Glu
                85                  90                  95
Cys Asn Arg Thr His Asn Arg Val Cys Glu Cys Lys Glu Gly Arg Tyr
            100                 105                 110
Leu Glu Ile Glu Phe Cys Leu Lys His Arg Ser Cys Pro Pro Gly Phe
        115                 120                 125
Gly Val Val Gln Ala Gly Thr Pro Glu Arg Asn Thr Val Cys Lys Arg
    130                 135                 140
Cys Pro Asp Gly Phe Phe Ser Asn Glu Thr Ser Ser Lys Ala Pro Cys
145                 150                 155                 160
Arg Lys His Thr Asn Cys Ser Val Phe Gly Leu Leu Leu Thr Gln Lys
                165                 170                 175
Gly Asn Ala Thr His Asp Asn Ile Cys Ser Gly Asn Ser Glu Ser Thr
            180                 185                 190
Gln Lys Cys Gly Ile Asp Val Thr Leu Cys Glu Glu Ala Phe Phe Arg
        195                 200                 205
Phe Ala Val Pro Thr Lys Phe Thr Pro Asn Trp Leu Ser Val Leu Val
    210                 215                 220
Asp Asn Leu Pro Gly Thr Lys Val Asn Ala Glu Ser Val Glu Arg Ile
225                 230                 235                 240

Lys Arg Gln His Ser Ser Gln Glu Gln Thr Phe Gln Leu Leu Lys Leu
                245                 250                 255
Trp Lys His Gln Asn Lys Asp Gln Asp Ile Val Lys Lys Ile Ile Gln
            260                 265                 270
Asp Ile Asp Leu Cys Glu Asn Ser Val Gln Arg His Ile Gly His Ala
        275                 280                 285
Asn Leu Thr Phe Glu Gln Leu Arg Ser Leu Met Glu Ser Leu Pro Gly
    290                 295                 300
Lys Lys Val Gly Ala Glu Asp Ile Glu Lys Thr Ile Lys Ala Cys Lys
305                 310                 315                 320
Pro Ser Asp Gln Ile Leu Lys Leu Leu Ser Leu Trp Arg Ile Lys Asn
                325                 330                 335
Gly Asp Gln Asp Thr Leu Lys Gly Leu Met His Ala Leu Lys His Ser
            340                 345                 350
Lys Thr Tyr His Phe Pro Lys Thr Val Thr Gln Ser Leu Lys Lys Thr
        355                 360                 365
Ile Arg Phe Leu His Ser Phe Thr Met Tyr Lys Leu Tyr Gln Lys Leu
    370                 375                 380
Phe Leu Glu Met Ile Gly Asn Gln Val Gln Ser Val Lys Ile Ser Cys
385                 390                 395                 400
Leu

(2) INFORMATION FOR SEQ ID NO:3:

(i) SEQUENCE CHARACTERISTICS:
(A) LENGTH: 1203 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

ATGAACAAGT TGCTGTGCTG CGCGCTCGTG TTTCTGGACA TCTCCATTAA GTGGACCACC      60
CAGGAAACGT TTCCTCCAAA GTACCTTCAT TATGACGAAG AAACTTCTCA TCAGCTGTTG     120
TGTGACAAAT GTCCTCCTGG TACCTACCTA AAACAACACT GTACAGCAAA GTGGAAGACC     180
GTGTGCGCCC CTTGCCCTGA CCACTACTAC ACAGACAGCT GGCACACCAG TGACGAGTGT     240
CTATACTGCA GCCCCGTGTG CAAGGAGCTG CAGTACGTCA AGCAGGAGTG CAATCGCACC     300
CACAACCGCG TGTGCGAATG CAAGGAAGGG CGCTACCTTG AGATAGAGTT CTGCTTGAAA     360
CATAGGAGCT GCCCTCCTGG ATTTGGAGTG GTGCAAGCTG GAACCCCAGA GCGAAATACA     420
GTTTGCAAAA GATGTCCAGA TGGGTTCTTC TCAAATGAGA CGTCATCTAA AGCACCCTGT     480
AGAAAACACA CAAATTGCAG TGTCTTTGGT CTCCTGCTAA CTCAGAAAGG AAATGCAACA     540
CACGACAACA TATGTTCCGG AAACAGTGAA TCAACTCAAA AATGTGGAAT AGATGTTACC     600
CTGTGTGAGG AGGCATTCTT CAGGTTTGCT GTTCCTACAA AGTTTACGCC TAACTGGCTT     660
AGTGTCTTGG TAGACAATTT GCCTGGCACC AAAGTAAACG CAGAGAGTGT AGAGAGGATA     720
AAACGGCAAC ACAGCTCACA AGAACAGACT TTCCAGCTGC TGAAGTTATG GAAACATCAA     780
AACAAAGACC AAGATATAGT CAAGAAGATC ATCCAAGATA TTGACCTCTG TGAAAACAGC     840
GTGCAGCGGC ACATTGGACA TGCTAACCTC ACCTTCGAGC AGCTTCGTAG CTTGATGGAA     900
AGCTTACCGG GAAAGAAAGT GGGAGCAGAA GACATTGAAA AAACAATAAA GGCATGCAAA     960
CCCAGTGACC AGATCCTGAA GCTGCTCAGT TTGTGGCGAA TAAAAAATGG CGACCAAGAC    1020
ACCTTGAAGG GCCTAATGCA CGCACTAAAG CACTCAAAGA CGTACCACTT TCCCAAAACT    1080
GTCACTCAGA GTCTAAAGAA GACCATCAGG TTCCTTCACA GCTTCACAAT GTACAAATTG    1140
TATCAGAAGT TATTTTTAGA AATGATAGGT AACCAGGTCC AATCAGTAAA AATAAGCTGC    1200
TTA                                                                  1203

(2) INFORMATION FOR SEQ ID NO:4:

(i) SEQUENCE CHARACTERISTICS:
(A) LENGTH: 4291 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: Coding Sequence
(B) LOCATION: 958...3216

(xi) SEQUENCE DESCRIPTION: SEQ ID NO:4:
GTCGACCCAC GCGTCCGAGG AGCTGGGGTG AGCCGCGGCG GCCGTCTCTC CCACCCGCAG 60
CAGCATCCTC TCTGCCCTTC TCTGCCACCC CGGGGAGAGC CGGGAGCTGC CTCTTTACAG 120
CTTCCACGAG CCAGGGGTGC AGGCAGCTGC CCCCAGGAAG TTTGGGCTTC TGCGTAGTTT 180
AGGGGTGCCT GCGAGCGCCC CAGAGGGCGA GGGGCCGAGG GCGATGTTGG GCGCCGCGCG 240
GGGCTGGGGG CGCCCAGAAG ACGTGCGAGT GTCCGCGGTC CTGCTGCTGT CTCCAGTACC 300
CTCCGCATCC CCCAAGTGAT GGGAACAAGG GCCCGCCCAG GCAGCCGCTG TCGCCGCACC 360
GCCCCCTCGC TCGCTCTCTG CGCGCGGAGT CACCCAGTCA CACTCCCGGC ACCCCGAGCC 420
CTTCCTCCGG AGCTGCTGCT TCTACTTTGG CTGCTATCGC CGCCGCCGCG GGTGGCCCGC 480
TGCTGACTGG GCTCGCCGGG AGACGGAGAA GCACTTTTTG GCCCTCCCTC AGCAGCTCTC 540
ACACCCCAAC TTTGCCGCCG CCGCCGCGCC TGCCCTCGCA GCGGCGCTCG GCCGCACATT 600
GTGAGGGCGC ACGCCGGGAG GCTCCGCAAG ACCGTGGAGG CAGGAAACGG CACTACTGCG 660
CTTCTGCCTC GGCTCTTTGT TGTTCGCTTT GGATGGTTCT TGAAAGTGTC TGAGCCTCCT 720
CGGAAATCCT GGGGCCGGAG AAGACAAACC TTGGAATTCT TCCTCTGCAA AAGTCTCTGA 780
GATACTGACA AGCGTCCGGA AAGGTCGACG AGTAATTGCC CTGAAAACTC TTGGCTAATT 840
GACCCACGTT GCTTATATTA AGCCTTTGTG TGTGGTGTGT GGCTTCATAC ATTTGGGGAC 900
CCTATTTCCA CTCCCTCCTC TTGGCATGAG ACTGTATACA GGATCCACCC GAGGACA ATG 960
ATT GCG GAG CCC GCT CAC TTT TAC CTG TTT GGA TTA ATA TGT CTC TGT 1008
TCA GGC TCC CGT CTT CGT CAG GAA GAT TTT CCA CCT CGC ATT GTT GAA 1056
CAC CCT TCA GAC CTG ATT GTC TCA AAA GGA GAA CCT GCA ACT TTG AAC 1104
TGC AAA GCT GAA GGC CGC CCC ACA CCC ACT ATT GAA TGG TAC AAA GGG 1152
GGA GAG AGA GTG GAG ACA GAC AAA GAT GAC CCT CGC TCA CAC CGA ATG 1200
TTG CTG CCG AGT GGA TCT TTA TTT TTC TTA CGT ATA GTA CAT GGA CGG 1248
AAA AGT AGA CCT GAT GAA GGA GTC TAT GTC TGT GTA GCA AGG AAT TAC 1296
CTT GGA GAG GCT GTG AGC CAC AAT GCA TCG CTG GAA GTA GCC ATA CTT 1344
CGG GAT GAC TTC AGA CAA AAC CCT TCG GAT GTC ATG GTT GCA GTA GGA 1392
GAG CCT GCA GTA ATG GAA TGC CAA CCT CCA CGA GGC CAT CCT GAG CCC 1440
ACC ATT TCA TGG AAG AAA GAT GGC TCT CCA CTG GAT GAT AAA GAT GAA 1488
AGA ATA ACT ATA CGA GGA GGA AAG CTC ATG ATC ACT TAC ACC CGT AAA 1536
AGT GAC GCT GGC AAA TAT GTT TGT GTT GGT ACC AAT ATG GTT GGG GAA 1584
CGT GAG AGT GAA GTA GCC GAG CTG ACT GTC TTA GAG AGA CCA TCA TTT 1632
GTG AAG AGA CCC AGT AAC TTG GCA GTA ACT GTG GAT GAC AGT GCA GAA 1680
TTT AAA TGT GAG GCC CGA GGT GAC CCT GTA CCT ACA GTA CGA TGG AGG 1728
AAA GAT GAT GGA GAG CTG CCC AAA TCC AGA TAT GAA ATC CGA GAT GAT 1776
CAT ACC TTG AAA ATT AGG AAG GTG ACA GCT GGT GAC ATG GGT TCA TAC 1824
ACT TGT GTT GCA GAA AAT ATG GTG GGC AAA GCT GAA GCA TCT GCT ACT 1872
CTG ACT GTT CAA GTT GGG TCT GAA CCT CCA CAT TTT GTT GTG AAA CCC 1920
CGT GAC CAG GTT GTT GCT TTG GGA CGG ACT GTA ACT TTT CAG TGT GAA 1968
GCA ACC GGA AAT CCT CAA CCA GCT ATT TTC TGG AGG AGA GAA GGG AGT 2016
CAG AAT CTA CTT TTC TCA TAT CAA CCA CCA CAG TCA TCC AGC CGA TTT 2064
TCA GTC TCC CAG ACT GGC GAC CTC ACA ATT ACT AAT GTC CAG CGA TCT 2112
GAT GTT GGT TAT TAC ATC TGC CAG ACT TTA AAT GTT GCT GGA AGC ATC 2160
ATC ACA AAG GCA TAT TTG GAA GTT ACA GAT GTG ATT GCA GAT CGG CCT 2208
CCC CCA GTT ATT CGA CAA GGT CCT GTG AAT CAG ACT GTA GCC GTG GAT 2256
GGC ACT TTC GTC CTC AGC TGT GTG GCC ACA GGC AGT CCA GTG CCC ACC 2304
ATT CTG TGG AGA AAG GAT GGA GTC CTC GTT TCA ACC CAA GAC TCT CGA 2352
ATC AAA CAG TTG GAG AAT GGA GTA CTG CAG ATC CGA TAT GCT AAG CTG 2400
GGT GAT ACT GGT CGG TAC ACC TGC ATT GCA TCA ACC CCC AGT GGT GAA 2448
GCA ACA TGG AGT GCT TAC ATT GAA GTT CAA GAA TTT GGA GTT CCA GTT 2496
CAG CCT CCA AGA CCT ACT GAC CCA AAT TTA ATC CCT AGT GCC CCA TCA 2544
AAA CCT GAA GTG ACA GAT GTC AGC AGA AAT ACA GTC ACA TTA TCA TGG 2592
CAA CCA AAT TTG AAT TCA GGA GCA ACT CCA ACA TCT TAT ATT ATA GAA 2640
GCC TTC AGC CAT GCA TCT GGT AGC AGC TGG CAG ACC GTA GCA GAG AAT 2688
GTG AAA ACA GAA ACA TCT GCC ATT AAA GGA CTC AAA CCT AAT GCA ATT 2736
TAC CTT TTC CTT GTG AGG GCA GCT AAT GCA TAT GGA ATT AGT GAT CCA 2784
AGC CAA ATA TCA GAT CCA GTG AAA ACA CAA GAT GTC CTA CCA ACA AGT 2832
CAG GGG GTG GAC CAC AAG CAG GTC CAG AGA GAG CTG GGA AAT GCT GTT 2880
CTG CAC CTC CAC AAC CCC ACC GTC CTT TCT TCC TCT TCC ATC GAA GTG 2928
CAC TGG ACA GTA GAT CAA CAG TCT CAG TAT ATA CAA GGA TAT AAA ATT 2976
CTC TAT CGG CCA TCT GGA GCC AAC CAC GGA GAA TCA GAC TGG TTA GTT 3024
TTT GAA GTG AGG ACG CCA GCC AAA AAC AGT GTG GTA ATC CCT GAT CTC 3072
AGA AAG GGA GTC AAC TAT GAA ATT AAG GCT CGC CCT TTT TTT AAT GAA 3120
TTT CAA GGA GCA GAT AGT GAA ATC AAG TTT GCC AAA ACC CTG GAA GAA 3168
GGC AAG TAT GAT GAA GCG TTT GAT TTC CAT GCA TTG CAG TAT ATT TGT T 3217
AGAATTTTAT TAGCTTAATT TAAATTGTTA TAATGTTAAT AGTGGCATTA ATTACTAACT 3277
TTCTTGATAT TGAACATACA TTTTGCTAAC AGTATTTGTT TATTTAGAAA ACTAGAAAAG 3337
CAAAATGCTA TAATAGTTGA CTTGCTTCAA TAGTGGCCTA CCTCAAATGT GTAAATGTGA 3397
ACATTATATT ACTTAATGGC ATATAGAAAT TTAATTATTC AGAACTAAAA TTCACATTTA 3457
AAAATGAAAT TGTTTTCCGT TTGGTTTATT TTCCATTCAG CATTTTCTAT TTGTTTATTT 3517
TGATCCATGA TTTGAATAAT GTGTTGATTT GCCCAGGACT TCGTGGGATT AGCATTTTCT 3577
TCCTAAATCT CATCTCTTTT AAAACACCTC AGTTCTAGAC AAATCACTAT AGCTGACCAC 3637
CCTATTCCTA GAAATTAAAC AATATTTACA TAAAGGTAAA AGATTACATT AACTAACAAC 3697
AGGATTTTTC CCAACATGTC TTTGTCACTT TGGCTGTATA AGTAATTATC ATGAATGATT 3757
AATATTCATT TTCATACTTT TGAACATTTC GGGGGAGAAC ATGAGACTAG AGTTACACGG 3817
TAGAAGAAAT AGGAATTGTC CTGGGATGTT TGGTGGAAAC AAGGTATTTA ACTTTGGTTA 3877
TGGAAAATTC ACCGTGGCAG TGAATTAGCA TTGAGTGAGG AACAACTTAG GTAGTTTGGG 3937
ACTATTCAGA CTTGGATGAG ATACATCTTT AAAACATATG CAGCTGGGCA CAGTGGCTCA 3997
CGCCTGTAAC CCCAGCACTT TAGGAGGCTG AGGCGGGCGG ATCACCTGAA GTCACGAGTT 4057
CGAGACCAGC CTGGCCAAGG CGGTGAAACC CCATCTCTAT TAAAAATACA AAAATTAGTG 4117
GGGCATGGTG GTGGGTGCCT GTAATCCCAG CTACTCAGGG GGCTGAGGCA GGAGAATCGC 4177
TTGAACCCGG GAGGGGGAGG TTGCAGTGAG CCGAGATCAT GTCACAGCAC TACAGCCTAG 4237
ATGACAGAGC GAGCCTCCGT CTCAAAAAAA AAAAAAAAAA AAAAGGGCGG CCGC 4291

(2) INFORMATION FOR SEQ ID NO:5:

(i) SEQUENCE CHARACTERISTICS:
(A) LENGTH: 753 amino acids
(B) TYPE: amino acid
(D) TOPOLOGY: linear

(ii) MOLECULE TYPE: protein

(v) FRAGMENT TYPE: internal

(xi) SEQUENCE DESCRIPTION: SEQ ID NO:5:

(2) INFORMATION FOR SEQ ID NO:6:

(i) SEQUENCE CHARACTERISTICS:
(A) LENGTH: 2259 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

ATGATTGCGG AGCCCGCTCA CTTTTACCTG TTTGGATTAA TATGTCTCTG TTCAGGCTCC 60
CGTCTTCGTC AGGAAGATTT TCCACCTCGC ATTGTTGAAC ACCCTTCAGA CCTGATTGTC 120
TCAAAAGGAG AACCTGCAAC TTTGAACTGC AAAGCTGAAG GCCGCCCCAC ACCCACTATT 180
GAATGGTACA AAGGGGGAGA GAGAGTGGAG ACAGACAAAG ATGACCCTCG CTCACACCGA 240
ATGTTGCTGC CGAGTGGATC TTTATTTTTC TTACGTATAG TACATGGACG GAAAAGTAGA 300
CCTGATGAAG GAGTCTATGT CTGTGTAGCA AGGAATTACC TTGGAGAGGC TGTGAGCCAC 360
AATGCATCGC TGGAAGTAGC CATACTTCGG GATGACTTCA GACAAAACCC TTCGGATGTC 420
ATGGTTGCAG TAGGAGAGCC TGCAGTAATG GAATGCCAAC CTCCACGAGG CCATCCTGAG 480
CCCACCATTT CATGGAAGAA AGATGGCTCT CCACTGGATG ATAAAGATGA AAGAATAACT 540
ATACGAGGAG GAAAGCTCAT GATCACTTAC ACCCGTAAAA GTGACGCTGG CAAATATGTT 600
TGTGTTGGTA CCAATATGGT TGGGGAACGT GAGAGTGAAG TAGCCGAGCT GACTGTCTTA 660
GAGAGACCAT CATTTGTGAA GAGACCCAGT AACTTGGCAG TAACTGTGGA TGACAGTGCA 720
GAATTTAAAT GTGAGGCCCG AGGTGACCCT GTACCTACAG TACGATGGAG GAAAGATGAT 780
GGAGAGCTGC CCAAATCCAG ATATGAAATC CGAGATGATC ATACCTTGAA AATTAGGAAG 840
GTGACAGCTG GTGACATGGG TTCATACACT TGTGTTGCAG AAAATATGGT GGGCAAAGCT 900
GAAGCATCTG CTACTCTGAC TGTTCAAGTT GGGTCTGAAC CTCCACATTT TGTTGTGAAA 960
CCCCGTGACC AGGTTGTTGC TTTGGGACGG ACTGTAACTT TTCAGTGTGA AGCAACCGGA 1020
AATCCTCAAC CAGCTATTTT CTGGAGGAGA GAAGGGAGTC AGAATCTACT TTTCTCATAT 1080
CAACCACCAC AGTCATCCAG CCGATTTTCA GTCTCCCAGA CTGGCGACCT CACAATTACT 1140
AATGTCCAGC GATCTGATGT TGGTTATTAC ATCTGCCAGA CTTTAAATGT TGCTGGAAGC 1200
ATCATCACAA AGGCATATTT GGAAGTTACA GATGTGATTG CAGATCGGCC TCCCCCAGTT 1260
ATTCGACAAG GTCCTGTGAA TCAGACTGTA GCCGTGGATG GCACTTTCGT CCTCAGCTGT 1320
GTGGCCACAG GCAGTCCAGT GCCCACCATT CTGTGGAGAA AGGATGGAGT CCTCGTTTCA 1380
ACCCAAGACT CTCGAATCAA ACAGTTGGAG AATGGAGTAC TGCAGATCCG ATATGCTAAG 1440
CTGGGTGATA CTGGTCGGTA CACCTGCATT GCATCAACCC CCAGTGGTGA AGCAACATGG 1500
AGTGCTTACA TTGAAGTTCA AGAATTTGGA GTTCCAGTTC AGCCTCCAAG ACCTACTGAC 1560
CCAAATTTAA TCCCTAGTGC CCCATCAAAA CCTGAAGTGA CAGATGTCAG CAGAAATACA 1620
GTCACATTAT CATGGCAACC AAATTTGAAT TCAGGAGCAA CTCCAACATC TTATATTATA 1680
GAAGCCTTCA GCCATGCATC TGGTAGCAGC TGGCAGACCG TAGCAGAGAA TGTGAAAACA 1740
GAAACATCTG CCATTAAAGG ACTCAAACCT AATGCAATTT ACCTTTTCCT TGTGAGGGCA 1800
GCTAATGCAT ATGGAATTAG TGATCCAAGC CAAATATCAG ATCCAGTGAA AACACAAGAT 1860
GTCCTACCAA CAAGTCAGGG GGTGGACCAC AAGCAGGTCC AGAGAGAGCT GGGAAATGCT 1920
GTTCTGCACC TCCACAACCC CACCGTCCTT TCTTCCTCTT CCATCGAAGT GCACTGGACA 1980
GTAGATCAAC AGTCTCAGTA TATACAAGGA TATAAAATTC TCTATCGGCC ATCTGGAGCC 2040
AACCACGGAG AATCAGACTG GTTAGTTTTT GAAGTGAGGA CGCCAGCCAA AAACAGTGTG 2100

GTAATCCCTG ATCTCAGAAA GGGAGTCAAC TATGAAATTA AGGCTCGCCC TTTTTTTAAT 2160
GAATTTCAAG GAGCAGATAG TGAAATCAAG TTTGCCAAAA CCCTGGAAGA AGGCAAGTAT 2220
GATGAAGCGT TTGATTTCCA TGCATTGCAG TATATTTGT 2259

(2) INFORMATION FOR SEQ ID NO:7:

(i) SEQUENCE CHARACTERISTICS:
(A) LENGTH: 85 amino acids
(B) TYPE: amino acid
(D) TOPOLOGY: linear

(ii) MOLECULE TYPE: peptide

(xi) SEQUENCE DESCRIPTION: SEQ ID NO:7:

Pro Ser Ala Pro Ser Lys Pro Glu Val Thr Asp Val Ser Arg Asn Thr
1               5                   10                  15
Val Leu Ser Trp Gln Pro Asn Leu Asn Ser Gly Ala Thr Pro Thr Ser
            20                  25                  30
Tyr Ile Ile Glu Ala Phe Ser His Ala Ser Gly Ser Ser Trp Gln Thr
        35                  40                  45
Val Ala Glu Asn Val Lys Thr Glu Thr Ser Ala Ile Lys Gly Leu Lys
    50                  55                  60
Pro Asn Ala Ile Tyr Leu Phe Leu Val Arg Ala Ala Asn Ala Tyr Gly
65                  70                  75                  80
Ile Ser Asp Pro Ser
                85

(2) INFORMATION FOR SEQ ID NO:8:

(i) SEQUENCE CHARACTERISTICS:
(A) LENGTH: 90 amino acids
(B) TYPE: amino acid
(D) TOPOLOGY: linear

(ii) MOLECULE TYPE: peptide

(xi) SEQUENCE DESCRIPTION: SEQ ID NO:8:

Gly Asn Ala Val Leu His Leu His Asn Pro Thr Val Leu Ser Ser Ser
1               5                   10                  15
Ser Ile Glu Val His Trp Thr Val Asp Gln Gln Ser Gln Tyr Ile Gln
            20                  25                  30
Gly Tyr Lys Ile Leu Tyr Arg Pro Ser Gly Ala Asn His Gly Glu Ser
        35                  40                  45
Asp Trp Leu Val Phe Glu Val Arg Thr Pro Ala Lys Asn Ser Val Val
    50                  55                  60
Ile Pro Asp Leu Arg Lys Gly Val Asn Tyr Glu Ile Lys Ala Arg Pro
65                  70                  75                  80
Phe Phe Asn Glu Phe Gln Gly Ala Asp Ser
                85                  90

(2) INFORMATION FOR SEQ ID NO:9:

(i) SEQUENCE CHARACTERISTICS:
(A) LENGTH: 68 amino acids
(B) TYPE: amino acid

(D) TOPOLOGY: linear

(ii) MOLECULE TYPE: peptide

(xi) SEQUENCE DESCRIPTION: SEQ ID NO:9:

Gly Glu Pro Ala Thr Leu Asn Cys Lys Ala Glu Gly Arg Pro Thr Pro
1               5                   10                  15
Thr Ile Glu Trp Tyr Lys Gly Gly Glu Arg Val Glu Thr Asp Lys Asp
            20                  25                  30
Asp Pro Arg Ser His Arg Met Leu Leu Pro Ser Gly Ser Leu Phe Phe
        35                  40                  45
Leu Arg Ile Val His Gly Arg Lys Ser Arg Pro Asp Glu Gly Val Tyr
    50                  55                  60
Val Cys Val Ala
65

(2) INFORMATION FOR SEQ ID NO:10:

(i) SEQUENCE CHARACTERISTICS:
(A) LENGTH: 59 amino acids
(B) TYPE: amino acid
(D) TOPOLOGY: linear

(ii) MOLECULE TYPE: peptide

(xi) SEQUENCE DESCRIPTION: SEQ ID NO:10:

Gly Glu Pro Ala Val Met Glu Cys Gln Pro Pro Arg Gly His Pro Glu
1               5                   10                  15
Pro Thr Ile Ser Trp Lys Lys Asp Gly Ser Pro Leu Asp Asp Lys Asp
            20                  25                  30
Glu Arg Ile Thr Ile Arg Gly Gly Lys Leu Met Ile Thr Tyr Thr Arg
        35                  40                  45
Lys Ser Asp Ala Gly Lys Tyr Val Cys Val Gly
    50                  55

(2) INFORMATION FOR SEQ ID NO:11:

(i) SEQUENCE CHARACTERISTICS:
(A) LENGTH: 57 amino acids
(B) TYPE: amino acid
(D) TOPOLOGY: linear

(ii) MOLECULE TYPE: peptide

(xi) SEQUENCE DESCRIPTION: SEQ ID NO:11:

Asp Asp Ser Ala Glu Phe Lys Cys Glu Ala Arg Gly Asp Pro Val Pro
1               5                   10                  15
Thr Val Arg Trp Arg Lys Asp Asp Gly Glu Leu Pro Lys Ser Arg Tyr
            20                  25                  30
Glu Ile Arg Asp Asp His Thr Leu Lys Ile Arg Lys Val Thr Ala Gly
        35                  40                  45
Asp Met Gly Ser Tyr Thr Cys Val Ala
    50                  55

(2) INFORMATION FOR SEQ ID NO:12:

(i) SEQUENCE CHARACTERISTICS:
(A) LENGTH: 66 amino acids
(B) TYPE: amino acid
(D) TOPOLOGY: linear

(ii) MOLECULE TYPE: peptide

(xi) SEQUENCE DESCRIPTION: SEQ ID NO:12:

Gly Arg Thr Val Thr Phe Gln Cys Glu Ala Thr Gly Asn Pro Gln Pro
1               5                   10                  15
Ala Ile Phe Trp Arg Arg Glu Gly Ser Gln Asn Leu Leu Phe Ser Tyr
            20                  25                  30
Gln Pro Pro Gln Ser Ser Ser Arg Phe Ser Val Ser Gln Thr Gly Asp
        35                  40                  45
Leu Thr Ile Thr Asn Val Gln Arg Ser Asp Val Gly Tyr Tyr Ile Cys
    50                  55                  60
Gln Thr
65

(2) INFORMATION FOR SEQ ID NO:13:

(i) SEQUENCE CHARACTERISTICS:
(A) LENGTH: 59 amino acids
(B) TYPE: amino acid
(D) TOPOLOGY: linear

(ii) MOLECULE TYPE: peptide

(xi) SEQUENCE DESCRIPTION: SEQ ID NO:13:

Asp Gly Thr Phe Val Leu Ser Cys Val Ala Thr Gly Ser Pro Val Pro
1               5                   10                  15
Thr Ile Leu Trp Arg Lys Asp Gly Val Leu Val Ser Thr Gln Asp Ser
            20                  25                  30
Arg Ile Lys Gln Leu Glu Asn Gly Val Leu Gln Ile Arg Tyr Ala Lys
        35                  40                  45
Leu Gly Asp Thr Gly Arg Tyr Thr Cys Val Ala
    50                  55

(2) INFORMATION FOR SEQ ID NO:14:

(i) SEQUENCE CHARACTERISTICS:
(A) LENGTH: 2122 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: Coding Sequence
(B) LOCATION: 1...1047

(xi) SEQUENCE DESCRIPTION: SEQ ID NO:14:

CCA CGC GTC CGC CGG AGC GCT CGC CCA GCC GCC GCC TCC AAG CCC CTG 48
Pro Arg Val Arg Arg Ser Ala Arg Pro Ala Ala Ala Ser Lys Pro Leu
1               5                   10                  15

AGG TTT CCG GGG ACC ACA ATG AAC AAG TTG CTG TGC TGC GCG CTC GTG 96
Arg Phe Pro Gly Thr Thr Met Asn Lys Leu Leu Cys Cys Ala Leu Val
            20                  25                  30

TTT CTG GAC ATC TCC ATT AAG TGG ACC ACC CAG GAA ACG TTT CCT CCA 144
Phe Leu Asp Ile Ser Ile Lys Trp Thr Thr Gln Glu Thr Phe Pro Pro
        35                  40                  45

AAG TAC CTT CAT TAT GAC GAA GAA ACC TCT CAT CAG CTG TTG TGT GAC 192
Lys Tyr Leu His Tyr Asp Glu Glu Thr Ser His Gln Leu Leu Cys Asp
    50                  55                  60

AAA TGT CCT CCT GGT ACC TAC CTA AAA CAA CAC TGT ACA GCA AAG TGG 240
Lys Cys Pro Pro Gly Thr Tyr Leu Lys Gln His Cys Thr Ala Lys Trp
65                  70                  75                  80

AAG ACC GTG TGC GCC CCT TGC CCT GAC CAC TAC TAC ACA GAC AGC TGG 288
Lys Thr Val Cys Ala Pro Cys Pro Asp His Tyr Tyr Thr Asp Ser Trp
                85                  90                  95

CAC ACC AGT GAC GAG TGT CTA TAC TGC AGC CCC GTG TGC AAG GAG CTG 336
His Thr Ser Asp Glu Cys Leu Tyr Cys Ser Pro Val Cys Lys Glu Leu
            100                 105                 110

CAG TAC GTC AAG CAG GAG TGC AAT CGC ACC CAC AAC CGC GTG TGC GAA 384
Gln Tyr Val Lys Gln Glu Cys Asn Arg Thr His Asn Arg Val Cys Glu
        115                 120                 125

TGC AAG GAA GGG CGC TAC CTT GAG ATA GAG TTC TGC TTG AAA CAT AGG 432
Cys Lys Glu Gly Arg Tyr Leu Glu Ile Glu Phe Cys Leu Lys His Arg
    130                 135                 140

AGC TGC CCT CCT GGA TTT GGA GTG GTG CAA GCT GGA ACC CCA GAG CGA 480
Ser Cys Pro Pro Gly Phe Gly Val Val Gln Ala Gly Thr Pro Glu Arg
145                 150                 155                 160

AAT ACA GTT TGC AAA AGA TGT CCA GAT GGG TTC TTC TCA AAT GAG ACG 528
Asn Thr Val Cys Lys Arg Cys Pro Asp Gly Phe Phe Ser Asn Glu Thr
                165                 170                 175

TCA TCT AAA GCA CCC TGT AGA AAA CAC ACA AAT TGC AGT GTC TTT GGT 576
Ser Ser Lys Ala Pro Cys Arg Lys His Thr Asn Cys Ser Val Phe Gly
            180                 185                 190

CTC CTG CTA ACT CAG AAA GGA AAT GCA ACA CAC GAC AAC ATA TGT TCC 624
Leu Leu Leu Thr Gln Lys Gly Asn Ala Thr His Asp Asn Ile Cys Ser
        195                 200                 205

GGA AAC AGT GAA TCA ACT CAA AAA TGT GGA ATA GAT GTT ACC CTG TGT 672
Gly Asn Ser Glu Ser Thr Gln Lys Cys Gly Ile Asp Val Thr Leu Cys
    210                 215                 220

GAG GAG GCA TTC TTC AGG TTT GCT GTT CCT ACA AAG TTT ACG CCT AAC     720
Glu Glu Ala Phe Phe Arg Phe Ala Val Pro Thr Lys Phe Thr Pro Asn
225                 230                 235                 240

TGG CTT AGT GTC TTG GTA GAC AAT TTG CCT GGC ACC AAA GTA AAC GCA     768
Trp Leu Ser Val Leu Val Asp Asn Leu Pro Gly Thr Lys Val Asn Ala
                245                 250                 255

GAG AGT GTA GAG AGG ATA AAA CGG CAA CAC AGC TCA CAA GAA CAG ACT     816
Glu Ser Val Glu Arg Ile Lys Arg Gln His Ser Ser Gln Glu Gln Thr
            260                 265                 270

TTC CAG CTG CTG AAG TTA TGG AAA CAT CAA AAC AAA GAC CAA GAT ATA     864
Phe Gln Leu Leu Lys Leu Trp Lys His Gln Asn Lys Asp Gln Asp Ile
        275                 280                 285

GTC CAG AAG ATC ATC CAA GAT ATT GAC CTC TGT GAA AAC AGC GTG CAG     912
Val Gln Lys Ile Ile Gln Asp Ile Asp Leu Cys Glu Asn Ser Val Gln
    290                 295                 300

CGG CAC ATT GGA CAT GCT AAC CTC ACC TTC GAG CAG CTT CGT AGC TTG     960
Arg His Ile Gly His Ala Asn Leu Thr Phe Glu Gln Leu Arg Ser Leu
305                 310                 315                 320

ATG GAA AGC TTA CCG GGG AAA GAA AGT GGG AGC AGG AAG GAC ATT GGA    1008
Met Glu Ser Leu Pro Gly Lys Glu Ser Gly Ser Arg Lys Asp Ile Gly
                325                 330                 335

AAA AAC AAT AAA GGC ATG CAA ACC CAG TGG ACC AGA TCC TGAAGCTGCT CA    1059
Lys Asn Asn Lys Gly Met Gln Thr Gln Trp Thr Arg Ser
            340                 345

GTTTGTGGCG AATAAAAAAT GGCGACCAAG ACACCTTGAA GGGCCTAATG CACGCACTAA    1119
AGCACTCAAA GACGTACCAC TTTCCCAAAA CTGTCACTCA GAGTCTAAAG AAGACCATCA    1179
GGTTCCTTCA CAGCTTCACA ATGTACAAAT TGTATCAGAA GTTATTTTTA GAAATGATAG    1239
GTAACCAGGT CCAATCAGTA AAAATAAGCT GCTTATAACT GGAAATGGCC ATTGAGCTGT    1299
TTCCTCACAA TTGGCGAGAT CCCCATGGAT GAGTAAACTG TTTCTCAGGC ACTTGAGGCT    1359
TTCAGTGATA TCTTTCTCAT TACCAGTGAC TAATTTTGCC ACAGGGTACT AAAAGAAACT    1419
ATGATGTGGA GAAAGGACTA ACATCTCCTC CAATAAACCC CAAATGGTTA ATCCAACTGT    1479
CAGATCTGGA TCGTTATCTA CTGACTATAT TTTCCCTTAT TACTGCTTGC AGTAATTCAA    1539
CTGGAAATTA AAAAAAAAAA ACTAGACTCC ATTGTGCCTT ACTAAATATG GGAATGTCTA    1599
ACTTAAATAG CTTTGAGATT TCAGCTATGC TAGAGGCTTT TATTAGAAAG CCATATTTTT    1659
TTCTGTAAAA GTTACTAATA TATCTGTAAC ACTATTACAG TATTGCTATT TATATTCATT    1719
CAGATATAAG ATTTGTACAT ATTATCATCC TATAAAGAAA CGGTATGACT TAATTTTAGA    1779
AAGAAAATTA TATTCTGTTT ATTATGACAA ATGAAAGAGA AAATATATAT TTTTAATGGA    1839
AAGTTTGTAG CATTTTTCTA ATAGGTACTG CCATATTTTT CTGTGTGGAG TATTTTTATA    1899
ATTTTATCTG TATAAGCTGT AATATCATTT TATAGAAAAT GCATTATTTA GTCAATTGTT    1959
TAATGTTGGA AAACATATGA AATATAAATT ATCTGAATAT TAGATGCTCT GAGAAATTGA    2019
ATGTACCTTA TTTAAAAGAT TTTATGGTTT TATAACTATA TAAATGACAT TATTAAAGTT    2079
TTCAAATTAT TTTTAAAAAA AAAAAAAAAA AAAAAAAAAA AAA                      2122

(2) INFORMATION FOR SEQ ID NO:15:

(i) SEQUENCE CHARACTERISTICS:
(A) LENGTH: 349 amino acids
(B) TYPE: amino acid
(D) TOPOLOGY: linear

(ii) MOLECULE TYPE: protein

(v) FRAGMENT TYPE: internal

(xi) SEQUENCE DESCRIPTION: SEQ ID NO:15:

Pro Arg Val Arg Arg Ser Ala Arg Pro Ala Ala Ala Ser Lys Pro Leu
1               5                   10                  15
Arg Phe Pro Gly Thr Thr Met Asn Lys Leu Leu Cys Cys Ala Leu Val
            20                  25                  30
Phe Leu Asp Ile Ser Ile Lys Trp Thr Thr Gln Glu Thr Phe Pro Pro
        35                  40                  45
Lys Tyr Leu His Tyr Asp Glu Glu Thr Ser His Gln Leu Leu Cys Asp
    50                  55                  60
Lys Cys Pro Pro Gly Thr Tyr Leu Lys Gln His Cys Thr Ala Lys Trp
65                  70                  75                  80
Lys Thr Val Cys Ala Pro Cys Pro Asp His Tyr Tyr Thr Asp Ser Trp
                85                  90                  95
His Thr Ser Asp Glu Cys Leu Tyr Cys Ser Pro Val Cys Lys Glu Leu
            100                 105                 110
Gln Tyr Val Lys Gln Glu Cys Asn Arg Thr His Asn Arg Val Cys Glu
        115                 120                 125
Cys Lys Glu Gly Arg Tyr Leu Glu Ile Glu Phe Cys Leu Lys His Arg
    130                 135                 140
Ser Cys Pro Pro Gly Phe Gly Val Val Gln Ala Gly Thr Pro Glu Arg
145                 150                 155                 160
Asn Thr Val Cys Lys Arg Cys Pro Asp Gly Phe Phe Ser Asn Glu Thr
                165                 170                 175
Ser Ser Lys Ala Pro Cys Arg Lys His Thr Asn Cys Ser Val Phe Gly
            180                 185                 190
Leu Leu Leu Thr Gln Lys Gly Asn Ala Thr His Asp Asn Ile Cys Ser
        195                 200                 205
Gly Asn Ser Glu Ser Thr Gln Lys Cys Gly Ile Asp Val Thr Leu Cys
    210                 215                 220
Glu Glu Ala Phe Phe Arg Phe Ala Val Pro Thr Lys Phe Thr Pro Asn
225                 230                 235                 240
Trp Leu Ser Val Leu Val Asp Asn Leu Pro Gly Thr Lys Val Asn Ala
                245                 250                 255
Glu Ser Val Glu Arg Ile Lys Arg Gln His Ser Ser Gln Glu Gln Thr
            260                 265                 270
Phe Gln Leu Leu Lys Leu Trp Lys His Gln Asn Lys Asp Gln Asp Ile
        275                 280                 285
Val Gln Lys Ile Ile Gln Asp Ile Asp Leu Cys Glu Asn Ser Val Gln
    290                 295                 300
Arg His Ile Gly His Ala Asn Leu Thr Phe Glu Gln Leu Arg Ser Leu
305                 310                 315                 320
Met Glu Ser Leu Pro Gly Lys Glu Ser Gly Ser Arg Lys Asp Ile Gly
                325                 330                 335
Lys Asn Asn Lys Gly Met Gln Thr Gln Trp Thr Arg Ser
            340                 345

(2) INFORMATION FOR SEQ ID NO:16:

(i) SEQUENCE CHARACTERISTICS:
(A) LENGTH: 84 amino acids
(B) TYPE: amino acid

(D) TOPOLOGY: linear

(ii) MOLECULE TYPE: peptide

(xi) SEQUENCE DESCRIPTION: SEQ ID NO:16:

Pro Ser Pro Pro Arg Asn Leu Arg Val Thr Asp Ile Thr Pro Thr Ser
1               5                   10                  15
Ile Thr Val Ser Trp Thr Pro Pro Glu Gly Asn Gly Pro Ile Thr Gly
            20                  25                  30
Tyr Arg Ile Gln Tyr Arg Trp Pro Val Asn Asp Asn Glu Trp Asn Glu
        35                  40                  45
Phe Asn Val Pro Arg Thr Thr Asn Ser Tyr Thr Ile Thr Asn Leu Arg
    50                  55                  60
Pro Gly Thr Glu Tyr Glu Phe Arg Val Gln Ala Val Asn Gly Asp Gly
65                  70                  75                  80
Gly Pro Trp Ser

(2) INFORMATION FOR SEQ ID NO:17:

(i) SEQUENCE CHARACTERISTICS:
(A) LENGTH: 47 amino acids
(B) TYPE: amino acid
(D) TOPOLOGY: linear

(ii) MOLECULE TYPE: peptide

(xi) SEQUENCE DESCRIPTION: SEQ ID NO:17:

Gly Gln Ser Val Thr Leu Thr Cys Met Val Ser Phe His Pro Gln Asp
1               5                   10                  15
Tyr Thr Ile Trp Trp Tyr Arg Asn Gly Gly Pro Ile Thr Leu Thr Ile
            20                  25                  30
Asn Ser Trp Gln Val Glu Asp Ser Gly Thr Tyr Trp Cys Met Val
        35                  40                  45

(2) INFORMATION FOR SEQ ID NO:18:

(i) SEQUENCE CHARACTERISTICS:
(A) LENGTH: 1650 amino acids
(B) TYPE: amino acid
(D) TOPOLOGY: linear

(ii) MOLECULE TYPE: protein

(xi) SEQUENCE DESCRIPTION: SEQ ID NO:18:

Met Lys Trp Lys His Val Pro Phe Leu Val Met Ile Ser Leu Leu Ser
1               5                   10                  15
Leu Ser Pro Asn His Leu Phe Leu Ala Gln Leu Ile Pro Asp Pro Glu
            20                  25                  30
Asp Val Glu Arg Gly Asn Asp His Gly Thr Pro Ile Pro Thr Ser Asp
        35                  40                  45
Asn Asp Asp Asn Ser Leu Gly Tyr Thr Gly Ser Arg Leu Arg Gln Glu
    50                  55                  60
Asp Phe Pro Pro Arg Ile Val Glu His Pro Ser Asp Leu Ile Val Ser

65                  70                  75                  80
Lys Gly Glu Pro Ala Thr Leu Asn Cys Lys Ala Glu Gly Arg Pro Thr
                85                  90                  95
Pro Thr Ile Glu Trp Tyr Lys Gly Gly Glu Arg Val Glu Thr Asp Lys
            100                 105                 110
Asp Asp Pro Arg Ser His Arg Met Leu Leu Pro Ser Gly Ser Leu Phe
        115                 120                 125
Phe Leu Arg Ile Val His Gly Arg Lys Ser Arg Pro Asp Glu Gly Val
    130                 135                 140
Tyr Val Cys Val Ala Arg Asn Tyr Leu Gly Glu Ala Val Ser His Asn
145                 150                 155                 160
Ala Ser Leu Glu Val Ala Ile Leu Arg Asp Asp Phe Arg Gln Asn Pro
                165                 170                 175
Ser Asp Val Met Val Ala Val Gly Glu Pro Ala Val Met Glu Cys Gln
            180                 185                 190
Pro Pro Arg Gly His Pro Glu Pro Thr Ile Ser Trp Lys Lys Asp Gly
        195                 200                 205
Ser Pro Leu Asp Asp Lys Asp Glu Arg Ile Thr Ile Arg Gly Gly Lys
    210                 215                 220
Leu Met Ile Thr Tyr Thr Arg Lys Ser Asp Ala Gly Lys Tyr Val Cys
225                 230                 235                 240
Val Gly Thr Asn Met Val Gly Glu Arg Glu Ser Glu Val Ala Glu Leu
                245                 250                 255
Thr Val Leu Glu Arg Pro Ser Phe Val Lys Arg Pro Ser Asn Leu Ala
            260                 265                 270
Val Thr Asp Asp Ser Ala Glu Phe Lys Cys Glu Ala Arg Gly Asp Pro
        275                 280                 285
Val Pro Thr Val Arg Trp Arg Lys Asp Asp Gly Glu Leu Pro Lys Ser
    290                 295                 300
Arg Tyr Glu Ile Arg Asp Asp His Thr Leu Lys Ile Arg Lys Val Thr
305                 310                 315                 320
Ala Gly Asp Met Gly Ser Tyr Thr Cys Val Ala Glu Asn Met Val Gly
                325                 330                 335
Lys Ala Glu Ala Ser Ala Thr Leu Thr Val Gln Glu Pro Pro His Phe
            340                 345                 350
Val Val Lys Pro Arg Asp Gln Val Val Ala Leu Gly Arg Thr Val Thr
        355                 360                 365
Phe Gln Cys Glu Ala Thr Gly Asn Pro Gln Pro Ala Ile Phe Trp Arg
    370                 375                 380
Arg Glu Gly Ser Gln Asn Leu Leu Phe Ser Tyr Gln Pro Pro Gln Ser
385                 390                 395                 400
Ser Ser Arg Phe Ser Val Ser Gln Thr Gly Asp Leu Thr Ile Thr Asn
                405                 410                 415
Val Gln Arg Ser Asp Val Gly Tyr Tyr Ile Cys Gln Thr Leu Asn Val
            420                 425                 430
Ala Gly Ser Ile Ile Thr Lys Ala Tyr Leu Glu Val Thr Asp Val Ile
        435                 440                 445
Ala Asp Arg Pro Pro Pro Val Ile Arg Gln Gly Pro Val Asn Gln Thr
    450                 455                 460
Val Ala Val Asp Gly Thr Phe Val Leu Ser Cys Val Ala Thr Gly Ser
465                 470                 475                 480
Pro Val Pro Thr Ile Leu Trp Arg Lys Asp Gly Val Leu Val Ser Thr
                485                 490                 495
Gln Asp Ser Arg Ile Lys Gln Leu Glu Asn Gly Val Leu Gln Ile Arg
            500                 505                 510
Tyr Ala Lys Leu Gly Asp Thr Gly Arg Tyr Thr Cys Ile Ala Ser Thr
        515                 520                 525

Pro Ser Gly Glu Ala Thr Trp Ser Ala Tyr Ile Glu Val Gln Glu Phe
    530                 535                 540
Gly Val Pro Val Gln Pro Pro Arg Pro Thr Asp Pro Asn Leu Ile Pro
545                 550                 555                 560
Ser Ala Pro Ser Lys Pro Glu Val Thr Asp Val Ser Arg Asn Thr Val
                565                 570                 575
Thr Leu Ser Trp Gln Pro Asn Leu Asn Ser Gly Ala Thr Pro Thr Ser
            580                 585                 590
Tyr Ile Ile Glu Ala Phe Ser His Ala Ser Gly Ser Ser Trp Gln Thr
        595                 600                 605
Val Ala Glu Asn Val Lys Thr Glu Thr Ser Ala Ile Lys Gly Leu Lys
    610                 615                 620
Pro Asn Ala Ile Tyr Leu Phe Leu Val Arg Ala Ala Asn Ala Tyr Gly
625                 630                 635                 640
Ile Ser Asp Pro Ser Gln Ile Ser Asp Pro Val Lys Thr Gln Asp Val
                645                 650                 655
Leu Pro Thr Ser Gln Gly Val Asp His Lys Gln Val Gln Arg Glu Leu
            660                 665                 670
Gly Asn Ala Val Leu His Leu His Asn Pro Thr Val Leu Ser Ser Ser
        675                 680                 685
Ser Ile Glu Val His Trp Thr Val Asp Gln Gln Ser Gln Tyr Ile Gln
    690                 695                 700
Gly Tyr Lys Ile Leu Tyr Arg Pro Ser Gly Ala Asn His Gly Glu Ser
705                 710                 715                 720
Asp Trp Leu Val Phe Glu Val Arg Thr Pro Ala Lys Asn Ser Val Val
                725                 730                 735
Ile Pro Asp Leu Arg Lys Gly Val Asn Tyr Glu Ile Lys Ala Arg Pro
            740                 745                 750
Phe Phe Asn Glu Phe Gln Gly Ala Asp Ser Glu Ile Lys Phe Ala Lys
        755                 760                 765
Thr Leu Glu Glu Ala Pro Ser Ala Pro Pro Gln Gly Val Thr Val Ser
    770                 775                 780
Lys Asn Asp Gly Asn Gly Thr Ala Ile Leu Val Ser Trp Gln Pro Pro
785                 790                 795                 800
Pro Glu Asp Thr Gln Asn Gly Met Val Gln Glu Tyr Lys Val Trp Cys
                805                 810                 815
Leu Gly Asn Glu Thr Arg Tyr His Ile Asn Lys Thr Val Asp Gly Ser
            820                 825                 830
Thr Phe Ser Val Val Ile Pro Phe Leu Val Pro Gly Ile Arg Tyr Ser
        835                 840                 845
Val Glu Val Ala Ala Ser Thr Gly Ala Gly Ser Gly Val Lys Ser Glu
    850                 855                 860
Pro Gln Phe Ile Gln Leu Asp Ala His Gly Asn Pro Val Ser Pro Glu
865                 870                 875                 880
Asp Gln Val Ser Leu Ala Gln Gln Ile Ser Asp Val Val Lys Gln Pro
                885                 890                 895
Ala Phe Ile Ala Gly Ile Gly Ala Ala Cys Trp Ile Ile Leu Met Val
            900                 905                 910
Phe Ser Ile Trp Leu Tyr Arg His Arg Lys Lys Arg Asn Gly Leu Thr
        915                 920                 925
Ser Thr Tyr Ala Gly Ile Arg Lys Val Pro Ser Phe Thr Phe Thr Pro
    930                 935                 940
Thr Val Thr Tyr Glu Arg Gly Gly Glu Ala Val Ser Ser Gly Gly Arg
945                 950                 955                 960
Pro Gly Leu Leu Asn Ile Ser Glu Pro Ala Ala Gln Pro Trp Leu Ala
                965                 970                 975
Asp Thr Trp Pro Asn Thr Gly Asn Asn His Asn Asp Cys Ser Ile Ser

            980                 985                 990
Cys Cys Thr Ala Gly Asn Gly Asn Ser Asp Ser Asn Leu Thr Thr Tyr
        995                 1000                1005
Ser Arg Pro Ala Asp Cys Ile Ala Asn Tyr Asn Asn Gln Leu Asp Asn
    1010                1015                1020
Lys Gln Thr Asn Leu Met Leu Pro Glu Ser Thr Val Tyr Gly Asp Val
025 1030 1035 1040
Asp Leu Ser Asn Lys Ile Asn Glu Met Lys Thr Phe Asn Ser Pro Asn
                1045                1050                1055
Leu Lys Asp Gly Arg Phe Val Asn Pro Ser Gly Gln Pro Thr Pro Tyr
            1060                1065                1070
Ala Thr Thr Gln Leu Ile Gln Ser Asn Leu Ser Asn Asn Met Asn Asn
        1075                1080                1085
Gly Ser Gly Asp Ser Gly Glu Lys His Trp Lys Pro Leu Gly Gln Gln
    1090                1095                1100
Lys Gln Glu Val Ala Pro Val Gln Tyr Asn Ile Val Glu Gln Asn Lys
105 1110 1115 1120
Leu Asn Lys Asp Tyr Arg Ala Asn Asp Thr Val Pro Pro Thr Ile Pro
                1125                1130                1135
Tyr Asn Gln Ser Tyr Asp Gln Asn Thr Gly Gly Ser Tyr Asn Ser Ser
            1140                1145                1150
Asp Arg Gly Ser Ser Thr Ser Gly Ser Gln Gly His Lys Lys Gly Ala
        1155                1160                1165
Arg Thr Pro Lys Val Pro Lys Gln Gly Gly Met Asn Trp Ala Asp Leu
    1170                1175                1180
Leu Pro Pro Pro Pro Ala His Pro Pro Pro His Ser Asn Ser Glu Glu
185 1190 1195 1200
Tyr Asn Ile Ser Val Asp Glu Ser Tyr Asp Gln Glu Met Pro Cys Pro
                1205                1210                1215
Val Pro Pro Ala Arg Met Tyr Leu Gln Gln Asp Glu Leu Glu Glu Glu
            1220                1225                1230
Glu Asp Glu Arg Gly Pro Thr Pro Pro Val Arg Gly Ala Ala Ser Ser
        1235                1240                1245
Pro Ala Ala Val Ser Tyr Ser His Gln Ser Thr Ala Thr Leu Thr Pro
    1250                1255                1260
Ser Pro Gln Glu Glu Leu Gln Pro Met Leu Gln Asp Cys Pro Glu Glu
265 1270 1275 1280
Thr Gly His Met Gln His Gln Pro Asp Arg Arg Arg Gln Pro Val Ser
                1285                1290                1295
Pro Pro Pro Pro Pro Arg Pro Ile Ser Pro Pro His Thr Tyr Gly Tyr
            1300                1305                1310
Ile Ser Gly Pro Leu Val Ser Asp Met Asp Thr Asp Ala Pro Glu Glu
        1315                1320                1325
Glu Glu Asp Glu Ala Asp Met Glu Val Ala Lys Met Gln Thr Arg Arg
    1330                1335                1340
Leu Leu Leu Arg Gly Leu Glu Gln Thr Pro Ala Ser Ser Val Gly Asp
345 1350 1355 1360
Leu Glu Ser Ser Val Thr Gly Ser Met Ile Asn Gly Trp Gly Ser Ala
                1365                1370                1375
Ser Glu Glu Asp Asn Ile Ser Ser Gly Arg Ser Ser Val Ser Ser Ser
            1380                1385                1390
Asp Gly Ser Phe Phe Thr Asp Ala Asp Phe Ala Gln Ala Val Ala Ala
        1395                1400                1405
Ala Ala Glu Tyr Ala Gly Leu Lys Val Ala Arg Arg Gln Met Gln Asp
    1410                1415                1420
Ala Ala Gly Arg Arg His Phe His Ala Ser Gln Cys Pro Arg Pro Thr
425 1430 1435 1440

Ser Pro Val Ser Thr Asp Ser Asn Met Ser Ala Ala Val Met Gln Lys
                1445                1450                1455
Thr Arg Pro Ala Lys Lys Leu Lys His Gln Pro Gly His Leu Arg Arg
            1460                1465                1470
Glu Thr Tyr Thr Asp Asp Leu Pro Pro Pro Pro Val Pro Pro Pro Ala
        1475                1480                1485
Ile Lys Ser Pro Thr Ala Gln Ser Lys Thr Gln Leu Glu Val Arg Pro
    1490                1495                1500
Val Val Val Pro Lys Leu Pro Ser Met Asp Ala Arg Thr Asp Arg Ser
505 1510 1515 1520
Ser Asp Arg Lys Gly Ser Ser Tyr Lys Gly Arg Glu Val Leu Asp Gly
                1525                1530                1535
Arg Gln Val Val Asp Met Arg Thr Asn Pro Gly Asp Pro Arg Glu Ala
            1540                1545                1550
Gln Glu Gln Gln Asn Asp Gly Lys Gly Arg Gly Asn Lys Ala Ala Lys
        1555                1560                1565
Arg Asp Leu Pro Pro Ala Lys Thr His Leu Ile Gln Glu Asp Ile Leu
    1570                1575                1580
Pro Tyr Cys Arg Pro Thr Phe Pro Thr Ser Asn Asn Pro Arg Asp Pro
585 1590 1595 1600
Ser Ser Ser Ser Ser Met Ser Ser Arg Gly Ser Gly Ser Arg Gln Arg
                1605                1610                1615
Glu Gln Ala Asn Val Gly Arg Arg Asn Ile Ala Glu Met Gln Val Leu
            1620                1625                1630
Gly Gly Tyr Glu Arg Gly Glu Asp Asn Asn Glu Glu Leu Glu Glu Thr
        1635                1640                1645
Glu Ser
    1650

## Description

### Background of the Invention

There is considerable medical interest in secreted and membrane-associated mammalian proteins. Many such proteins, for example, cytokines, are important for inducing the growth or differentiation of cells with which they interact or for triggering one or more specific cellular responses.

The demonstrated clinical utility of several secreted proteins in the treatment of human disease, for example, erythropoietin, granulocyte-macrophage colony stimulating factor (GM-CSF), human growth hormone, and various interleukins, illustrates the importance of secreted proteins.

Many membrane-associated proteins are receptors which bind a ligand(s) and transmit an intracellular signal. As such, membrane-associated proteins can be used to identify (or design) small molecules which act as agonists or antagonists of the ligand.

### Summary of the Invention

The present invention is based, at least in part, on the discovery of a gene encoding FTHMA-070, a protein having homology to tumor necrosis factor (TNF) receptor and on the discovery of a gene encoding T85 (also referred to as FMHB-SD4 or FMHB-6D4).

The FTHMA-070 cDNA described below (SEQ ID NO:1) has a 1203 nucleotide open reading frame (nucleotides 73-1275 of SEQ.ID NO:1; SEQ ID NO:3) which encodes a 403 amino acid protein (SEQ ID NO:2). This protein includes a predicted signal sequence of about 21 amino acids (from amino acid I to about amino acid 21 of SEQ ID NO:2) and a predicted mature protein of about 382 amino acids (from about amino acid 22 to amino acid 403 of SEQ ID NO:2; SEQ ID NO:4).

The T85 cDNA described below (SEQ ID NO:5) has a 2259 nucleotide open reading frame (nucleotides 958 - 3216 of SEQ ID NO:5; SEQ ID NO:7) which encodes a 753 amino acid protein (SEQ ID NO:6). This protein includes a predicted signal sequence of about 20 amino acids (from amino acid 1 to about amino acid 20 of SEQ ID NO:6) and a predicted mature protein of about 733 amino acids (from about amino acid 21 to amino acid 753 of SEQ ID NO:6; SEQ ID NO:8).

The nucleic acid and polypeptide molecules of the present invention are useful as modulating agents in regulating a variety of cellular processes. Accordingly, in one aspect, this invention provides isolated nucleic acid molecules encoding FTHMA-070 proteins or biologically active portions thereof, as well as nucleic acid fragments suitable as primers or hybridization probes for the detection of FTHMA-070-encoding nucleic acids. In another aspect, this invention provides isolated nucleic acid molecules encoding T85 proteins or biologically active portions thereof, as well as nucleic acid fragments suitable as primers or hybridization probes for the detection of T85-encoding nucleic acids.

The invention features a nucleic acid molecule which is at least 45% (or 55%, 65%, 75%, 85%, 95%, or 98%) identical to the nucleotide sequence shown in SEQ ID NO:1, or SEQ ID NO:3, or the nucleotide sequence of the cDNA insert of the plasmid deposited with ATCC as Accession Number (the "cDNA of ATCC "), or a complement thereof. The invention features a nucleic acid molecule which includes a fragment of at least 300 (325, 350, 375, 400, 425, 450, 500, 550, 600, 650, 700, 800, 900, 1000, or 1290) nucleotides of the nucleotide sequence shown in SEQ ID NO:1, or SEQ ID NO:3, or the nucleotide sequence of the cDNA ATCC , or a complement thereof.

The invention also features a nucleic acid molecule which includes a nucleotide sequence encoding a protein having an amino acid sequence that is at least 45% (or 55%, 65%, 75%, 85%, 95%, or 98%) identical to the amino acid sequence of SEQ ID NO:2, SEQ ID NO:4, or the amino acid sequence encoded by the cDNA of ATCC . In a preferred embodiment, a FTHMA-070 nucleic acid molecule has the nucleotide sequence shown -SEQ ID NO:1, or SEQ ID NO:3, or the nucleotide sequence of the cDNA of ATCC .

Also within the invention is a nucleic acid molecule which encodes a fragment of a polypeptide having the amino acid sequence of SEQ ID NO:2 or SEQ ID NO:4, the fragment including at least 15 (25, 30, 50, 100, 150, 300, or 400) contiguous amino acids of SEQ ID NO:2 or SEQ ID NO:4 or the polypeptide encoded by the cDNA of ATCC Accession Number .

The invention includes a nucleic acid molecule which encodes a naturally occurring allelic variant of a polypeptide comprising the amino acid sequence of SEQ ID NO:2 or SEQ ID NO:4 or an amino acid sequence encoded by the cDNA of ATCC Accession Number , wherein the nucleic acid molecule hybridizes to a nucleic acid molecule comprising SEQ ID NO:1 or SEQ ID NO:3 under stringent conditions.

Also within the invention are: an isolated FTHMA-070 protein having an amino acid sequence that is at least about 65%, preferably 75%, 85%, 95%, or 98% identical to the amino acid sequence of SEQ ID NO:4 (mature human FTHMA-070) or the amino acid sequence of SEQ ID NO:2 (immature human FTHMA-070).

Also within the invention are: an isolated FTHMA-070 protein which is encoded by a nucleic acid molecule having a nucleotide sequence that is at least about 65%, preferably 75%, 85%, or 95% identical to SEQ ID NO:3 or the cDNA of ATCC ; and an isolated FTHMA-070 protein which is encoded by a nucleic acid molecule having a nucleotide sequence which hybridizes under stringent hybridization conditions to a nucleic acid molecule having the nucleotide sequence of SEQ ID NO:3 or the non-coding strand of the cDNA of ATCC .

Also within the invention is a polypeptide which is a naturally occurring allelic variant of a polypeptide that includes the amino acid sequence of SEQ ID NO:2 or SEQ ID NO:4 or an amino acid sevence encoded by the cDNA insert of the plasmid deposited with ATCC as Accession Number , wherein the polypeptide is encoded by a nucleic acid molecule which hybridizes to a nucleic acid molecule comprising SEQ ID NO:1 or SEQ ID NO:3 under stringent conditions;

Another embodiment of the invention features FTHMA-070 nucleic acid molecules which specifically detect FTHMA-070 nucleic acid molecules. For example, in one embodiment, a FTHMA-070 nucleic acid molecule hybridizes under stringent conditions to a nucleic acid molecule comprising the nucleotide sequence of SEQ ID NO:1, SEQ ID NO:3, or the cDNA of ATCC , or a complement thereof. In another embodiment, the FTHMA-070 nucleic acid molecule is at least 300 (325, 350, 375, 400, 425, 450, 500, 550, 600, 650, 700, 800, 900, 1000, or 1200) nucleotides in length and hybridizes under stringent conditions to a nucleic acid molecule comprising the nucleotide sequence shown in SEQ ID NO:1, SEQ ID NO:3, the cDNA of ATCC , or a complement thereof. In another embodiment, the invention provides an isolated nucleic acid molecule which is antisense to the coding strand of a FTHMA-070 nucleic acid.

Another aspect of the invention provides a vector, e.g., a recombinant expression vector, comprising a FTHMA-070 nucleic acid molecule of the invention. In another embodiment the invention provides a host cell containing such a vector. The invention also provides a method for producing FTHAM-070 protein by culturing, in a suitable medium, a host cell of the invention containing a recombinant expression vector such that a FTHMA-070 protein is produced.

Another aspect of this invention features isolated or recombinant FTHMA-070 proteins and polypeptides. Preferred FTHMA-070 proteins and.polypeptides possess at least one biological activity possessed by naturally occurring human FTHMA-070, e.g., the ability to form protein:protein interactions with other proteins.

The FTHMA-070 proteins of the present invention, or biologically active portions thereof, can be operatively linked to a non-FTHMA-070 polypeptide (e.g., heterologous amino acid sequences) to form FTHMA-070 fusion proteins. The invention further features antibodies that specifically bind FTHMA-070 proteins, such as monoclonal or polyclonal antibodies. In addition, the FTHMA-070 proteins or biologically active portions thereof can be incorporated into pharmaceutical compositions, which optionally include pharmaceutically acceptable carriers.

In another aspect, the present invention provides a method for detecting the presence of FTHMA-070 activity or expression in a biological sample by contacting the biological sample with an agent capable of detecting an indicator of **FTHMA-070** activity such that the presence of **FTHMA-070** activity is detected in the biological sample.

In another aspect, the invention provides a method for modulating **FTHMA-070** activity comprising contacting a cell with an agent that modulates (inhibits or stimulates) FTHMA-070 activity or expression such that FTHMA-070 activity or expression in the cell is modulated. In one embodiment, the agent is an antibody that specifically binds to FTHMA-070 protein. In another embodiment, the agent modulates expression of FTHMA-070 by modulating transcription of a FTHMA-070 gene, splicing of a FTHMA-070 mRNA, or translation of a FTHMA-070 mRNA. In yet another embodiment, the agent is a nucleic acid molecule having a nucleotide sequence that is antisense to the coding strand of the FTHMA-070 mRMA or the FTHMA-070 gene.

In one embodiment, the methods of the present invention are used to treat a subject having a disorder characterized by aberrant FTHMA-070 protein or nucleic acid expression or activity by administering an agent which is a FTHMA-070 modulator to the subject. In one embodiment, the FTHMA-070 modulator is a FTHMA-070 protein. In another embodiment the FTHMA-070 modulator is a FTHMA-070 nucleic acid molecule. In other embodiments, the FTHMA-070 modulator is a peptide, peptidomimetic, or other small molecule.

The present invention also provides a diagnostic assay for identifying the presence or absence of a genetic lesion or mutation characterized by at least one of: (i) aberrant modification or mutation of a gene encoding a FTHMA-070 protein; (ii) mis-regulation of a gene encoding a FTHMA-070 protein; and (iii) aberrant post-translational modification of a FTHMA-070 protein, wherein a wild-type form of the gene encodes a protein with a FTHMA-070 activity.

In another aspect, the invention provides a method for identifying a compound that binds to or modulates the activity of a FTHMA-070 protein. In general, such methods entail measuring a biological activity of a FTHMA-070 protein in the presence and absence of a test compound and identifying those compounds which alter the activity of the FTHMA-070 protein.

The invention also features methods for identifying a compound which modulates the expression of FTHMA-070 by measuring the expression of FTHKA-070 in the presence and absence of a compound.

The invention features a nucleic acid molecule which is at least 45% (or 55%, 65%, 75%, 85%, 95%, or 98%) identical to the nucleotide sequence shown in SEQ ID NO:5, or SEQ ID NO:7, or the nucleotide sequence of the cDNA insert of the plasmid deposited with ATCC as Accession Number (the "cDNA of ATCC "), or a complement thereof. The invention features a nucleic acid molecule which includes a fragment of at least 300 (325, 350, 375, 400, 425, 450, 500, 550, 600, 650, 700, 800, 900, 1000, or 1290) nucleotides of the nucleotide sequence shown in SEQ ID NO:5, or SEQ ID NO:7, or the nucleotide sequence of the cDNA ATCC , or a complement thereof.

The invention also features a nucleic acid molecule which includes a nucleotide sequence encoding a protein having an amino acid sequence that is at least 45% (or 55%, 65%, 75%, 85%, 95%, or 98%) identical to the amino acid sequence of SEQ ID NO:6, SEQ ID NO:8, or the amino acid sequence encoded by the cDNA of ATCC . In a preferred embodiment, a T85 nucleic acid molecule has the nucleotide sequence shown SEQ ID NO:5, or SEQ ID NO:7, or the nucleotide sequence of the cDNA of ATCC .

Also within the invention is a nucleic acid molecule which encodes a fragment of a polypeptide having the amino acid sequence of SEQ ID NO:6 or SEQ ID NO:8, the fragment including at least 15 (25, 30, 50, 100, 150, 300, or 400) contiguous amino acids of SEQ ID NO:6 or SEQ ID NO:8 or the polypeptide encoded by the cDNA of ATCC Accession Number .

The invention includes a nucleic acid molecule which encodes a naturally occurring allelic variant of a polypeptide comprising the amino acid sequence of SEQ ID NO:6 or SEQ ID NO:8 or an amino acid sequence encoded by the cDNA of ATCC Accession Number , wherein the nucleic acid molecule hybridizes to a nucleic acid molecule comprising SEQ ID NO:5 or SEQ ID NO:7 under stringent conditions.

Also within the invention are: an isolated T85 protein having an amino acid sequence that is at least about 65%, preferably 75%, 85%, 95%, or 98% identical to the amino acid sequence of SEQ ID NO:8 (mature human T85) or the amino acid sequence of SEQ ID NO:6 (immature human T85); and an isolated T85 protein having an amino acid sequence that is at least about 85%, 95%, or 98% identical to one or more of the fibronectin type III domains and Ig superfamily domains described herein.

Also within the invention are: an isolated T85 protein which is encoded by a nucleic acid molecule having a nucleotide sequence that is at least about 65%, preferably 75%, 85%, or 95% identical to SEQ ID NO:7 or the cDNA of ATCC ; an isolated T85 protein which is encoded by a nucleic acid molecule having a nucleotide sequence at least about 65% preferably 75%, 85%, or 95% identical a fibronectin III or Ig superfamily domain encoding portion of SEQ ID NO:5; and an isolated T85 protein which is encoded by a nucleic acid molecule having a nucleotide sequence which hybridizes under stringent hybridization conditions to a nucleic acid molecule having the nucleotide sequence of SEQ ID NO:7 or the non-coding strand of the cDNA of ATCC .

Also within the invention is a polypeptide which is a naturally occurring allelic variant of a polypeptide that includes the amino acid sequence of SEQ ID NO:6 or SEQ ID NO: 8 or an amino acid sequence encoded by the cDNA insert of the plasmid deposited with ATCC as Accession Number , wherein the polypeptide is encoded by a nucleic acid molecule which hybridizes to a nucleic acid molecule comprising SEQ ID NO:5 or SEQ ID NO:7 under stringent conditions;

Another embodiment of the invention features T85 nucleic acid molecules which specifically detect T85 nucleic acid molecules. For example, in one embodiment, a T85 nucleic acid molecule hybridizes under stringent conditions to a nucleic acid molecule comprising the nucleotide sequence of SEQ ID NO:5, SEQ ID NO:7, or the cDNA of ATCC , or a complement thereof. In another embodiment, the T85 nucleic acid molecule is at least 300 (325, 350, 375, 400, 425, 450, 500, 550, 600, 650, 700, 800, 900, 1000, or 1290) nucleotides in length and hybridizes under stringent conditions to a nucleic acid molecule comprising the nucleotide sequence shown in SEQ ID NO:5, SEQ ID NO:7, the cDNA of ATCC , or a complement thereof. In a preferred embodiment, an isolated T85 nucleic acid molecule comprises a nucleodtide sequence of SEQ ID NO:5 which encodes a fibronectin type III domain, or a complement thereof. In another preferred embodiment, an isolated T85 nucleic acid molecule comprises a nucleotide of SEQ ID NO:5 which encodes an Ig superfamily domain, or a complement thereof. In another embodiment, the invention provides an isolated nucleic acid molecule which is antisense to the coding strand of a T85 nucleic acid.

Another aspect of the invention provides a vector, e.g., a recombinant expression vector, comprising a T85 nucleic acid molecule of the invention. In another embodiment the invention provides a host cell containing such a vector. The invention also provides a method for producing T85 protein by culturing, in a suitable medium, a host cell of the invention containing a recombinant expression vector such that a T85 protein is produced.

Another aspect of this invention features isolated or recombinant T85 proteins and polypeptides. Preferred T85 proteins and polypeptides possess at least one biological activity possessed by naturally occurring human T85, e.g., the ability to form protein:protein interactions with other proteins.

The T85 proteins of the present invention, or biologically active portions thereof, can be operatively linked to a non-T85 polypeptide (e.g., heterologous amino acid sequences) to form T85 fusion proteins. The invention further features antibodies that specifically bind T85 proteins, such as monoclonal or polyclonal antibodies. In addition, the T85 proteins or biologically active portions thereof can be incorporated into pharmaceutical compositions, which optionally include pharmaceutically acceptable carriers.

In another aspect, the present invention provides a method for detecting the presence of T85 activity or expression in a biological sample by contacting the biological sample with an agent capable of detecting an indicator of T85 activity such that the presence of T85 activity is detected in the biological sample.

In another aspect, the invention provides a method for modulating T85 activity comprising contacting a cell with an agent that modulates (inhibits or stimulates) T85 activity or expression such that T85 activity or expression in the cell is modulated. In one embodiment, the agent is an antibody that specifically binds to T85 protein. In another embodiment, the agent modulates expression of T85 by modulating transcription of a T85 gene, splicing of a T85 mRNA, or translation of a T85 mRNA. In yet another embodiment, the agent is a nucleic acid molecule having a nucleotide sequence that is antisense to the coding strand of the T85 mRNA or the T85 gene.

In one embodiment, the methods of the present invention are used to treat a subject having a disorder characterized by aberrant T85 protein or nucleic acid expression or activity by administering an agent which is a T85 modulator to the subject. In one embodiment, the T85 modulator is a T85 protein. In another embodiment the T85 modulator is a T85 nucleic acid molecule. In other embodiments, the T85 modulator is a peptide, peptidomimetic, or other small molecule.

The present invention also provides a diagnostic assay for identifying the presence or absence of a genetic lesion or mutation characterized by at least one of: (i) aberrant modification or mutation of a gene encoding a T85 protein; (ii) mis-regulation of a gene encoding a T85 protein; and (iii) aberrant post-translational modification of a T85 protein, wherein a wild-type form of the gene encodes a protein with a T85 activity.

In another aspect, the invention provides a method for identifying a compound that binds to or modulates the activity of a T85 protein. In general, such methods entail measuring a biological activity of a T85 protein in the presence and absence of a test compound and identifying those compounds which alter the activity of the T85 protein.

The invention also features methods for identifying a compound which modulates the expression of T85 by measuring the expression.of T85 in the presence and absence of a compound.

All patents, patent applications, and publications cited herein are incorporated by reference.

Other features and advantages of the invention will be apparent from the following detailed description and claims.

### Brief Description of the Drawings

Figure 1 depicts the cDNA sequence (SEQ ID NO:1) and predicted amino acid sequence (SEQ ID NO:2) of human FTHMA-070. The open reading frame of SEQ ID NO:1 extends from nucleotide 73 to nucleotide 1275 (SEQ ID NO:3).

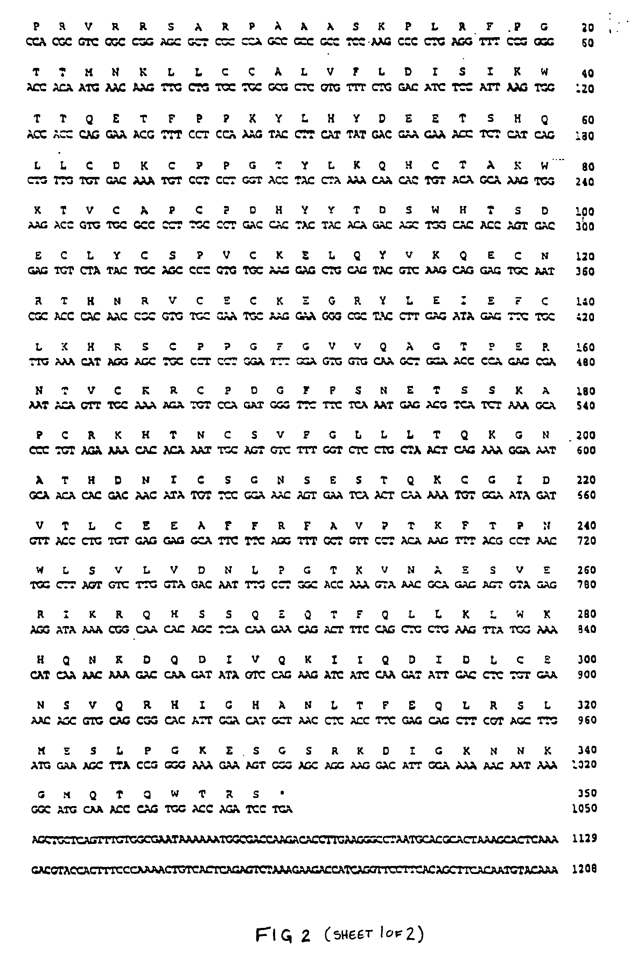

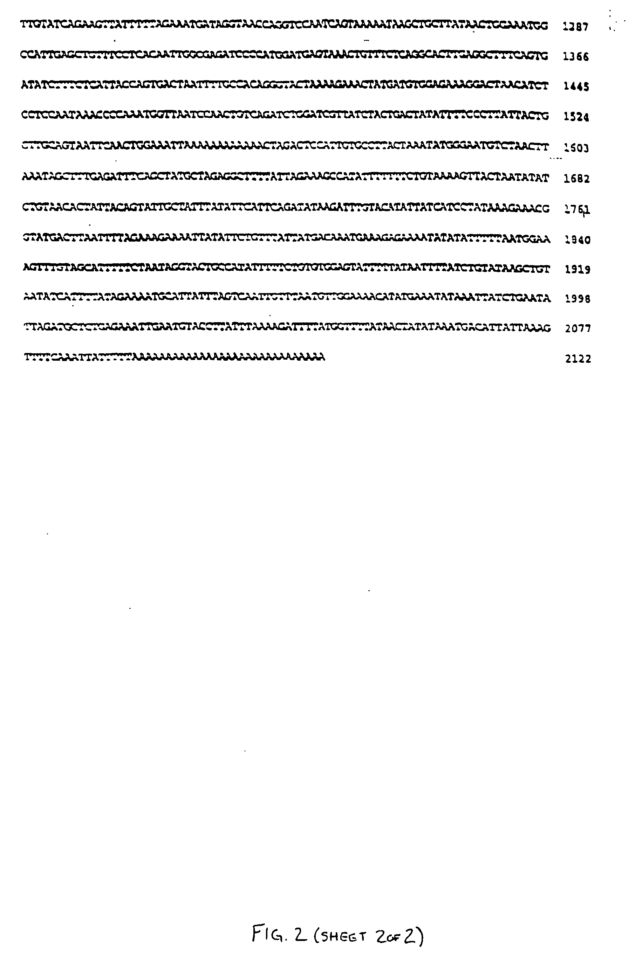

Figure 2 depicts an a partial cDNA sequence (SEQ ID NO:16) and predicted amino acid sequence (SEQ ID NO:17) of human FTHMA-070.
Figure 3 depicts the cDNA sequence (SEQ ID NO:5) and predicted amino acid sequence (SEQ ID NO:6) of human T85. The open reading frame of SEQ ID NO:5 extends from nucleotide 958 to nucleotide 3216 (SEQ ID NO:7).
Figure 4 is a hydropathy plot of T85. The location of cysteines (cys; first set of vertical bars immediately below the plot) and N-glycosylation sites (Ngly; second set of vertical bars below the plot) are indicated. Relative hydrophilicity is shown above the dotted line, and relative hydrophobicity is shown below the line.
Figure 5 is a series of sequence comparisons between portions of the amino acid sequence of T85 and a fibronectin type III domain (PFOOO41) and an Ig superfamily domain (PF00047) derived from a hidden Markov model.
Figure 6 depicts a comparison of the amino acid sequence of T85 and human Robo protein.

### Detailed Description of the Invention

The present invention is based, in part, on the discovery of a cDNA molecule encoding human FTHMA-070, a member of the TNF receptor superfamily.

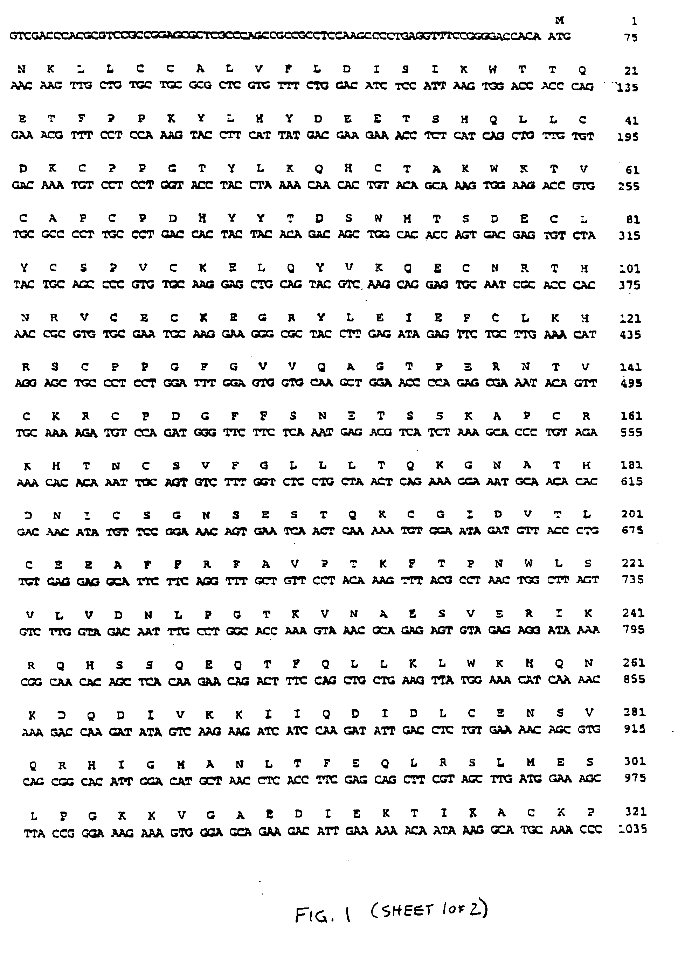

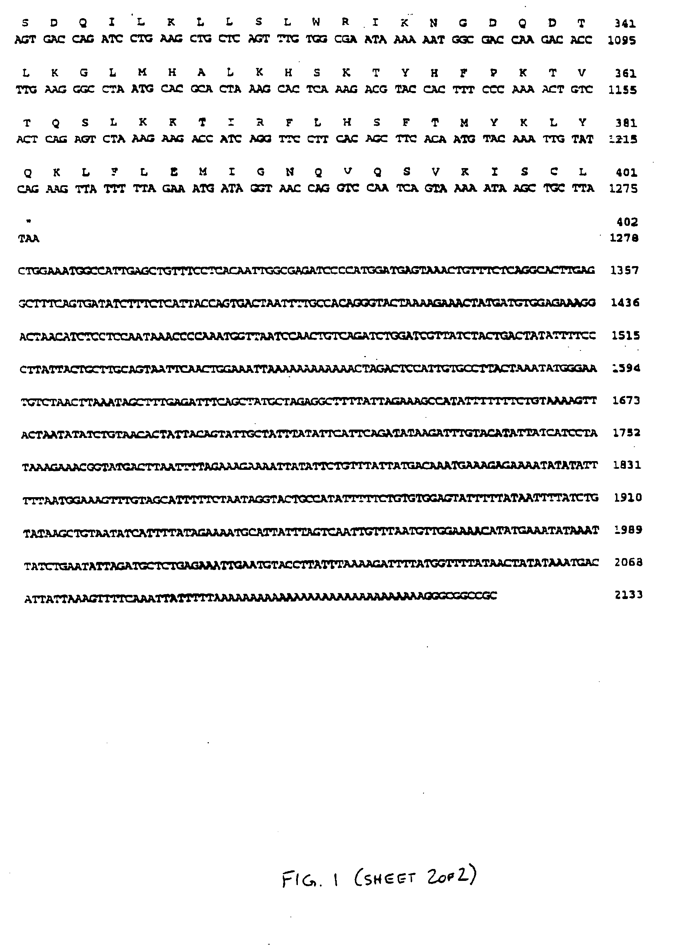

A nucleotide sequence encoding a human FTHMA-070. protein is shown in Figure 1 (SEQ ID NO:1; SEQ ID NO:3 includes the open reading frame only). A predicted amino acid sequence of FTHMA-070 protein is also shown in Figure 1 (SEQ ID NO: 2).

The FTHMA-070 cDNA of Figure 1 (SEQ ID NO:1), which is approximately 2133 nucleotides long including untranslated regions, encodes a 401 amino acid protein. A plasmid containing a cDNA encoding human FTHMA-070 (with the cDNA insert name of ) was deposited with American Type Culture Collection (ATCC), Rockville, Maryland on and assigned Accession Number . This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

Human FTHMA-070 is one member of a family of molecules (the "FTHKA-070 family") having certain conserved structural and functional features. The term "family" when referring to the protein and nucleic acid molecules of the invention is intended to mean two or more proteins or nucleic acid molecules having a common structural domain and having sufficient amino acid or nucleotide sequence identity as defined herein. Such family members can be naturally occurring and can be from either the same or different species. For example, a family can contain a first protein of human origin and a homologue of that protein of murine origin, as well as a second, distinct protein of human origin and a murine homologue of that protein. Members of a family may also have common functional characteristics.

As used interchangeably herein a "FTHMA-070 activity", "biological activity of FTHMA-070" or "functional activity of FTHMA-070", refers to an activity exerted by a FTHMA-070 protein, polypeptide or nucleic acid molecule on a FTHMA-070 responsive cell as determined *in vivo,* or *in vitro*, according to standard techniques. A FTHMA-070 activity can be a direct activity, such as an association with or an enzymatic activity on .a second protein or an indirect activity.

Accordingly, another embodiment of the invention features isolated FTHMA-070 proteins and polypeptides having a FTHMA-070 activity.

Yet another embodiment of the invention features FTHMA-070 molecules which contain a signal sequence. Generally, a signal sequence (or signal peptide) is a peptide containing about 20 amino acids which occurs at the extreme N-terminal end of secretory and integral membrane proteins and which contains large numbers of hydrophobic amino acid residues and serves to direct a protein containing such a sequence to a lipid bilayer.

The present invention is also based, in part, on the discovery of a cDNA molecule encoding human T85, a protein which appears to be a secreted (non-membrane bound) form of human Robo protein, a protein which is a nerve axon guidance receptor.

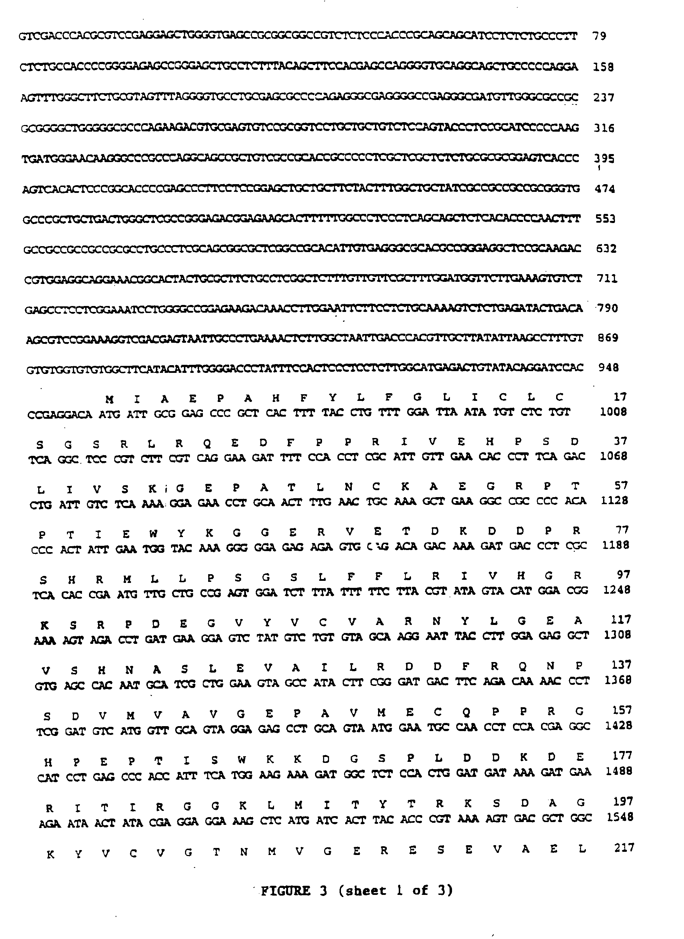

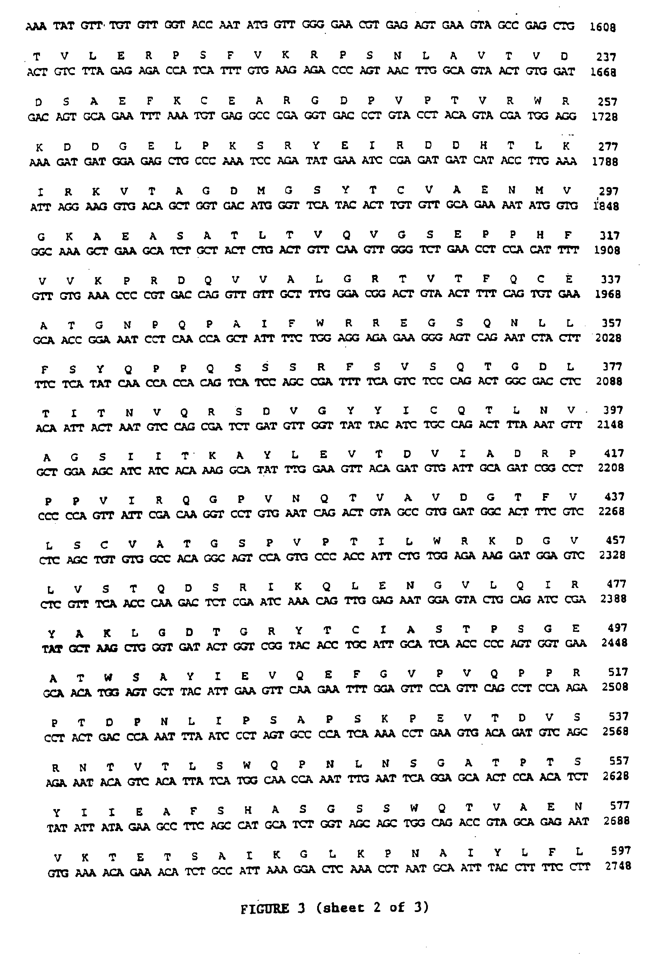

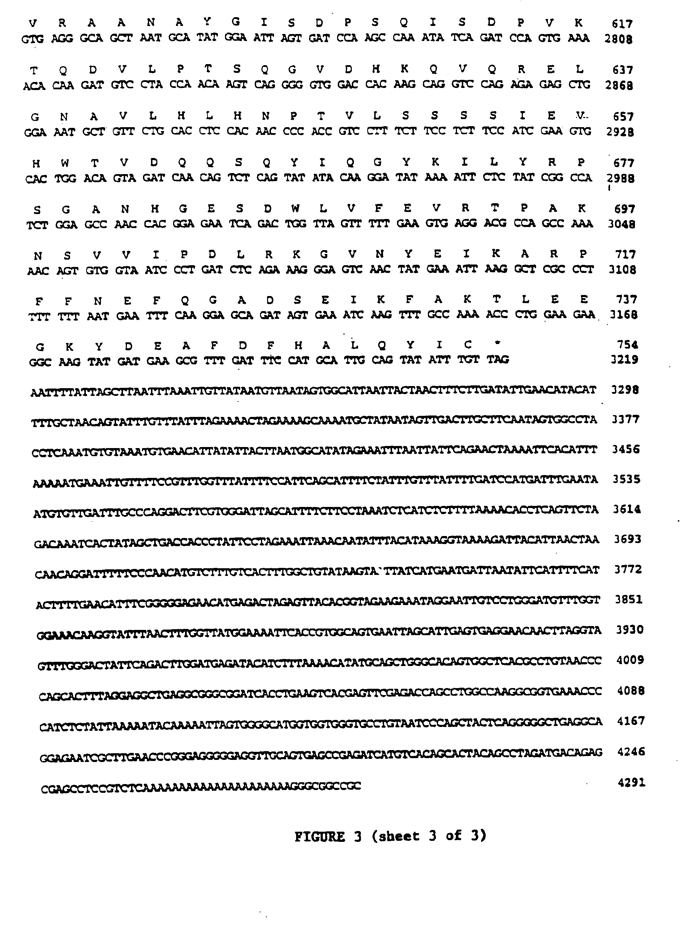

A nucleotide sequence encoding a human T85 protein is shown in Figure 3 (SEQ ID NO:5; SEQ ID NO:7 includes the open reading frame only). A predicted amino acid sequence of FTMA-070 protein is also shown in Figure 3 (SEQ ID NO: 6).

The FTMA-070 cDNA of Figure 3 (SEQ ID NO:5), which is approximately 4291 nucleotides long including untranslated regions, encodes a 753 amino acid protein. A plasmid containing a cDNA encoding human FTMA-070 (with the cDNA insert name of )was deposited with American Type Culture Collection (ATCC), Rockville, Maryland on and assigned Accession Number . This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

Human T85 is one member of a family of molecules (the "T85 family") having certain conserved structural and functional features. The term "family" when referring to the protein and nucleic acid molecules of the invention is intended to mean two or more proteins or nucleic acid molecules having a common structural domain and having sufficient amino acid or nucleotide sequence identity as defined herein. Such family members can be naturally occurring and can be from either the same or different species. For example, a family can contain a first protein of human origin and a homologue of that protein of murine origin, as well as a second, distinct protein of human origin and a murine homologue of that protein. Members of a family may also have common functional characteristics.

In one embodiment, a T85 protein includes a fibronectin type III domain having at least about 65%, preferably at least about 75%, and more preferably about 85%, 95%, or 98% amino acid sequence identity to a fibronectin type III domain of SEQ ID NO: 9, or 10.

In one embodiment, a T85 protein includes an Ig superfamily domain having at least about 65%, preferably at least about 75%, and more preferably about 85%, 95%, or 98% amino acid sequence identity to an Ig superfamily domain of SEQ ID NO:11, 12, 13, 14, or 15.

Preferred TB5 polypeptides of the present invention have an amino acid sequence sufficiently identical to a sequence identity to a fibronectin type III domain of SEQ ID NO: 9, or 10 or an Ig superfamily domain of SEQ ID NO:11, 12, 13, 14, or 15. As used herein, the term "sufficiently identical" refers to a first amino acid or nucleotide sequence which contains a sufficient or minimum number of identical or equivalent (e.g., an amino acid residue which has a similar side chain) amino acid residues or nucleotides to a second amino acid or nucleotide sequence such that the first and second amino acid or nucleotide sequences have a common structural domain and/or common functional activity. For example, amino acid or nucleotide sequences which contain a common structural domain having about 65% identity, preferably 75% identity, more preferably 85%, 95%, or 98% identity are defined herein as sufficiently identical.

As used interchangeably herein a "T85 activity", "biological activity of T85" or "functional activity of T85", refers to an activity exerted by a T85 protein, polypeptide or nucleic acid molecule on a T85 responsive cell as determined in *vivo,* or *in vitro,* according to standard techniques. A T85 activity can be a direct activity, such as an association with or an enzymatic activity on a second protein.

Accordingly, another embodiment of the invention features isolated T85 proteins and polypeptides having a T85 activity.

Yet another embodiment of the invention features T85 molecules which contain a signal sequence. Generally, a signal sequence (or signal peptide) is a peptide containing about 20 amino acids which occurs at the extreme N-terminal end of secretory and integral membrane proteins and which contains large numbers of hydrophobic amino acid residues and serves to direct a protein containing such a sequence to a lipid bilayer.

Various aspects of the invention are described in further detail in the following subsections.

Various aspects of the invention are described in further detail in the following subsections.

### I. Isolated Nucleic Acid Molecules

One aspect of the invention pertains to isolated nucleic acid molecules that encode FTHMA-070 or T85 proteins or biologically active portions thereof, as well as nucleic acid molecules sufficient for use as hybridization probes to identify FTHMA-070 or T85-encoding nucleic acids (e.g., FTHMA-070 or T85 mRNA) and fragments for use as PCR primers for the amplification or mutation of FTHMA-070 or T85 nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid. Preferably, an "isolated" nucleic acid is free of sequences (preferably protein encoding sequences) which naturally flank the nucleic acid (i.e., sequences located at the 5' and 3' ends of the nucleic acid) in the genomic DNA of the organism from which the nucleic acid is derived. For example, in various embodiments, the isolated FTHMA-070 or T85 nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleic acid molecule having the nucleotide sequence of SEQ ID NO:1, SEQ ID NO:3, SEQ ID NO:5 or SEQ ID NO:6 or the cDNA of ATCC or , or a complement of any of these nucleotide sequences, can be isolated using standard molecular biology techniques and the sequence information provided herein. Using all or portion of the nucleic acid sequences described herein or the cDNA of ATCC as a hybridization probe, FTHMA-070 or T85 nucleic acid molecules can be isolated using standard hybridization and cloning techniques (e.g., as described in Sambrook et al., eds., *Molecular Cloning*: A *Laboratory Manual*. 2nd, *ed*., Cold Spring *Harbor* Laboratory, Cold Spring Harbor Laboratory Press, Cold Spring Harbor, NY, 1999).

A nucleic acid of the invention can be amplified using cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to FTHMA-070 or T85 nucleotide sequences can be prepared by standard synthetic techniques, e.g., using an automated DNA synthesizer.

In other preferred embodiments, an isolated nucleic acid molecule of the invention comprises a nucleic acid molecule which is a complement of the nucleotide sequence described herein (e.g., SEQ ID NO:1, SEQ ID NO:3, SEQ ID NO:5 or SEQ ID NO:6) or the cDNA of ATCC , or a portion thereof. A nucleic acid molecule which is complementary to a given nucleotide sequence is one which is sufficiently complementary to the given nucleotide sequence that it can hybridize to the given nucleotide sequence thereby forming a stable duplex.

Moreover, a nucleic acid molecule of the invention can comprise only a portion of a nucleic acid sequence encoding FTHMA-070 or T85, for example, a fragment which can be used as a probe or primer or a fragment encoding a biologically active portion of FTHMA-070 or T85. The nucleotide sequence determined from the cloning of the human FTHMA-070 or T85 gene allows for the generation of probes and primers designed for use in identifying and/or cloning FTHMA-070 or T85 homologues in other cell types, e.g., from other tissues, as well as FTHMA-070 or T85 homologues from other mammals. The probe/primer typically comprises substantially purified oligonucleotide. The oligonucleotide typically comprises a region of nucleotide sequence that hybridizes under stringent conditions to at least about 12, preferably about 25, more preferably about 50, 75, 100, 125, 150, 175, 200, 250, 300, 350 or 400 consecutive nucleotides of the sense or anti-sense sequence of SEQ ID NO:1, SEQ ID NO:3, SEQ ID NO:5, SEQ ID NO:6 or the cDNA of ATCC or of a naturally occurring mutant of SEQ ID NO:1, SEQ ID NO:3, SEQ ID NO:5, SEQ ID NO:6 or the cDNA of ATCC .

Probes based on the human FTHMA-070 or T85 nucleotide sequence can be used to detect transcripts or genomic sequences encoding the same or identical proteins. The probe comprises a label group attached thereto, e.g., a radioisotope, a fluorescent compound, an enzyme, or an enzyme co-factor. Such probes can be used as a part of a diagnostic test kit for identifying cells or tissue which mis-express a FTHMA-070 or T85 protein, such as by measuring a level of a FTHMA-070 or T85-encoding nucleic acid in a sample of cells from a subject, e.g., detecting FTHMA-070 or T85 mRNA levels or determining whether a genomic FTHMA-070 or T85 gene has been mutated or deleted.

A nucleic acid fragment encoding a "biologically active portion of FTHMA-070 or T85" can be prepared by isolating a portion of SEQ ID NO:1, SEQ ID NO:3, SEQ ID NO:5, SEQ ID NO:6 or the nucleotide sequence of the cDNA of ATCC which encodes a polypeptide having a FTHMA-070 or T85 biological activity, expressing the encoded portion of FTHMA-070 or T85 protein (e.g., by recombinant expression in vitro) and assessing the activity of the encoded portion of FTHMA-070 or T85.

The invention further encompasses nucleic acid molecules that differ from the nucleotide sequence of SEQ ID NO:1, SEQ ID NO:3, SEQ ID NO:5, SEQ ID NO:6 or the cDNA of ATCC due to degeneracy of the genetic code and thus encode the same FTHMA-070 or T85 protein as that encoded by the nucleotide sequence shown in SEQ ID NO:1, SEQ ID NO:3, SEQ ID NO:5, SEQ ID NO:6 or the cDNA of ATCC .

In addition to the human FTHMA-070 or T85 nucleotide sequence shown in SEQ ID NO:1, SEQ ID NO:3, SEQ ID NO:5, SEQ ID NO:6 or the cDNA of ATCC , it will be appreciated by those skilled in the art that DNA sequence polymorphisms that lead to changes in the amino acid sequences of FTHMA-070 or T85 may exist within a population (e.g., the human population). Such genetic polymorphism in the FTHMA-070 or T85 gene may exist among individuals within a population due to natural allelic variation. As used herein, the terms "gene" and "recombinant gene" refer to nucleic acid molecules comprising an open reading frame encoding a FTHMA-070, or T85 protein, preferably a mammalian FTHMA-070 or T85 protein. Such natural allelic variations can typically result in 1-5% variance in the nucleotide sequence of the FTHMA-070 or T85 gene. Any and all such nucleotide variations and resulting amino acid polymorphisms in FTHMA-070 or T85 that are the result of natural allelic variation and that do not alter the functional activity of FTHMA-070 or T85 are intended to be within the scope of the invention. Moreover, nucleic acid molecules encoding FTHMA-070 or T85 proteins from other species (FTHMA-070 or T85 homologues), which have a nucleotide sequence which differs from that of a human FTHMA-070 or T85, are intended to be within the scope of the invention. Nucleic acid molecules corresponding to natural allelic variants and homologues of the FTHMA-070 or T85 cDNA of the invention can be isolated based on their identity to the human FTHMA-070 or T85 nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 300 (325, 350, 375, 400, 425, 450, 500, 550, 600, 650, 700, 800, 900, 1000, or 1290) nucleotides in length and hybridizes under stringent conditions to the nucleic acid molecule comprising the nucleotide sequence, preferably the coding sequence, of SEQ ID NO:1, SEQ ID NO:3, SEQ ID NO:5, SEQ ID NO:6 or the cDNA of ATCC .

As used herein, the term "hybridizes under stringent conditions", is intended to describe conditions for hybridization and washing under which nucleotide sequences at least 60% (65%, 70%, preferably 75%) identical to each other typically remain hybridized to each other. Such stringent conditions are known to those skilled in the art and can be found in *current Protocols in Molecular Biology*, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to the sequence of SEQ ID NO:1, SEQ ID NO:3, SEQ ID NO:5, SEQ ID NO:6, or the cDNA of ATCC corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally-occurring allelic variants of the FTHMA-070 or T85 sequence that may exist in the population, the skilled artisan will further appreciate that changes can be introduced by mutation into the nucleotide sequence of SEQ ID NO:1, SEQ ID NO:3, SEQ ID NO:5, SEQ ID NO:6 or the cDNA of ATCC , thereby leading to changes in the amino acid sequence of the encoded FTHMA-070 or T85 protein, without altering the functional ability of the FTHMA-070 or T85 protein. For example, one can make nucleotide substitutions leading to amino acid substitutions at "non-essential" amino acid residues. A "non-essential" amino acid residue is a residue that can be altered from the wild-type sequence of FTHMA-070 or T85 (e.g., the sequence of SEQ ID NO:2) without altering the biological activity, whereas an "essential" amino acid residue is required for biological activity. For example, amino acid residues that are conserved among the FTHMA-070 or T85 proteins of various species are predicted to be particularly unamenable to alteration.

For example, preferred T85 proteins of the present invention, contain at least one fibronectin III or Ig superfamily domain. Such conserved domains are less likely to be amenable to mutation. Other amino acid residues, however, (e.g., those that are not conserved or only semi-conserved among T85 of various species) may not be essential for activity and thus are likely to be amenable to alteration.

Accordingly, another aspect of the invention pertains to nucleic acid molecules encoding FTHMA-070 or T85 proteins that contain changes in amino acid residues that are not essential for activity. Such FTHMA-070 or T85 proteins differ in amino acid sequence from SEQ ID NO:2 or SEQ ID NO:6, respectively, yet retain biological activity.

An isolated nucleic acid molecule encoding a FTHMA-070 or T8S protein having a sequence which differs from that of SEQ ID NO:2 or SEQ ID NO:6, respectively, can be created by introducing one or more nucleotide substitutions, additions or deletions into the nucleotide sequence of SEQ ID NO-1, SEQ ID NO:3, SEQ ID NO:5, SEQ ID NO:6, or the cDNA of ATCC such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in FTHMA-070 or T85 is preferably replaced with another amino acid residue from the same side chain family. Alternatively, mutations can be introduced randomly along all or part of a FTHMA-070 or T85 coding sequence, such as by saturation mutagenesis, and the resultant mutants can be screened for FTHMA-070 or T85 biological activity to identify mutants that retain activity. Following mutagenesis, the encoded protein can be expressed recombinantly and the activity of the protein can be determined.

In a preferred embodiment, a mutant FTHMA-070 or T85 protein can be assayed for the ability to form protein:protein interactions with other proteins.

The present invention encompasses antisense nucleic acid molecules, i.e., molecules which are complementary to a sense nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire FTHMA-070 or T85 coding strand, or to only a portion thereof, e.g., all or part of the protein coding region (or open reading frame). An antisense nucleic acid molecule can be antisense to a noncoding region of the coding strand of a nucleotide sequence encoding FTHMA-070 or T85. The noncoding regions ("5' and 3' untranslated regions") are the 5' and 3' sequences which flank the coding region and are not translated into amino acids.

Given the coding strand sequences encoding FTHMA-070 or T85 disclosed herein, antisense nucleic acids of the invention can be designed according to the rules of Watson and Crick base pairing. The antisense nucleic acid molecule can be complementary to the entire coding region of FTHMA-070 or T85 mRNA, but more preferably is an oligonucleotide which is antisense to only a portion of the coding or noncoding region of FTHMA-070 or T85 mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated in situ such that they hybridize with or bind to cellular mRNA and/or genomic DNA encoding a FTHMA-070 or T85 protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anomeric nucleic acid molecule forms specific double-stranded hybrids with complementary RNA in which, contrary to the usual **β**-units, the strands run parallel to each other (Gaultier et al. (1987) Nucleic Acids. *Res.* 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) *FEBS Lett.* 215:327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically cleave FTHMA-070 or T85 mRNA transcripts to thereby inhibit translation of FTHMA-070 or T85 mRNA. A ribozyme having specificity for a FTHMA-070 or T85-encoding nucleic acid can be designed based upon the nucleotide sequence of a FTHMA-070 or T85 cDNA disclosed herein. For example, a derivative of a Tetrahymena L-19 IVS RNA can be constructed in which the nucleotide sequence of the active site is complementary to the nucleotide sequence to be cleaved in a FTHMA-070 or T85-encoding mRNA. See, e.g., Cech et al. U.S. Patent No. 4,987,071; and Cech et al. U.S. Patent No. 5,116,742. Alternatively, FTHMA-070 or T85 mRNA can be used to select a catalytic RNA having a specific ribonuclease activity from a pool of RNA molecules. See, e.g., Bartel and Szostak (1993) *Science* 261:1411-1418.

The invention also encompasses nucleic acid molecules which form triple helical structures. For example, FTHMA-070 or T85 gene expression can be inhibited by targeting nucleotide sequences complementary to the regulatory region of the FTHMA-070 or T85 (e.g., the FTHMA-070 or T85 promoter and/or enhancers) to form triple helical structures that prevent transcription of the FTHMA-070 or T85 gene in target cells. See generally, Helene (1991) *Anticancer Drug Des*. 6 (6) :569-84; Helene (1992) *Ann. N.Y. Acad. Sci.* 660:27-36; and Maher, L.J. (1992) *Bioassays* 14(12):807-15.

In preferred embodiments, the nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup et al. (1996) Bioorganic & *Medicinal Chemistry* 4 (1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al. (1996) supra; Perry-O'Keefe et al. (1996) Proc. Natl. Acad. Sci. USA 93: 14670-675.

PNAs of FTHMA-070 or T85 can be used therapeutic and diagnostic applications. For example, PNAs can be used as antisense or antigene agents for sequence-specific modulation of gene expression by, e.g., inducing transcription or translation arrest or inhibiting replication. PNAs of FTHMA-070 or T85 can also be used, e.g., in the analysis of single base pair mutations in a gene by, e.g., PNA directed PCR clamping; as 'artificial restriction enzymes when used in combination with other enzymes, e.g., S1 nucleases (Hyrup (1996) *supra;* or as probes or primers for DNA sequence and hybridization (Hyrup (1996) supra; Perry-O' Keefe et al. (1996) Proc. Natl. Acad. Sci. USA 93: 14670-675).

In another embodiment, PNAs of FTHMA-070 or T85 can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of FTHMA-070 or T85 can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) supra). The synthesis of PNA-DNA chimeras can be performed as described in Hyrup (1996) supra and Finn et al. (1996) Nucleic Acids Research 24(17):3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag et al. (1989) Nucleic Acid Res. 17:5973-88). PNA monomers are then coupled in a stepwise manner to produce a chimeric molecule with a 5' PNA segment and a 3' DNA segment (Finn et al. (1996) Nucleic *Acids* Research 24(17):3357-63). Alternatively, chimeric molecules can be synthesized with a 5' DNA segment and a 3' PNA segment (Peterser et al. (1975) Bioorganic Med. *Chem.* Lett. 5:1119-11124).

In other embodiments, the oligonucleotide may include other appended groups such as peptides (e.g., for targeting host cell receptors in vivo), or agents facilitating transport across the cell membrane (see, e.g., Letsinger et al. (1989) *Proc. Natl. Acad. Sci. USA* 86:6553-6556; Lemaitre et al. (1987) Proc. *Natl.* Acad. *Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (*See*, *e.g.*, Krol et al. (1988) *Bio*/*Techniques* 6:958-976) or intercalating agents (*See*, *e.g.*, Zon (1988) *Pharm. Res.* 5:539-549). To this end, the oligonucleotide may be conjugated to another molecule, e.g., a peptide, hybridization triggered cross-linking agent, transport agent, hybridization-triggered cleavage agent, etc.

### II. Isolated FTHMA-070 or T85 Proteins and Anti-FTHMA-070 or Anti-T85 Antibodies

One aspect of the invention pertains to isolated FTHMA-070 or T85 proteins, and biologically active portions thereof, as well as polypeptide fragments suitable for use as immunogens to raise anti-FTHMA-070 or anti-T85 antibodies. In one embodiment, native FTHMA-070 or T85 proteins can be isolated from cells or tissue sources by an appropriate purification scheme using standard protein purification techniques. In another embodiment, FTHMA-070 or T85 proteins are produced by recombinant DNA techniques. Alternative to recombinant expression, a FTHMA-070 or T85 protein or polypeptide can be synthesized chemically using standard peptide synthesis techniques.

An "isolated" or "purified" protein or biologically active portion thereof is substantially free of cellular material or other contaminating proteins from the cell or tissue source from which the FTHMA-070 or T85 protein is derived, or substantially free from chemical precursors or other chemicals when chemically synthesized. The language "substantially free of cellular material" includes preparations of FTHMA-070 or T85 protein in which the protein is separated from cellular components of the cells from which it is isolated or recombinantly produced. Thus, FTHMA-070 or T85 protein that is substantially free of cellular material includes preparations of FTHMA-070 or T85 protein having less than about 30%, 20%, 10%, or 5% (by dry weight) of non-FTHMA-070 or T85 protein (also referred to herein as a "contaminating protein"). When the FTHMA-070 or T85 protein or biologically active portion thereof is recombinantly produced, it is also preferably substantially free of culture medium, i.e., culture medium represents less than about 20%, 10%, or 5% of the volume of the protein preparation. When FTHMA-070 or T85 protein is produced by chemical synthesis, it is preferably substantially free of chemical precursors or other chemicals, i.e., it is separated from chemical precursors or other chemicals which are involved in the synthesis of the protein. Accordingly such preparations of FTHMA-070 or T85 protein have less than about 30%, 20%, 10%, 5% (by dry weight) of chemical precursors or non-FTHMA-070 or T85 chemicals.

Biologically active portions of a FTHMA-070 or T85 protein include peptides comprising amino acid sequences sufficiently identical to or derived from the amino acid sequence of the FTHMA-070 or T65 protein, which include less amino acids than the full length FTHMA-070 or TB5 proteins, and exhibit at least one activity of a FTHMA-070 or T85 protein. Typically, biologically active portions comprise a domain or motif with at least one activity of the FTHMA-070 or T85 protein. A biologically active portion of a FTHMA-070 or T85 protein can be a polypeptide which is, for example, 10, 25, 50, 100 or more amino acids in length. Preferred biologically active polypeptides include one or more identified structural domains.

Moreover, other biologically active portions, in which other regions of the protein are deleted, can be prepared by recombinant techniques and evaluated for one or more of the functional activities of a native FTHMA-070 or T85 protein. Preferred FTHMA-070 or T85 protein has the amino acid sequence shown of SEQ ID NO:2 or SEQ ID NO:6, respectively. Other useful FTHMA-070 or T85 proteins are substantially identical to SEQ ID NO:2 or SEQ ID NO:6, respectively and retain the functional activity of the reference protein yet differ in amino acid sequence due to natural allelic variation or mutagenesis.

Accordingly, a useful FTHMA-070 protein is a protein which includes an amino acid sequence at least about 45%, preferably 55%, 65%, 75%, 85%, 95%, or 99% identical to the amino acid sequence of SEQ ID NO:2 and retains the functional activity of the FTHMA-070 or T85 proteins of SEQ ID NO:2. In a preferred embodiment, the FTHMA-070 protein retains the functional activity of the FTHMA-070 protein of SEQ ID NO:2.

Accordingly, a useful T85 protein is a protein which includes an amino acid sequence at least about 45%, preferably 55%, 65%, 75% 85%, 95%, or 99% identical to the amino acid sequence of SEQ ID NO:6 and retains the functional activity of the T85 proteins of SEQ ID NO:6. In other instances, the T85 protein is a protein having an amino acid sequence 55%, 65%, 75%, 85%, 95%, or 98% identical to one of the 85 fibronectin type III or Ig superfamily domains (SEQ ID NOs:9-15). In a preferred embodiment, the T85 protein retains the functional activity of the T85 protein of SEQ ID NO:6.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions x 100).

The determination of percent homology between two sequences can be accomplished using a mathematical algorithm. A preferred, on-limiting example of a mathematical algorithm utilized for the comparison of two sequences is the algorithm of Karlin and Altschul (1990) *Proc. Nat'l Acad. Sci. USA* 87:2264-2268, modified as in Karlin and Altschul (1993) *Proc. Nat'l Acad. Sci*. *USA* 90:5873-5877. Such an algorithm is incorporated into the NBLAST and XBLAST programs of Altschul, et al. (1990) *J. Mol. Biol.* 215:403-410. BLAST nucleotide searches can be performed with the NBLAST program, score = 100, wordlength = 12 to obtain nucleotide sequences homologous to FTHMA-070 or T85 nucleic acid molecules of the invention. BLAST protein searches can be performed with the XBLAST program, score = 50, wordlength = 3 to obtain amino acid sequences homologous to FTHMA-070 or T85 protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithm utilized for the comparison of sequences is the algorithm of Myers and Miller, CABIOS (1989). ;Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, only exact matches are counted.

The invention also provides FTHMA-070 or T85 chimeric or fusion proteins. As used herein, a FTHMA-070 or T85 "chimeric protein" or "fusion protein" comprises a FTHMA-070 or T85 polypeptide operatively linked to a non-FTHMA-070 or T85 polypeptide. A FTHMA-070 polypeptide or T85 polypeptide refers to a polypeptide having an amino acid sequence corresponding to FTHMA-070 or T85, whereas a non-FTHMA-070 or non-T85 polypeptide refers to a polypeptide having an amino acid sequence corresponding to a protein which is not substantially identical to the FTHMA-070 or T85 protein, e.g., a protein which is different from the FTHMA-070 or T85 protein and which is derived from the same or a different organism. Within a FTHMA-070 or T85 fusion protein the FTHMA-070 or T85 polypeptide can correspond to all or a portion of a FTHMA-070 or T85 protein, preferably at least one biologically active portion of a FTHMA-070 or T85 protein. Within the fusion protein, the term "operatively linked" is intended to indicate that the FTHMA-070 or T85 polypeptide and the non-FTHMA-070 or T85 polypeptide are fused in-frame to each other. The non-FTHMA-070 or T85 polypeptide can be fused to the N-terminus or C-terminus of the FTHMA-070 or T8S polypeptide.

One useful fusion protein is a GST-FTHMA-070 or T85 fusion protein in which the FTHMA-070 or T85 sequences are fused to the C-terminus of the GST sequences. Such fusion proteins can facilitate the purification of recombinant FTHMA-070 or T85.

In another embodiment, the fusion protein is a FTHMA-070 or T85 protein containing a heterologous signal sequence at its N-terminus. For example, the native FTHMA-070 or T85 signal sequence can be removed and replaced with a signal sequence from another protein. In certain host cells (e.g., mammalian host cells), expression and/or secretion of FTHMA-070 or T85 can be increased through use of a heterologous signal sequence.

In yet another embodiment, the fusion protein is an FTHMA-070-immunoglobulin or T85-immunoglobulin fusion protein in which all or part of FTHMA-070 or T85 is fused to sequences derived from a member of the immunoglobulin protein family. The FTHMA-070 or T85-immunoglobulin fusion proteins of the invention can be incorporated into pharmaceutical compositions and administered to a subject to inhibit an interaction between a FTHMA-070 or T85 ligand and a FTHMA-070 or T85 protein. The FTHMA-070 or T85-immunoglobulin fusion proteins can be used to affect the bioavailability of a FTHMA-070 or T85 cognate ligand. Inhibition of the FTHMA-070 or T85 ligand/FTHMA-070 or T85 interaction may be useful therapeutically. Moreover, the FTHMA-070 or T85-immunoglobulin fusion proteins of the invention can be used as immunogens to produce anti-FTHMA-070 or T85 antibodies in a subject, to purify FTHMA-070 or T85 ligands and in screening assays to identify molecules which inhibit the interaction of FTHMA-070 or T85 with a FTHMA-070 or T85 ligand.

Preferably, a FTHMA-070 or T85 chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (*see*, *e.g.*, Current Protocols in *Molecular Biology*, Ausubel et al. eds., John Wiley & Sons: 1992). Moreover, many expression vectors are commercially available that already encode a fusion moiety (e.g., a GST polypeptide). An FTHMA-070 or T85-encoding nucleic acid can be cloned into such an expression vector such that the.fusion moiety is linked in-frame to the FTHMA-070 or T85 protein.

The present invention also pertains to variants of the FTHMA-070 or T85 proteins which function as either FTHMA-070 or T85 agonists (mimetics) or as FTHMA-070 or T85 antagonists. Variants of the FTHMA-070 or T85 protein can be generated by mutagenesis, e.g., discrete point mutation or truncation of the FTHMA-070 or T85 protein. An agonist of the FTHMA-070 or T85 protein can retain substantially the same, or a subset, of the biological activities of the naturally occurring form of the FTHMA-070 or T85 protein. An antagonist of the FTHMA-070 or T85 protein can inhibit one or more of the activities of the naturally occurring form of the FTHMA-070 or T85 protein by, for example, competitively binding to a downstream or upstream member of a cellular signaling cascade which includes the FTHMA-070 or T85 protein. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the FTHMA-070 or T85 proteins.

Variants of the FTHMA-070 or T85 protein which function as either FTHMA-070 or T85 agonists (mimetics) or as FTHMA-070 or T85 antagonists can be identified by screening combinatorial libraries of mutants, e.g., truncation mutants, of the FTHMA-070 or T85 protein for FTHMA-070 or T85 protein agonist or antagonist activity. In one embodiment, a variegated library of FTHMA-070 or T85 variants is generated by combinatorial mutagenesis at the nucleic acid level and is encoded by a variegated gene library. A variegated library of FTHMA-070 or T85 variants can be produced by, for example, enzymatically ligating a mixture of synthetic oligonucleotides into gene sequences such that a degenerate set of potential FTHMA-070 or T85 sequences is expressible as individual polypeptides, or alternatively, as a set of larger fusion proteins (e.g., for phage display) containing the set of FTHMA-070 or T85 sequences therein. There are a variety of methods which can be used to produce libraries of potential FTHMA-070 or T85 variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential FTHMA-070 or T85 sequences. Methods for synthesizing degenerate oligonucleotides are known in the art *(see, e.g.,* Narang (1983) *Tetrahedron* 39:3; Itakura et al. (1984) Annu. *Rev*. *Biochem.* 53:323; Itakura et al. (1984) *Science* 198:1056; Ike et al. (1983) Nucleic Acid Res. 11:477).

In addition, libraries of fragments of the FTHMA-070 or T85 protein coding sequence can be used to generate a variegated population of FTHMA-070 or T85 fragments for screening and subsequent selection of variants of a FTHMA-070 or T85 protein. In one embodiment, a library of coding sequence fragments can be generated by treating a double stranded PCR fragment of a FTHMA-070 or T85 coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the FTHMA-070 or T85 protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of FTHMA-070 or T85 proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify FTHMA-070 or T85 variants (Arkin and Yourvan (1992) Proc. *Natl.* Acad. *Sci. USA* 89:7811-7815; Delgrave et al. (1993) Protein Engineering 6(3):327-331).

An isolated FTHMA-070 or T85 protein, or a portion or fragment thereof, can be used as an immunogen to generate antibodies that bind FTHMA-070 or T85 using standard techniques for polyclonal and monoclonal antibody preparation. The full-length FTHMA-070 or T85 protein can be used or, alternatively, the invention provides antigenic peptide fragments of FTHMA-070 or T85 for use as immunogens. The antigenic peptide of FTHMA-070 or T85 comprises at least 8 (preferably 10, 15, 20, or 30) amino acid residues of the amino acid sequence shown in SEQ ID NO:2 and encompasses an epitope of FTHMA-070 or T85 such that an antibody raised against the peptide forms a specific immune complex with FTHMA-070 or T85.

Preferred epitopes encompassed by the antigenic peptide are regions of FTHMA-070 or T85 that are located on the surface of the Protein, e.g., hydrophilic regions.

A FTHMA-070 or T85 immunogen typically is used to prepare antibodies by immunizing a suitable subject, (e.g., rabbit, goat, mouse or other mammal) with the immunogen. An appropriate immunogenic preparation can contain, for example, recombinantly expressed FTHMA-070 or T85 protein or a chemically synthesized **FTHMA-070** or T85 polypeptide. The preparation can further include an adjuvant, such as Freund's complete or incomplete adjuvant, or similar immunostimulatory agent. Immunization of a suitable subject with an immunogenic FTHMA-070 or T85 preparation induces a polyclonal anti-FTHMA-070 or T85 antibody response.

Accordingly, another aspect of the invention pertains to anti-FTHMA-070 or T85 antibodies. The term "antibody" as used herein refers to immunoglobulin molecules and immunologically active portions of immunoglobulin molecules, i.e., molecules that contain an antigen binding site which specifically binds an antigen, such as FTHMA-070 or T85. A molecule which specifically binds to FTHMA-070 or T8S is a molecule which binds FTHMA-070 or T85, but does not substantially bind other molecules in a sample, e.g., a biological sample, which naturally contains FTHMA-070 or T85. Examples of immunologically active portions of immunoglobulin molecules include F(ab) and F(ab')₂ fragments which can be generated by treating the antibody with an enzyme such as pepsin. The invention provides polyclonal and monoclonal antibodies that bind FTHMA-070 or T85. The term "monoclonal antibody" or "monoclonal antibody composition", as used herein, refers to a population of antibody molecules that contain only one species of an antigen binding site capable of immunoreacting with a particular epitope of FTHMA-070 or T85. A monoclonal antibody composition thus typically displays a single binding affinity for a particular FTHMA-070 or T85 protein with which it immunoreacts.

Polyclonal anti-FTHMA-070 or T85 antibodies can be prepared as described above by immunizing a suitable subject with a FTHMA-070 or T85 immunogen. The anti-FTHMA-070 or T85 antibody titer in the immunized subject can be monitored over time by standard techniques, such as with an enzyme linked immunosorbent assay (ELISA) using immobilized FTHMA-070 or T85. If desired, the antibody molecules directed against FTHMA-070 or T85 can be isolated from the mammal (e.g., from the blood) and further purified by well-known techniques, such as protein A chromatography to obtain the IgG fraction. At an appropriate time after immunization, e.g., when the anti-FTHMA-070 or T85 antibody titers are highest, antibody-producing cells can be obtained from the subject and used to prepare monoclonal antibodies by standard techniques, such as the hybridoma technique originally described by Kohler and-Milstein (1975) Nature 256:495-497, the human B cell hybridoma technique (Kozbor et al. (1983) *Immunol Today* 4:72), the EBV-hybridoma technique (Cole et al. (1985), Monoclonal Antibodies *and Cancer Therapy*, Alan R. Liss, Inc., pp. 77-96) or trioma techniques. The technology for producing various antibodies monoclonal antibody hybridomas is well known (*see generally* Current Protocols in Immunology (1994) Coligan et al. (eds.) John Wiley & Sons, Inc., New York, NY). Briefly, an immortal cell line (typically a myeloma) is fused to lymphocytes (typically splenocytes) from a mammal immunized with a FTHMA-070 or T85 immunogen as described above, and the culture supernatants of the resulting hybridoma cells are screened to identify a hybridoma producing a monoclonal antibody that binds FTHMA-070 or T85.

Any of the many well known protocols used for fusing lymphocytes and immortalized cell lines can be applied for the purpose of generating an anti-FTHMA-070 or T85 monoclonal antibody (see, e.g., Current Protocols in Immunology, supra; Galfre et al. (1977) Nature 266:55052; R.H. Kenneth, in Monoclonal Antibodies: A New *Dimension In Biological* Analyses, Plenum Publishing Corp., New York, New York (1980); and Lerner (1981) *Yale J*. *Biol.* Med., 54:387-402. Moreover, the ordinarily skilled worker will appreciate that there are many variations of such methods which also would be useful. Typically, the immortal, cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line, e.g., a myeloma cell line that is sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-AgB.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind FTHMA-070 or T85. e.g., using a standard ELISA assay.

Alternative to preparing monoclonal antibody-secreting hybridomas, a monoclonal anti-FTHMA-070 or T85 antibody can be identified and isolated by screening a recombinant combinatorial immunoglobulin library (e.g., an antibody phage display library) with FTHMA-070 or T65 to thereby isolate immunoglobulin library members that bind FTHMA-070 or T85. Kits for generating and screening phage display libraries are commercially available (e.g., the Pharmacia *Recombinant Phage* Antibody System, Catalog No. 27-9400-01; and the Stratagene *SurfZAP*™ *Phage Display Kit*, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication WO 92/20791; PCT Publication No. WO 92/15679; PCT Publication WO 93/01288; PCT Publication No. WO 92/01047; PCT Publication No. WO 92/09690; PCT Publication No. WO 90/02809; Fuchs et al. (1991) *Bio*/*Technology* 9:1370-1372; Hay et al. (1992) *Hum. Antibod. Hybridomas* 3:81-85; Huse et al. (1989) *Science* 246:1275-1281; Griffiths et al. (1993) *EMBO J* 12:725-734.

Additionally, recombinant anti-FTHMA-070 or T85 antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT Application No. PCT/US86/02269; European Patent Application 184,187; European Patent Application 171,496; European Patent Application 173,494; PCT Publication No. WO 86/01533; U.S. Patent No. 4,816,567; European Patent Application 125,023; Better et al. (1988) *Science* 240:1041-1043; Liu et al. (1987) *Proc. Natl. Acad*. *Sci. USA* 84:3439-3443; Liu et al. (1987) *J. Immunol*. 139:3521-3526; Sun et al. (1987) *Proc.* Natl. *Acad. Sci. USA* 84:214-218; Nishimura et al. (1987) *Canc. Res.* 47:999-1005; Wood et al. (1985) Nature 314:446-449; and Shaw et al. (1988) *J*. *Natl. Cancer Inst.* 80:1553-1559); Morrison, (1985) *Science* 229:1202-1207; Oi et al. (1986) Bio/Techniques 4:214; U.S. Patent 5,225,539; Jones et al. (1986) Nature 321:552-525; Verhoeyan et al. (1988) Science 239:1534; and Beidler et al. (1988) *J. Immunol.* 141:4053-4060.

An anti-FTHMA-070 or T85 antibody (e.g., monoclonal antibody) can be used to isolate FTHMA-070 or T85 by standard techniques, such as affinity chromatography or immunoprecipitation. An anti-FTHMA-070 or T85 antibody can facilitate the purification of natural FTHMA-070 or T85 from cells and of recombinantly produced FTHMA-070 or T85 expressed in host cells. Moreover, an anti-FTHMA-070 or T85 antibody can be used to detect FTHMA-070 or T85 protein (e.g., in a cellular lysate or cell supernatant) in order to evaluate the abundance and pattern of expression of the FTHMA-070 or T85 protein. Anti-FTHMA-070 or T85 antibodies can be used diagnostically to monitor protein levels in tissue as part of a clinical testing procedure, e.g., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### III. Recombinant Expression Vectors and Host Cells

Another aspect of the invention pertains to vectors, preferably expression vectors, containing a nucleic acid encoding FTHMA-070 or T85 (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectore). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operatively linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; *Gene Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., FTHMA-070 or T85 proteins, mutant forms of FTHMA-070 or T85, fusion proteins, etc.).

The recombinant expression vectors of the invention can be designed for expression of FTHMA-070 or T85 in prokaryotic or eukaryotic cells, e.g., bacterial cells such as *E*. coli, insect cells (using baculovirus expression vectors) yeast cells or mammalian cells. Suitable host cells are discussed further in Goeddel, Gene *Expression* Technology: *Methods in Enzymology* 185, Academic Press, San Diego, CA (1990). Alternatively, the recombinant expression vector can be transcribed and translated in vitro, for example using T7 promoter regulatory sequences and T7 polymerase.

Expression of proteins in prokaryotes is most often carried out in *E. coli* with vectors containing constitutive or inducible promoters directing the expression of either' fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) Gene 67:31-40), pMAL (New England Biolabs, Beverly, MA) and pRIT5 (Pharmacia, Piscataway, NJ) which fuse glutathione *S*-transferase (GST), maltose E binding protein, or protein A, respectively, to the target recombinant protein.

Examples of suitable inducible non-fusion *E. coli* expression vectors include pTrc (Amann et al., (1988) Gene 69:301-315) and pET 11d (Studier et al., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident λ prophage harboring a T7 gn1 gene under the transcriptional control of the lacUV 5 promoter.

One strategy to maximize recombinant protein expression in E. coli is to express the protein in a host bacteria with an impaired capacity to proteolytically cleave the recombinant protein (Gottesman, Gene *Expression Technology*: *Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al. (1992) *Nucleic Acids Res.* 20:2111-2118). Such alteration of nucleic acid sequences of the invention can be carried out by standard DNA synthesis techniques.

In another embodiment, the FTHMA-070 or T85 expression vector is a yeast expression vector. Examples of vectors for expression in yeast S. *cerivisae* include pYepSecl (Baldari et al. (1987) *EMBO J.* 6:229-234), pMFa (Kurjan and Herskowitz, (1982) *Cell* 30:933-943), pJRY88 (Schultz et al. (1987) Gene 54:113-123), pYES2 (Invitrogen Corporation, San Diego, CA), and picZ (InVitrogen Corp, San Diego, CA).

Alternatively, FTHMA-070 or T85 can be expressed in insect cells using baculovirus expression vectors. Baculovirus vectors available for expression of proteins in cultured insect cells (e.g., Sf 9 cells) include the pAc series (Smith et al. (1983) *Mol*. Cell *Biol.* 3:2156-2165) and the pVL series (Lucklow and Summers (1989) Virology 170:31-39).

In yet another embodiment, a nucleic acid of the invention is expressed in mammalian cells using a mammalian expression vector. Examples of mammalian expression vectors include pCDM8 (Seed (1987) Nature 329:840) and pMT2PC (Kaufman et al. (1987) *EMBO J.* 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al. *(supra).*

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) *Adv. Immunol*. 43:235-275), in particular promoters of T cell receptors (Winoto and Baltimore (1989) *EMBO J.* 8:729-733) and immunoglobulins (Banerji et al. (1983) *Cell* 33:729-740; Queen and Baltimore (1983) Cell 33:741-748), neuron-specific promoters (e.g., the neurofilament promoter; Byrne and Ruddle (1989) Proc. *Natl. Acad. Sci. USA* 86:5473-5477), pancreas-specific promoters (Edlund et al. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to FTHMA-070 or T85 mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes See Weintraub et al., *Reviews - Trends* in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, FTHMA-070 or T85 protein can be expressed in bacterial cells such as *E*. coli, insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook, et al. (supra), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding FTHMA-070 or T85 or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (i.e., express) FTHMA-070 or TB5 protein. Accordingly, the invention further provides methods for producing FTHMA-070 or T85 protein using the host cells of the invention. In one embodiment, the method comprises culturing the host cell of invention (into which a recombinant expression vector encoding FTHMA-070 or TB5 has been introduced) in a suitable medium such that FTHMA-070 or T85 protein is produced. In another embodiment, the method further comprises isolating FTHMA-070 or T85 from the medium or the host cell.

The host cells of the invention can also be used to produce nonhuman transgenic animals. For example, in one embodiment, a host cell of the invention is a fertilized oocyte or an embryonic stem cell into which FTHMA-070 or T85-coding sequences have been introduced. Such host cells can then be used to create non-human transgenic animals in which exogenous FTHMA-070 or T85 sequences have been introduced into their genome or homologous recombinant animals in which endogenous FTHMA-070 or T85 sequences have been altered. Such animals are useful for studying the function and/or activity of FTHMA-070 or T85 and for identifying and/or evaluating modulators of FTHMA-070 or T85 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, an "homologous recombinant animal" is a non-human animal, preferably a mammal, more preferably a mouse, in which an endogenous FTHMA-070 or T85 gene has been altered by homologous recombination between the endogenous gene and an exogenous DNA molecule introduced into a cell of the animal, e.g., an embryonic cell of the animal, prior to development of the animal.

A transgenic animal of the invention can be created by introducing FTHMA-070 or T85-encoding nucleic acid into the male pronuclei of a fertilized oocyte, e.g., by microinjection, retroviral infection, and allowing the oocyte to develop in a pseudopregnant female foster animal. The FTHMA-070 or T85 cDNA sequence can be introduced as a transgene into the genome of a non-human animal. Alternatively, a nonhuman homologue of the human FTHMA-070 or T85 gene, such as a mouse FTHMA-070 or T85 gene, can be isolated based on hybridization to the human FTHMA-070 or T85 cDNA and used as a transgene. Intronic sequences and polyadenylation signals can also be included in the transgene to increase the efficiency of expression of the transgene. A tissue-specific regulatory sequence(s) can be operably linked to the FTHMA-070 or T85 transgene to direct expression of FTHMA-070 or T85 protein to particular cells. Methods for generating transgenic animals via embryo manipulation and microinjection, particularly animals such as mice, have become conventional in the art and are described, for example, in U.S. Patent Nos. 4,736,866 and 4,870,009, U.S. Patent No. 4,873,191 and in Hogan, *Manipulating the Mouse Embryo*, (Cold Spring Harbor Laboratory Press, Cold Spring Harbor, N.Y., 1986). Similar methods are used for production of other transgenic animals. A transgenic founder animal can be identified based upon the presence of the FTHMA-070 or T85 transgene in its genome and/or expression of FTHMA-070 or T85 mRNA in tissues or cells of the animals. A transgenic founder animal can then be used to breed additional animals carrying the transgene. Moreover, transgenic animals carrying a transgene encoding FTHMA-070 or T85 can further be bred to other transgenic animals carrying other transgenes.

To create an homologous recombinant animal, a vector is prepared which contains at least a portion of a FTHMA-070 or T85 gene (e.g., a human or a non-human homolog of the FTHMA-070 or T85 gene, e.g., a murine FTHMA-070 or T85 gene) into which a deletion, addition or substitution has been introduced to thereby alter, e.g., functionally disrupt, the FTHMA-070 or T85 gene. In a preferred embodiment, the vector is designed such that, upon homologous recombination, the endogenous FTHMA-070 or T85 gene is functionally disrupted (i.e., no longer encodes a functional protein; also referred to as a "knock out" vector). Alternatively, the vector can be designed such that, upon homologous recombination, the endogenous FTHMA-070 or T85 gene is mutated or otherwise altered but still encodes functional protein (e.g., the upstream regulatory region can be altered to thereby alter the expression of the endogenous FTHMA-070 or T85 protein). In the homologous recombination vector, the altered portion of the FTHMA-070 or T85 gene is flanked at its 5' and 3' ends by additional nucleic acid of the FTHMA-070 or T85 gene to allow for homologous recombination to occur between the exogenous FTHMA-070 or T85 gene carried by the vector and an endogenous FTHMA-070 or T85 gene in an embryonic stem cell. The additional flanking FTHMA-070 or T85 nucleic acid is of sufficient length for successful homologous recombination with the endogenous gene. Typically, several kilobases of flanking DNA (both at the 5' and 3' ends) are included in the vector *(see* e.g., Thomas and Capecchi (1987) Cell 51:503 for a description of homologous recombination vectors). The vector is introduced into an embryonic stem cell line (e.g., by electroporation) and cells in which the introduced FTHMA-070 or T85 gene has homologously recombined with the endogenous FTHMA-070 or T85 gene are selected (see e.g., Li et al. (1992) Cell 69:915). The selected cells are then injected into a blastocyst of an animal (e.g., a mouse) to form aggregation chimeras (see, e.g., Bradley in *Teratocarcinomas* and *Embryonic* Stem Cells: A Practical Approach, Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley (1991) *Current Opinion in Bio*/Technology 2:823-829 and in PCT Publication Nos. WO 90/11354, WO 91/01140, WO 92/0968, and WO 93/04169.

In another embodiment, transgenic non-humans animals can be produced which contain selected systems which allow for regulated expression of the transgene. One example of such a system is the *cre*/*loxP* recombinase system of bacteriophage P1. For a description of the *cre*/*loxP* recombinase system, see, e.g., Lakso et al. (1992) *Proc. Natl*. *Acad. Sci*. USA 89:6232-6236. Another example of a recombinase system is the FLP recombinase system of *Saccharomyces* cerevisiae (O'Gorman et al. (1991) Science 251:1351-1355. If a *cre*/*loxP* recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut et al. (1997) Nature 385:810-813 and PCT Publication Nos. WO 97/07668 and WO 97/07669. In brief, a cell, e.g., a somatic cell, from the transgenic animal can be isolated and induced to exit the growth cycle and enter Gₒ phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, e.g., the somatic cell, is isolated.

### IV. Pharmaceutical Compositions

The FTMA-070 or T85 nucleic acid molecules, FTHMA-070 or T85 proteins, and anti-FTHMA-070 or T85 antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF; Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a FTHMA-070 or T85 protein or anti-FTHMA-070 or T85 antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administrations (see U.S. Patent 5,328,470) or by estereotactic injection *(see e.g.,* Chen et al. (1994) *Proc. Natl*. *Acad. Sci*. *USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The nucleic acid molecules, proteins, protein homologues, and antibodies described herein can be used in one or more of the following methods: a) screening assays; b) detection assays (e.g., chromosomal mapping, tissue typing, forensic biology), c) predictive medicine (e.g., diagnostic assays, prognostic assays, monitoring clinical trials, and pharmacogenomics); and d) methods of treatment (e.g., therapeutic and prophylactic). The isolated nucleic acid molecules of the invention can be used to express FTHMA-070 or T85 protein (e.g., via a recombinant expression vector in a host cell in gene therapy applications), to detect FTHMA-070 or T85 mRNA (e.g., in a biological sample) or a genetic lesion in a FTHMA-070 or T85 gene, and to modulate FTHMA-070 or T85 activity. In addition, the FTHMA-070 or T85 proteins can be used to screen drugs or compounds which modulate the FTHMA-070 or T85 activity or expression as well as to treat disorders characterized by insufficient or excessive production of FTHMA-070 or T85 protein or production of FTHMA-070 or T85 protein forms which have decreased or aberrant activity compared to FTHMA-070 or T85 wild type protein. In addition, the anti-FTHMA-070 or T85 antibodies of the invention can be used to detect and isolate FTHMA-070 or T85 proteins and modulate FTHMA-070 or T85 activity.

This invention further pertains to novel agents identified by the above-described screening assays and uses thereof for treatments as described herein.

### A. Screening Assays

The invention provides a method (also referred to herein as a "screening assay") for identifying modulators, i.e., candidate or test compounds or agents (e.g., peptides, peptidomimetics, small molecules or other drugs) which bind to FTHMA-070 or T85 proteins or have a stimulatory or inhibitory effect on, for example, FTHMA-070 or T85 expression or FTHMA-070 or T85 activity.

In one embodiment, the invention provides assays for screening candidate or test compounds which bind to or modulate the activity of the membrane-bound form of a FTHMA-070 or T85 protein or polypeptide or biologically active portion thereof. The test compounds of the present invention can be obtained.using any of the numerous approaches in combinatorial library methods known in the art, including: biological libraries; spatially addressable parallel solid phase or solution phase libraries; synthetic library methods requiring deconvolution; the one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, (1997) *Anticancer Drug* Des. 12:145).

Examples of methods for the synthesis of molecular libraries can be found in the art, for example in: DeWitt, et al. (1993) *Proc. Natl. Acad*. *Sci*. *U.S.A.* 90:6909; Erb et al. (1994) *Proc. Natl.* Acad. *Sci. USA* 91:11422; Zuckermann et al. (1994). *J. Med. Chem.* 37:2678; Cho et al. (1993) *Science* 261:1303; Carrell et al. (1994) *Angew. Chem. Int. Ed. Engl*. 33:2059; Carell et al. (1994) *Angew. Chem. Int. Ed. Engl.* 33:2061; and Gallop et al. (1994) *J*. *Med.* Chem. 37:1233.

Libraries of compounds may be presented in solution (e.g., Houghten (1992) *Bio*/*Techniques* 13:412-421), or on beads (Lam (1991) Nature 354:82-84), chips (Fodor (1993) Nature 364:555-556), bacteria (U.S. Patent No. 5,223,409), spores (Patent Nos. 5,571,698; 5,403,484; and 5,223,409), plasmids (Cull et al. (1992) *Proc. Natl. Acad. Sci. USA* 89:1865-1869) or on phage (Scott and Smith (1990) *Science* 249:386-390; Devlin (1990) *Science* 249:404-406; Cwirla et al. (1990) *Proc. Natl. Acad. Sci.* 87:6378-6382; and Felici (1991) *J*. *Mol*. *Biol.* 222:301-310).

In one embodiment, an assay is a cell-based assay in which a cell which expresses a membrane-bound form of FTHMA-070 or T85 protein, or a biologically active portion thereof, on the cell surface is contacted with a test compound and the ability of the test compound to bind to a FTHMA-070 or T85 protein determined. The cell, for example, can be a yeast cell or a cell of mammalian origin. Determining the ability of the test compound to bind to the FTHMA-070 or T85 protein can be accomplished, for example, by coupling the test compound with a radioisotope or enzymatic label such that binding of the test compound to the FTHMA-070 or T85 protein or biologically active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish peroxidase, alkaline phosphatase, or luciferase, and the enzymatic label detected by determination of conversion of an appropriate substrate to product. In a preferred embodiment, the assay comprises contacting a cell which expresses a membrane-bound form of FTHMA-070 or T85 protein, or a biologically active portion thereof, on the cell surface with a known compound which binds FTHMA-070 or T85 to form an assay mixture, contacting the assay mixture with a test compound, and determining the ability of the test compound to interact with a FTHMA-070 or T85 protein, wherein determining the ability of the test compound to interact with a FTHMA-070 or T85 protein comprises determining the ability of the test compound to preferentially bind to FTHMA-070 or T85 or a biologically active portion thereof as compared to the known compound.

In another embodiment, an assay is a cell-based assay comprising contacting a cell expressing a membrane-bound form of FTHMA-070 or T85 protein, or a biologically active portion thereof, on the cell surface with a test compound and determining the ability of the test compound to modulate (e.g., stimulate or inhibit) the activity of the FTHMA-070 or T85 protein or biologically active portion thereof. Determining the ability of the test compound to modulate the activity of FTHMA-070 or T85 or a biologically active portion thereof can be accomplished, for example, by determining the ability of the FTHMA-070 or T85 protein to bind to or interact with a FTHMA-070 or T85 target molecule. As used herein, a "target molecule" is a molecule with which a FTHMA-070 or T85 protein binds or interacts in nature, for example, a molecule on the surface of a cell which expresses a FTHMA-070 or T85 protein, a molecule on the surface of a second cell, a molecule in the extracellular milieu, a molecule associated with the internal surface of a cell membrane or a cytoplasmic molecule. A FTHMA-070 or T85 target molecule can be a non-FTHMA-070 or T85 molecule or a FTHMA-070 or T85 protein or polypeptide of the present invention. In one embodiment, a FTHMA-070 or T85 target molecule is a component of a signal transduction pathway which facilitates transduction of an extracellular signal (e.g., a signal generated by binding of a compound to a membrane-bound FTHMA-070 or T85 molecule) through the cell membrane and into the cell. The target, for example, can be a second intercellular protein which has catalytic activity or a protein which facilitates the association of downstream signaling molecules with FTHMA-070 or T85.

Determining the ability of the FTHMA-070 or T85 protein to bind to or interact with a FTHMA-070 or T85 target molecule can be accomplished by one of the methods described above for determining direct binding. In a preferred embodiment, determining the ability of the FTHMA-070 or T85 protein to bind to or interact with a FTHMA-070 or T85 target molecule can be accomplished by determining the activity of the target molecule. For example, the activity of the target molecule can be determined by detecting induction of a cellular second messenger of the target (e.g., intracellular Ca²⁺, diacylglycerol, IP3, etc.), detecting catalytic/enzymatic activity of the target an appropriate substrate, detecting the induction of a reporter gene (e.g., a FTHMA-070 or T85-responsive regulatory element operatively linked to a nucleic acid encoding a detectable marker, e.g. luciferase), or detecting a cellular response, for example, cell survival, cellular differentiation, or cell proliferation.

In yet another embodiment, an assay of the present invention is a cell-free assay comprising contacting a FTHMA-070 or T85 protein or biologically active portion thereof with a test compound and determining the ability of the test compound to bind to the FTHMA-070 or T85 protein or biologically active portion thereof. Binding of the test compound to the FTHMA-070 or T85 protein can be determined either directly or indirectly as described above. In a preferred embodiment, the assay includes contacting the FTHMA-070 or T85 protein or biologically active portion thereof with a known compound which binds FTHMA-070 or T85 to form an assay mixture, contacting the assay mixture with a test compound, and determining the ability of the test compound to interact with a FTHMA-070 or T85 protein, wherein determining the ability of the test compound to interact with a FTHMA-070 or T85 protein comprises determining the ability of the test compound to preferentially bind to FTHMA-070 or T85 or biologically active portion thereof as compared to the known compound.

In another embodiment, an assay is a cell-free assay comprising contacting FTHMA-070 or T85 protein or biologically active portion thereof with a test compound and determining the ability of the test compound to modulate (e.g., stimulate or inhibit) the activity of the FTHMA-070 or T85 protein or biologically active portion thereof. Determining the ability of the test compound to modulate the activity of FTHMA-070 or T85 can be accomplished, for example, by determining the ability of the FTHMA-070 or T85 protein to bind to a FTHMA-070 or T85 target molecule by one of the methods described above for determining direct binding. In an alternative embodiment, determining the ability of the test compound to modulate the activity of FTHMA-070 or T85 can be accomplished by determining the ability of the FTHMA-070 or T85 protein further modulate a FTHMA-070 or T85 target molecule. For example, the catalytic/enzymatic activity of the target molecule on an appropriate substrate can be determined as previously described.

In yet another embodiment, the cell-free assay comprises contacting the FTHMA-070 or T85 protein or biologically active portion thereof with a known compound which binds FTHMA-070 or T85 to form an assay mixture, contacting the assay mixture with a test compound, and determining the ability of the test compound to interact with a FTHMA-070 or T85 protein, wherein determining the ability of the test compound to interact with a FTHMA-070 or T85 protein comprises determining the ability of the FTHMA-070 or T85 protein to preferentially bind to or modulate the activity of a FTHMA-070 or T85 target molecule.

In the case of cell-free assays, it may be desirable to utilize a solubilizing agent such that the membrane-bound form of FTHMA-070 or T85 is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Tritons® X-100, Triton® X-114, Thesit®, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either FTHMA-070 or TB5 or its target mr to facilitate separation of complexed from uncomplexed forms of one or both of the proteins, as well as to accommodate automation of the assay Binding of a test compound to FTHMA-070 or T85, or interaction of FTHMA-070 or T85 with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/ FTHMA-070 or T85 fusion proteins or glutathione-S-transferase/target fusion proteins can be adsorbed onto glutathione sepharose beads (Sigma Chemical; St. Louis, Mo) or glutathione derivatized microtitre plates, which are then combined with the test compound or the test compound and either the non-adsorbed target protein or FTHMA-070 or T85 protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of FTHMA-070 or T85 binding or activity determined using standard techniques.

Other techniques for immobilizing proteins on matrices can also be used in the screening assays of the invention. For example, either FTHMA-070 or T85 or its target molecule can be immobilized utilizing conjugation of biotin and streptavidin. Biotinylated FTHMA-070 or T85 or target molecules can be prepared from biotin-NHS (N-hydroxy-succinimide) using techniques well known in the art (e.g., biotinylation kit, Pierce Chemicals; Rockford, IL), and immobilized in the wells of streptavidin-coated 96 well plates (Pierce Chemical). Alternatively, antibodies reactive with FTHMA-070 or T85 or target molecules but which do not interfere with binding of the FTHMA-070 or T85 protein to its target molecule can be derivatized to the wells of the plate, and unbound target or FTHMA-070 or T85 trapped in the wells by antibody conjugation. Methods for detecting such complexes, in addition to those described above for the GST-immobilized complexes, include immunodetection of complexes using antibodies reactive with the FTHMA-070 or T85 or target molecule, as well as enzyme-linked assays which rely on detecting an enzymatic activity associated with the FTHMA-070 or T85 or target molecule.

In another embodiment, modulators of FTHMA-070 or T85 expression are identified in a method in which a cell is contacted with a candidate compound and the expression of FTHMA-070 or T85 mRNA or protein in the cell is determined. The level of expression of FTHMA-070 or T85 mRNA or protein in the presence of the candidate compound is compared to the level of expression of FTHMA-070 or T85 mRNA or protein in the absence of the candidate compound. The candidate compound can then be identified as a modulator of FTHMA-070 or T85 expression based on this comparison. For example, when expression of FTHMA-070 or T85 mRNA or protein is greater (statistically significantly greater) in the presence of the candidate compound than in its absence, the candidate compound is identified as a stimulator of FTHMA-070 or T85 mRNA or protein expression. Alternatively, when expression of FTHMA-070 or T85 mRNA or protein is less (statistically significantly less) in the presence of the candidate compound than in its absence, the candidate compound is identified as an inhibitor of FTHMA-070 or T85 mRNA or protein expression. The level of FTHMA-070 or T85 mRNA or protein expression in the cells can be determined by methods described herein for detecting FTHMA-070 or T85 mRNA or protein.

In yet another aspect of the invention, the FTHMA-070 or T85 proteins can be used as "bait proteins" in a two-hybrid assay or three hybrid assay (see, e.g., U.S. Patent No. 5,283,317; Zervos et al. (1993) Cell 72:223-232; Madura et al. (1993) *J. Biol. Chem.* 268:12046-12054; Bartel et al. (1993) *Bio*/*Techniques* 14:920-924; Iwabuchi et al. (1993) Oncogene 8:1693-1696; and WO94/10300), to identify other proteins, which bind to or interact with FTHMA-070 or T85 and modulate FTHMA-070 or T85 activity. Such FTHMA-070 or T85-binding proteins are also likely to be involved in the propagation of signals by the FTHMA-070 or T85 proteins as, for example, upstream or downstream elements of the FTHMA-070 or T85 pathway.

The two-hybrid system is based on the modular nature of most transcription factors, which consist of separable DNA-binding and activation domains. Briefly, the assay utilizes two different DNA constructs. In one construct, the gene that codes for FTHMA-070 or T85 is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming an FTHMA-070 or TB5-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with FTHMA-070 or T85.

This invention further pertains to novel agents identified by the above-described screening assays and uses thereof for treatments as described herein.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. Accordingly, FTHMA-070 or T85 nucleic acid molecules described herein or fragments thereof, can be used to map the location of FTHMA-070 or T85 genes on a chromosome. The mapping of the FTHMA-070 or T85 sequences to chromosomes is an important first step in correlating these sequences with genes associated with disease.

Briefly, FTHMA-070 or T85 genes can be mapped to chromosomes by preparing PCR primers (preferably 15-25 bp in length) from the FTHMA-070 or T85 sequences. Computer analysis of FTHMA-070 or T85 sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the FTHMA-070 or T85 sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but human cells can, the one human chromosome that contains the gene encoding the needed enzyme, will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. (D'Eustachio et al. (1983) Science 220:919-924). Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the FTHHA-070 or T85 sequences to design oligonucleotide primers, sublocalization can be achieved with panels of fragments from specific chromosomes. Other mapping strategies which can similarly be used to map a FTHMA-070 or T85 sequence to its chromosome include in situ hybridization (described in Fan et al. (1990) *Proc. Natl*. *Acad. Sci. USA* 87:6223-27), pre-screening with labeled flow-sorted chromosomes, and pre-selection by hybridization to chromosome specific cDNA libraries.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques (Pergamon Press, New York, 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between genes and disease, mapped to the same chromosomal region, can then be identified through linkage analysis (co-inheritance of physically adjacent genes), described in, e.g., Egeland et al. (1987) Nature, 325:783-787.

Moreover, differences in the DNA sequences between individuals affected and unaffected with a disease associated with the FTHMA-070 or T85 gene can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 2. Tissue Typing

The FTHMA-070 or T85 sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the FTHMA-070 or T85 sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The FTHMA-070 or T85 sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 or SEQ ID NO:5 can comfortably provide positive individual identification with a panel of perhaps 10 to 1,000 primers which each yield a noncoding amplified sequence of 100 bases. If predicted coding sequences, such as those in SEQ ID NO:3 or SEQ ID NO:7 are used, a more appropriate number of primers for positive individual identification would be 500-2,000.

If a panel of reagents from FTHMA-070 or T85 sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial FTHMA-070 or T85 Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions are particularly appropriate for this use as greater numbers of polymorphisms occur in the noncoding regions, making it easier to differentiate individuals using this technique. Examples of polynucleotide reagents include the FTHMA-070 or T85 sequences or portions thereof, e.g., fragments derived from the noncoding regions having a length of at least 20 or 30 bases.

The FTHMA-070 or T85 sequences described herein can further be used to provide polynucleotide reagents, e.g., labeled or labelable probes which can be used in, for example, an in situ hybridization technique, to identify a specific tissue, e.g., brain tissue. This can be very useful in cases where a forensic pathologist is presented with a tissue of unknown origin. Panels of such FTHMA-070 or T85 probes can be used to identify tissue by species and/or by organ type.

In a similar fashion, these reagents, e.g., FTHMA-070 or T85 primers or probes can be used to screen tissue culture for contamination (i.e., screen for the presence of a mixture of different types of cells in a culture).

### C. Predictive Medicine

The present invention also pertains to the field of predictive medicine in which diagnostic assays, prognostic assays, pharmacogenomics, and monitoring clinical trails are used for prognostic (predictive) purposes to thereby treat an individual prophylactically. Accordingly, one aspect of the present invention relates to diagnostic assays for determining FTHM. 070 or T85 protein and/or nucleic acid expression as cell as FTHMA-070 or T85 activity, in the context of a biological sample (e.g., blood, serum, cells, tissue) to thereby determine whether an individual is afflicted with a disease or disorder, or is at risk of developing a disorder, associated with aberrant FTHMA-070 or T85 expression or activity. The invention also provides for prognostic (or predictive) assays for determining whether an individual is at risk of developing a disorder associated with FTHMA-070 or T85 protein, nucleic acid expression or activity. For example, mutations in a FTHMA-070 or T85 gene can be assayed in a biological sample. Such assays can be used for prognostic or predictive purpose to thereby prophylactically treat an individual prior to the onset of a disorder characterized by or associated with FTHMA-070 or T85 protein, nucleic acid expression or activity.

Another aspect of the invention provides methods for determining FTHMA-070 or T85 protein, nucleic acid expression or FTHMA-070 or T85 activity in an individual to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (e.g., the genotype of the individual examined to determine the ability of the individual to respond to a particular agent.)

Yet another aspect of the.invention pertains to monitoring the influence of agents (e.g., drugs or other compounds) on the expression or activity of FTHMA-070 or T85 in clinical trials.

These and other agents are described in further detail in the following sections.

### 1. Diagnostic Assays

An exemplary method for detecting the presence or absence of FTHMA-070 or T85 in a biological sample involves obtaining a biological sample from a test subject and contacting the biological sample with a compound or an agent capable of detecting FTHMA-070 or T85 protein or nucleic acid (e.g., mRNA, genomic DNA) that encodes FTHMA-070 or T85 protein such that the presence of FTHMA-070 or T85 is detected in the biological sample. A preferred agent for detecting FTHMA-070 or T85 mRNA or genomic DNA is a labeled nucleic acid probe capable of hybridizing to FTHMA-070 or T85 mRNA or genomic DNA. The nucleic acid probe can be, for example, a full-length FTHMA-070 or T85 nucleic acid, or a portion thereof, such as an oligonucleotide of at least 15, 30, 50, 100, 250 or 500 nucleotides in length and sufficient to specifically hybridize under stringent conditions to FTHMA-070 or T85 mRNA or genomic DNA. other suitable probes for use in the diagnostic assays of the invention are described herein.

A preferred agent for detecting FTHMA-070 or T85 protein is an antibody capable of binding to FTHMA-070 or T85 protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect FTHMA-070 or T85 mRNA, protein, or genomic DNA in a biological sample *in vitro* as well as *in vivo*. For example, *in vitro* techniques for detection of FTHMA-070 or T85 mRNA include Northern hybridizations and *in* situ hybridizations. In vitro techniques for detection of FTHMA-070 or T85 protein include enzyme linked immunosorbent assays (ELISAs), Western blots, immunoprecipitations and immunofluorescence. *In vitro* techniques for detection of FTHMA-070 or T85 genomic DNA include Southern hybridizations. Furthermore, *in vivo* techniques for detection of FTHMA-070 or T85 protein include introducing into a subject a labeled anti-FTHMA-070 or T85 antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A'preferred biological sample is a peripheral blood leukocyte sample isolated by conventional means from a subject.

In another embodiment, the methods further involve obtaining a control biological sample from a control subject, contacting the control sample with a compound or agent capable of detecting FTHMA-070 or T85 protein, mRNA, or genomic DNA, such that the presence of FTHMA-070 or T85 protein, mRNA or genomic DNA is detected in the biological sample, and comparing the presence of FTHMA-070 or T85 protein, mRNA or genomic DNA in the control sample with the presence of FTHMA-070 or T85 protein, mRNA or genomic DNA in the test sample.

The invention also encompasses kits for detecting the presence of FTHMA-070 or T85 in a biological sample (a test sample). Such kits can be used to determine if a subject is suffering from or is at increased risk of developing a disorder associated with aberrant expression of FTHMA-070 or T85 (e.g., an immunological disorder). For example, the kit can comprise a labeled compound or agent capable of detecting FTHMA-070 or T85 protein or mRNA in a biological sample and means for determining the amount of FTHMA-070 or T85 in the sample (e.g., an anti-FTHMA-070 or T85 antibody or an oligonucleotide probe which binds to DNA encoding FTHMA-070 or T85). Kits may also include instruction for observing that the tested subject is suffering from or is at risk of developing a disorder associated with aberrant expression of FTHMA-070 or T85 if the amount of FTHMA-070 or T85 protein or mRNA is above or below a normal level.

For antibody-based kits, the kit may comprise, for example: (1) a first antibody (e.g., attached to a solid support) which binds to FTHMA-070 or T85 protein; and, optionally, (2) a second, different antibody which binds to FTHMA-070 or T85 protein or the first antibody and is conjugated to a detectable agent.

For oligonucleotide-based kits, the kit may comprise, for example: (1) a oligonucleotide, e.g., a detectably labelled oligonucleotide, which hybridizes to a FTHMA-070 or T85 nucleic acid sequence or (2) a pair of primers useful for amplifying a FTHMA-070 or T85 nucleic acid molecule;

The kit may also comprise, e.g., a buffering agent, a preservative, or a protein stabilizing agent. The kit may also comprise components necessary for detecting the detectable agent (e.g., an enzyme or a substrate). The kit may also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a disorder associated with aberrant expression of FTHMA-070 or T85.

### 2. Prognostic Assays

The methods described herein can furthermore be utilized as diagnostic or prognostic assays to identify subjects having or at risk of developing a disease or disorder associated with aberrant FTHMA-070 or T85 expression or activity. For example, the assays described herein, such as the preceding diagnostic assays or the following assays, can be utilized to identify a subject having or at risk of developing a disorder associated with FTHMA-070 or T85 protein, nucleic acid expression or activity such as an immune system disorder. Alternatively, the prognostic assays can be utilized to identify a subject having or at risk for developing such a disease or disorder. Thus, the present invention provides a method in which a test sample is obtained from a subject and FTHMA-070 or T85 protein or nucleic acid (e.g., mRNA, genomic DNA) is detected, wherein the presence of FTHMA-070 or T85 protein or nucleic acid is diagnostic for a subject having or at risk of developing a disease or disorder associated with aberrant FTHMA-070 or T85 expression or activity. As used herein, a "test sample" refers to a biological sample obtained from a subject of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant FTHMA-070 or T85 expression or activity. For example, such methods can be used to determine whether a subject can be effectively treated with a specific agent or class of agents (e.g., agents of a type which decrease FTHMA-070 or T85 activity). Thus, the present invention provides methods for determining whether a subject can be effectively treated with an agent for a disorder associated with aberrant FTHMA-070 or T85 expression or activity in which a test sample is obtained and FTHMA-070 or T85 protein or nucleic acid is detected (e.g., wherein the presence of FTHMA-070 or T85 protein or nucleic acid is diagnostic for a subject that can be administered the agent to treat a disorder associated with aberrant FTHMA-070 or T85 expression or activity).

The methods of the invention can also be used to detect genetic lesions or mutations in a FTHMA-070 or T85 gene, thereby determining if a subject with the lesioned gene is at risk for a disorder characterized by aberrant cell proliferation and/or differentiation. In preferred embodiments, the methods include detecting, in a sample of cells from the subject, the presence or absence of a genetic lesion characterized by at least one of an alteration affecting the integrity of a gene encoding a FTHMA-070 or T85-protein, or the mis-expression of the FTHMA-070 or T85 gene. For example, such genetic lesions can be detected by ascertaining the existence of at least one of 1) a deletion of one or more nucleotides from a FTHMA-070 or T85 gene; 2) an addition of one or more nucleotides to a FTHMA-070 or T85 gene; 3) a substitution of one or more nucleotides of a FTHMA-070 or T85 gene, 4) a chromosomal rearrangement of a FTHMA-070 or T85 gene; 5) an alteration in the level of a messenger RNA transcript of a FTHMA-070 or T85 gene, 6) aberrant modification of a FTHMA-070 or T85 gene, such as of the methylation pattern of the genomic DNA, 7) the presence of a non-wild type splicing pattern of a messenger RNA transcript of a FTHMA-070 or T85 gene, B) a non-wild type level of a FTHMA-070 or T85-protein, 9) allelic loss of a FTHMA-070 or T85 gene, and 10) inappropriate post-translational modification of a FTHMA-070 or T85-protein. As described herein, there are a large number of assay techniques known in the art which can be used for detecting lesions in a FTHMA-070 or T85 gene. A preferred biological sample is a peripheral blood leukocyte sample isolated by conventional means from a subject.

In certain embodiments, detection of the lesion involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, e.g., Landegran et al. (1988) Science 241:1077-1080; and Nakazawa et al. (1994) *Proc. Natl. Acad. Sci*. *USA* 91:360-364), the latter of which can be particularly useful for detecting point mutations in the FTHMA-070 or T85-gene (see Abravaya et al. (1995) *Nucleic Acids Res.* 23:675-682). This method can include the steps of collecting a sample of cells from a patient, isolating nucleic acid (e.g., genomic, mRNA or both) from the cells of the sample, contacting the nucleic acid sample with one or more primers which specifically hybridize to a FTHMA-070 or T85 gene under conditions such that hybridization and amplification of the FTHMA-070 or T85-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al. (1990) *Proc. Natl. Acad. Sci. USA* 87:1874-1878), transcriptional amplification system (Kwoh, et al. (1989) *Proc. Natl*. *Acad. Sci. USA* 86:1173-1177), Q-Beta Replicase (Lizardi et al. (1988) *Bio*/*Technology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a FTHMA-070 or T85 gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, *e.g.,* U.S. Patent No. 5,4.98,531) can be used to score for the presence of specific mutations by development or loss of a ribozyme cleavage site.

In other embodiments, genetic mutations in FTHMA-070 or T85 can be identified by hybridizing a sample and control nucleic acids, e.g., DNA or RNA, to high density arrays containing hundreds or thousands of oligonucleotides probes (Cronin et al. (1996) Human Mutation 7:244-255; Kozal et al. (1996) Nature Medicine 2:753-759). For example, genetic mutations in FTHMA-070 or T85 can be identified in two-dimensional arrays containing light-generated DNA probes as described in Cronin et al. supra. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the FTHMA-070 or T85 gene and detect mutations by comparing the sequence of the sample FTHMA-070 or T85 with the corresponding wild-type (control) sequence. Examples of sequencing reactions include those based on techniques developed by Maxim and Gilbert ((1977) *Proc. Natl. Acad. Sci. USA* 74:560) or Sanger ((1977) *Proc. Natl. Acad. Sci. USA* 74:5463). It is also contemplated that any of a variety of automated sequencing procedures can be utilized when performing the diagnostic assays ((1995) *Bio*/*Techniques* 19:448), including sequencing by mass spectrometry (*see*, *e.g.*, PCT Publication No. WO 94/16101; Cohen et al. (1996) Adv. *Chromatogr.* 36:127-162; and Griffin et al. (1993) *Appl.* Biochem. Biotechnol. 38:147-159).

Other methods for detecting mutations in the FTHMA-070 or T85 gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type FTHMA-070 or T85 sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, e.g., Cotton et al (1988) *Proc*. *Natl Acad Sci USA* 85:4397; Saleeba et al (1992) Methods *Enzymol.* 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in FTHMA-070 or T85, cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) *Carcinogenesis* 15:1657-1662). According to an exemplary embodiment, a probe based on a FTHMA-070 or T85 sequence, e.g., a wild-type FTHMA-070 or T85 sequence, is hybridized to a cDNA or other DNA product from a test cell(s). The duplex is treated with a DNA mismatch repair enzyme, and the cleavage products, if any, can be detected from electrophoresis protocols or the like. See, e.g., U.S. Patent No. 5,459,039.

In other embodiments, alterations in electrophoretic mobility will be used to identify mutations in FTHMA-070 or T85 genes. For example, single strand conformation polymorphism (SSCP) may be used to detect differences in electrophoretic mobility between mutant and wild type nucleic acids (Orita et al. (1999) *Proc Natl. Acad. Sci USA:* 86:2766, *see also* Cotton (1993) *Mutat. Res*. 285:125-144; and Hayashi (1992) *Genet* Anal *Tech Appl* 9:73-79). Single-stranded DNA fragments of sample and control FTHMA-070 or T85 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys* Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) *Proc*. *Natl Acad. Sci* USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) *Tibtech* 11:238). In addition, it may be desirable to introduce a novel restriction site in the region of the mutation to create cleavage-based detection (Gasparini et' al. (1992) *Mol.* Cell *Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving a FTHMA-070 or T85 gene.

Furthermore, any cell type or tissue, preferably peripheral blood leukocytes, in which FTHMA-070 or T85 is expressed may be utilized in the prognostic assays described herein.

### 3. Pharmacogenomics

Agents, or modulators which have a stimulatory or inhibitory effect on FTHMA-070 or T85 activity (e.g., FTHMA-070 or T85 gene expression) as identified by a screening assay described herein can be administered to individuals to treat (prophylactically or therapeutically) disorders (e.g., an immunological disorder) associated with aberrant FTHMA-070 or T85 activity. In conjunction with such treatment, the pharmacogenomics (i.e., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of FTHMA-070 or T85 protein, expression of FTHMA-070 or T85 nucleic acid, or mutation content of FTHMA-070 or T85 genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See, e.g., Linder (1997) *Clin. Chem.* 43(2):254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of FTHMA-070 or T85 protein, expression of FTHMA-070 or T85 nucleic acid, or mutation content of FTHMA-070 or T85 genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a FTHMA-070 or TB5 modulator, such as a modulator identified by one of the exemplary screening assays described herein.

### 4. Monitoring of Effects During Clinical Trials

Monitoring the influence of agents (e.g., drugs, compounds) on the expression or activity of FTHMA-070 or T85 (e.g., the ability to modulate aberrant cell proliferation and/or differentiation) can be applied not only in basic drug screening, but also in clinical trials. For example, the effectiveness of an agent determined by a screening assay as described herein to increase FTHMA-070 or T85 gene expression, protein levels, or upregulate FTHMA-070 or T85 activity, can be monitored in clinical trails of subjects exhibiting decreased FTHMA-070 or T85 gene expression, protein levels, or downregulated FTHMA-070 or T85 activity. Alternatively, the effectiveness of an agent determined by a screening assay to decrease FTHMA-070 or T85 gene expression, protein levels, or downregulated FTHMA-070 or T85 activity, can be monitored .in clinical trails of subjects exhibiting increased FTHMA-070 or T85 gene expression, protein levels, or upregulated FTHMA-070 or T85 activity. In such clinical trials, the expression or activity of FTHMA-070 or T85 and, preferably, other genes that have been implicated in, for example, a cellular proliferation disorder can be used as a "read out" or markers of the immune responsiveness of a particular cell.

For example, and not by way of limitation, genes, including FTHMA-070 or T85, that are modulated in cells by treatment with an agent (e.g., compound, drug or small molecule) which modulates FTHMA-070 or T85 activity (e.g., identified in a screening assay as described herein) can be identified. Thus, to study the effect of agents on cellular proliferation disorders, for example, in a clinical trial, cells can be isolated and RNA prepared and analyzed for the levels of expression of FTHMA-070 or T85 and other genes implicated in the disorder. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of FTHMA-070 or T85 or other genes. In this way, the gene expression pattern can serve as a marker, indicative of the physiological response of the cells to the agent. Accordingly, this response state may be determined before, and at various points during, treatment of the individual with the agent.

In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of expression of a FTHMA-070 or T85 protein, mRNA, or genomic DNA in the preadministration sample; (iii) obtaining one or more post-administration samples from the subject; (iv) detecting the level of expression or activity of the FTHMA-070 or T85 protein, mRNA, or genomic DNA in the post-administration samples; (v) comparing the level of expression or activity of the FTHMA-070 or T85 protein, mRNA, or genomic DNA in the pre-administration sample with the FTHMA-070 or T85 protein, mRNA, or genomic DNA in the post administration sample or samples; and (vi) altering the administration of the agent to the subject accordingly. For example, increased administration of the agent may be desirable to increase the expression or activity of FTHMA-070 or T85 to higher levels than detected, i.e., to increase the effectiveness of the agent. Alternatively, decreased administration of the agent may be desirable to decrease expression or activity of FTHMA-070 or T85 to lower levels than detected, i.e., to decrease the effectiveness of the agent.

### C. Methods of Treatment

The present invention provides for both prophylactic and therapeutic methods of treating a subject at risk of (or susceptible to) a disorder or having a disorder associated with aberrant FTHMA-070 or T85 expression or activity.

### 1. Prophylactic Methods

In one aspect, the invention provides a method for preventing in a subject a disease or condition associated with an aberrant FTHMA-070 or T85 expression or activity, by administering to the subject an agent which modulates FTHMA-070 or T85 expression or at least one FTHMA-070 or T85 activity. Subjects at risk for a disease which is caused or contributed to by aberrant FTHMA-070 or T85 expression or activity can be identified by, for example, any or a combination of diagnostic or prognostic assays as described herein. Administration of a prophylactic agent can occur prior to the manifestation of symptoms characteristic of the FTHMA-070 or T85 aberrancy, such that a disease or disorder is prevented or, alternatively, delayed in its progression. Depending on the type of FTHMA-070 or T85 aberrancy, for example, a FTHMA-070 or T85 agonist or FTHMA-070 or T85 antagonist agent can be used for treating the subject. The appropriate agent can be determined based on screening assays described herein.

### 2. Therapeutic Methods

Another aspect of the invention pertains to methods of modulating FTHMA-070 or T85 expression or activity for therapeutic purposes. The modulatory method of the invention involves contacting a cell with an agent that modulates one or more of the activities of FTHMA-070 or T85 protein activity associated with the cell. An agent that modulates FTHMA-070 or T85 protein activity can be an agent as described herein, such as a nucleic acid or a protein, a naturally-occurring cognate ligand of a FTHMA-070 or T85 protein, a peptide, a FTHMA-070 or T85 peptidomimetic, or other small molecule. In one embodiment, the agent stimulates one or more of the biological activities of FTHMA-070 or T85 protein. Examples of such stimulatory agents include active FTHMA-070 or T85 protein and a nucleic acid molecule encoding FTHMA-070 or T85 that has been introduced into the cell. In another embodiment, the agent inhibits one or more of the biological activities of FTHMA-070 or T85 protein. Examples of such inhibitory agents include antisense FTHMA-070 or T85 nucleic acid molecules and anti-FTHMA-070 or T85 antibodies. These modulatory methods can be performed in vitro (e.g., by culturing the cell with the agent) or, alternatively, *in vivo* (e.g, by administering the agent to a subject). As such, the present invention provides methods of treating an individual afflicted with a disease or disorder characterized by aberrant expression or activity of a FTHMA-070 or TB5 protein or nucleic acid molecule. In one embodiment, the method involves administering an agent (e.g., an agent identified by a screening assay described herein), or combination of agents that modulates (e.g., upregulates or downregulates) FTHMA-070 or T85 expression or activity. In another embodiment, the method involves administering a FTHMA-070 or T85 protein or nucleic acid molecule as therapy to compensate for reduced or aberrant FTHMA-070 or T85 expression or activity.

Stimulation of FTHMA-070 or T85 activity is desirable in situations in which FTHMA-070 or T85 is abnormally downregulated and/or in which increased FTHMA-070 or T85 activity is likely to have a beneficial effect. Conversely, inhibition of FTHMA-070 or T85 activity is desirable in situations in which FTHMA-070 or T85 is abnormally upregulated and/or in which decreased FTHMA-070 or T85 activity is likely to have a beneficial effect.

This invention is further illustrated by the following examples which should not be construed as limiting. The contents of all references, patents and published patent applications cited throughout this application are hereby incorporated by reference.

### EXAMPLES

### Example 1: Isolation and Characterization of Human FTHMA-070 cDNAs

The nucleic acid molecule encoding FTMA-070 was identified during the sequencing of clones present in a cardiac coronary artery smooth muscle cell library. A clone was identified which appeared to have some homology to TNF receptor. This clone proved to encode FTHMA-070. The nucleic acid sequence (SEQ ID NO:1) and deduced amino acid sequence (SEQ ID NO:2) of FTHMA-070, which has homology to tumor necrosis factor receptor (including the death domain) are shown in Figure 1.

### Example 2: Characterization of FTHMA-070 Proteins

The human FTHMA-070 cDNA isolated as described above (Figure 1; SEQ ID NO:1) encodes a 401 amino acid protein (Figure 1; SEQ ID NO:2). FTHMA-070 is predicted to include a 21 amino acid signal peptide (amino acid 1 to about amino acid 21 of SEQ ID NO:2) preceding the 380 mature protein (about amino acid 22 to amino acid 401; SEQ ID NO:4).

### Example 3: Preparation of FTHMA-070 Proteins

Recombinant FTHMA-070 can be produced in a variety of expression systems. For example, the mature FTHMA-070 peptide can be expressed as a recombinant glutathione-S-transferase (GST) fusion protein in **E.** *coli* and the fusion protein can be isolated and characterized. Specifically, as described above, FTHMA-070 can be fused to GST and this fusion protein can be expressed in *E*. *coli* strain PEB199. Expression of the GST-FTHMA-070 fusion protein in PEB199 can be induced with IPTG. The recombinant fusion protein can be purified from crude bacterial lysates of the induced PEB199 strain by affinity chromatography on glutathione beads.

### Example 4: Isolation and Characterization of Human FTHMA-070 cDNAs

The nucleic acid molecule encoding T85 (originally called FMHB-6D4 and FMHB-SD4) was identified using a screen designed to identify genes encoding proteins having a functional signal sequence. Briefly, a library was prepared in which each of the clones contained a human fetal brain cDNA ligated to a sequence encoding a detectable protein which lacked a signal sequence. If the human fetal cDNA encodes a functional signal sequence, it will permit the secretion and detection of the detecable protein. This clone library was used to transfect mammalian cells. Clones which secreted the detectable protein were then identified and the corresponding human fetal brain cDNA was isolated and sequenced using standard techniques. In this way it was possible to identify a clone encoding T85. The nucleic acid sequence (SEQ ID NO:5) and deduced amino acid sequence (SEQ ID NO:6) of T85 are shown in Figure 3.

### Example 5: Characterization of T85 Proteins

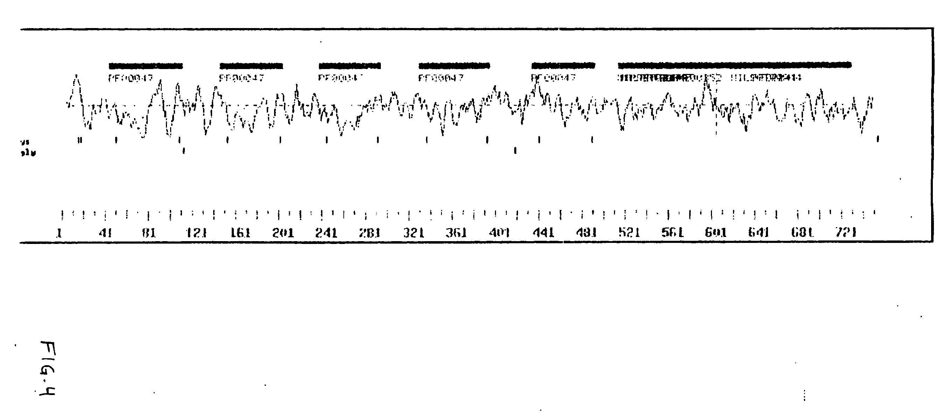

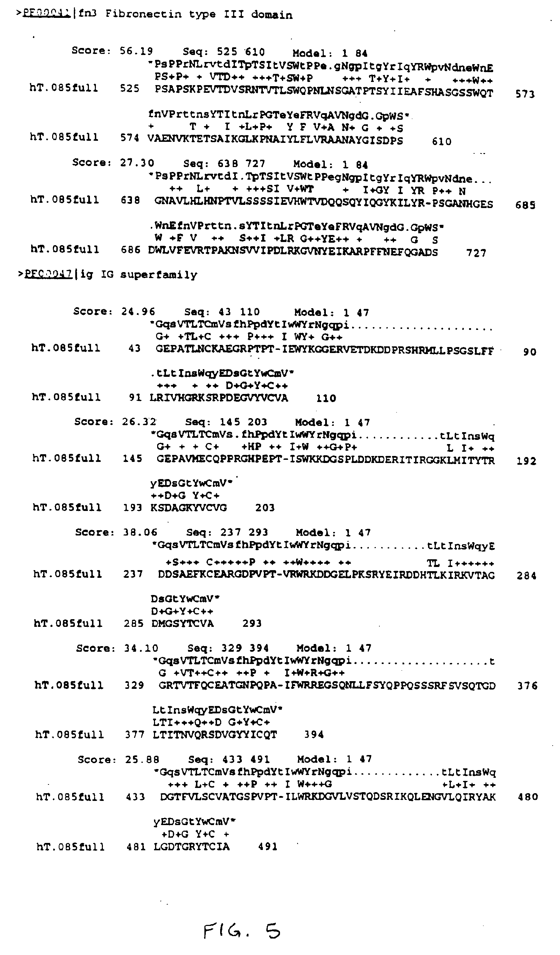

The human T85 cDNA isolated as described above (Figure 3; SEQ ID NO:5) encodes a 753 amino acid protein (Figure 3; SEQ ID NO:6). The signal peptide prediction program SIGNALP (Nielsen et al. (1997) Protein *Engineering* 10:1-6) predicted that T85 includes a 20 amino acid signal peptide (amino acid 1 to about amino acid 20 of SEQ ID NO:6) preceding the 733 amino acid mature protein (about amino acid 21 to amino acid 753 of SEQ ID NO:6; SEQ ID NO:8). A hydropathy plot of T85 is presented in Figure 4. This plot the location of cysteines ("cys"; short vertical lines just below plot) and the most significant PFAM identifiers (PF00047 and PF00041; bars just above plot). For general information regarding PFAM identifiers and Hidden Markov Model (HMM) consensus sequences refer to Sonnhammer et al. (1997) Protein 28:405-420 and http://www.psc.edu/general/software/packages/pfamjpfam.html.

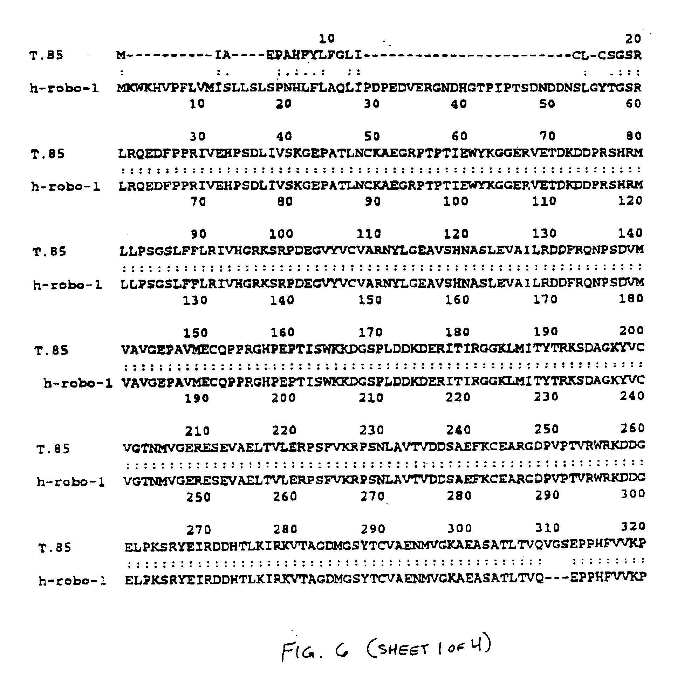

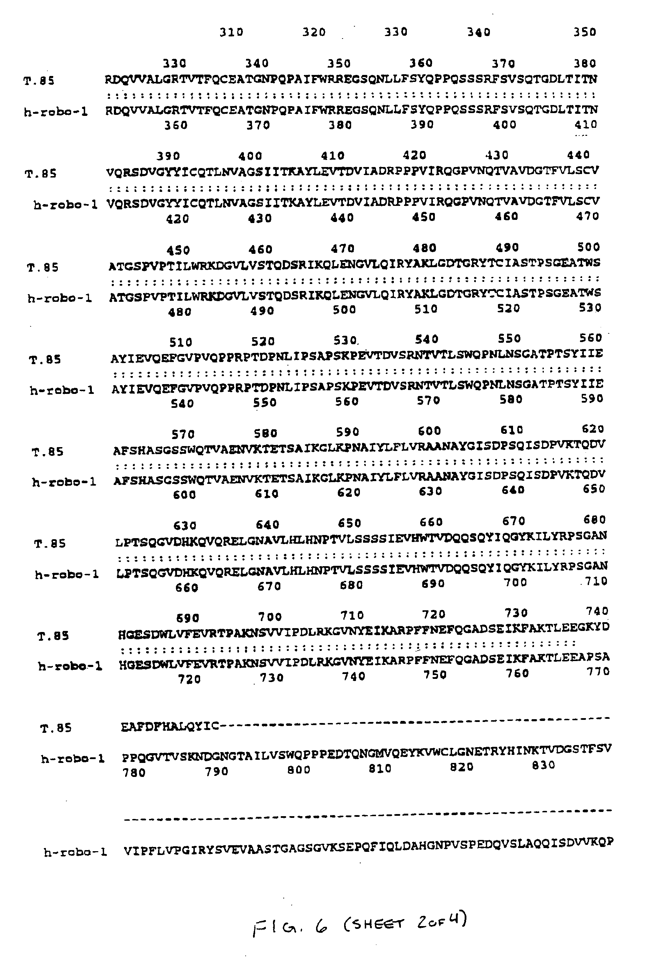

As shown in Figure 5,'T85 has a two regions (amino acids 525 - 610 and 638 - 727 of SEQ ID NO:6; SEQ ID NO:9 and SEQ ID NO:10, respectively) of homology to a fibronectin type III domain (based on HMM PF00041; SEQ ID NO-18).

Also as shown in Figure 5, T85 has a five regions (amino acids 43 - 101; 145 - 203; 237 - 298; 329 - 394; and 433 - 491 of SEQ ID NO:6; SEQ ID NOs:11-15) of homology to a Ig superfamily domain (based on HMM PF00047; SEQ ID NO:19). T85 also includes an RGD motif starting at amino acid 247 of SEQ ID NO:6; a cytokine receptor homolgy N-terminal (BC) domain (CC-CC) at amino acids 516 - 600 of SEQ ID NO:6.

### Example 6: Preparation of T85 Proteins

Recombinant T85 can be produced in a variety of expression systems. For example, the mature T85 peptide can be expressed as a recombinant glutathione-S-transferase (GST) fusion protein in *E*. *coli* and the fusion protein can be isolated and characterized. Specifically, as described above, FTHMA-070 can be fused to GST and this fusion protein can be expressed in *E. coli* strain PEB199. Expression of the GST-T85 fusion protein in PEB199 can be induced with IPTG. The recombinant fusion protein can be purified from crude bacterial lysates of the induced PEB199 strain by affinity chromatography on glutathione beads.

### Example 7

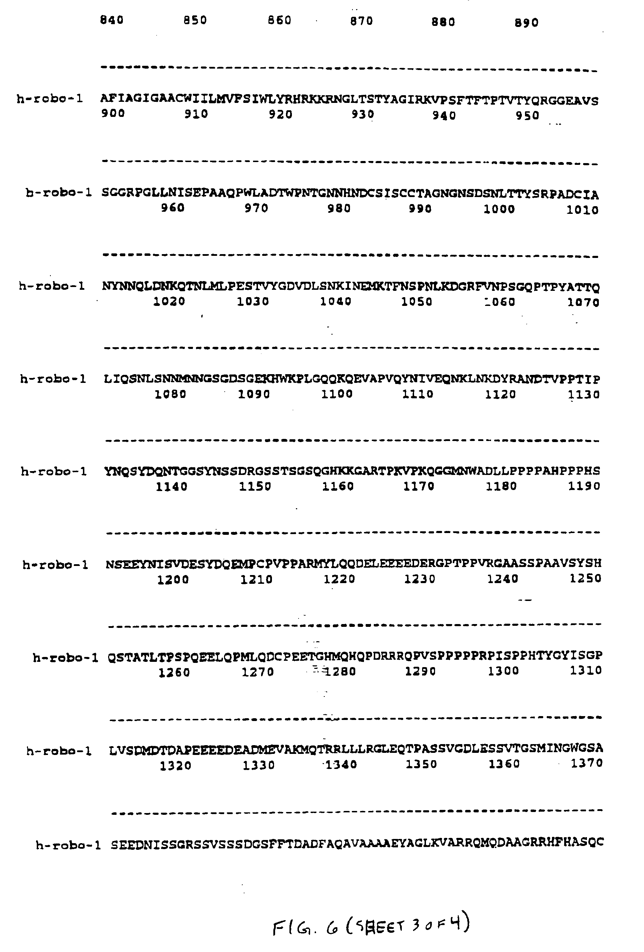

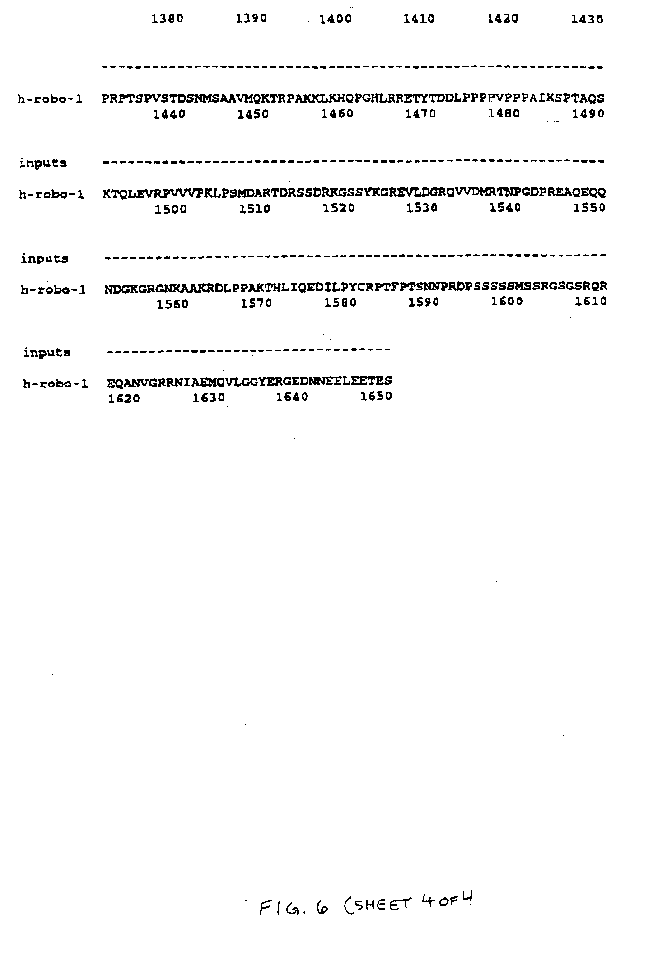

As shown in Figure 6, T85 exhibits considerable homology to human Robo protein (Kidd et al. (1997) Cell 92:205-215), an axon guidance receptor that is thought to play and important role in neuronal development, specifically, control of midline crossing. Accordingly, T85 may also play a role in neuronal development. Thus, T85 nucleic acids, polpeptides, T85 agonists, and T85 antagonists may be useful in the treatment of neurological disorders.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a) a nucleic acid molecule comprising a nucleotide sequence which is at least 55 % identical to the nucleotide sequence of SEQ ID NO: 5 or SEQ ID NO: 7, or a complement thereof;
b) a nucleic acid molecule comprising a fragment of at least 300 nucleotides of the nucleotide sequence of SEQ 11) NO: 5 or SEQ ID NO: 7, or a complement thereof;
c) nucleic acid molecule which encodes a polypeptide comprising the amino acid sequence of SEQ ID NO:6 or SEQ ID NO:8;
d) a nucleic acid molecule which encodes a fragment of a polypeptide comprising the amino acid sequence of SEQ ID NO: 6 or SEQ ID NO: 8, wherein the fragment comprises at least 15 contiguous amino acids of SEQ ID NO: 6 or SEQ ID NO: 8; and
e) a nucleic acid molecule which encodes a naturally occurring allelic variant of a polypeptide comprising the amino acid sequence of SEQ ID NO:6 or SEQ ID NO:8, wherein the nucleic acid molecule hybridizes to a nucleic acid molecule comprising SEQ ID NO:5 or SEQ ID NO:7 under stringent conditions.

2. The isolated nucleic acid molecule of claim 1, which is selected from the group consisting of:
a) a nucleic acid comprising the nucleotide sequence of SEQ ID NO:5 or SEQ ID NO:7; or a complement thereof; and
b) a nucleic acid molecule which encodes a polypeptide comprising the amino acid sequence of SEQ ID NO: 6 or SEQ ID NO: 8.

3. The nucleic acid molecule of claim 1 or 2 further comprising vector nucleic acid sequences.

4. The nucleic acid molecule of any of claims 1 to 3 further comprising nucleic acid sequences encoding a heterologous polypeptide.

5. A host cell (eg a mammalian host cell) which contains the nucleic acid molecule of any of claims 1 to 4.

6. A non-human mammalian host cell containing the nucleic acid molecule of any of claims 1 to 4.

7. An isolated polypeptide selected from the group consisting of:
a) a fragment of a polypeptide comprising the amino acid sequence of SEQ ID NO:6 or SEQ ID NO:8, wherein the fragment comprises at least 15 contiguous amino acids of SEQ ID NO:6 or SEQ ID NO:8;
b) a naturally occurring allelic variant of a polypeptide comprising the amino- acid sequence of SEQ ID NO:6 or SEQ ID NO:8, wherein the polypeptide is encoded by a nucleic acid molecule which hybridizes to a nucleic acid molecule comprising SEQ ID NO:5 or SEQ ID NO:7 under stringent conditions;
c) a polypeptide which is encoded by a nucleic acid molecule comprising a nucleotide sequence which is at least 55 % identical to a nucleic acid comprising the nucleotide sequence of SEQ ID NO:5 or SEQ ID NO:7.

8. The isolated polypeptide of claim 7 comprising the amino acid sequence of SEQ m NO:6 or SEQ ID NO:8.

9. The polypeptide of claim 7 or 8 further comprising heterologous amino acid sequences.

10. An antibody which selectively binds to a polypeptide of any of claims 7 to 9.

11. A method for producing a polypeptide selected from the group consisting of:
a) a polypeptide comprising the amino acid sequence of SEQ ID NO:6 or SEQ ID NO:8;
b) a fragment of a polypeptide comprising the amino acid sequence of SEQ ID NO:6 or SEQ ID NO:8,
wherein the fragment comprises at least 15 contiguous amino acids of SEQ ID NO: 6 or SEQ ID NO: 8; and
c) a naturally occurring allelic variant of a polypeptide comprising the amino acid sequence of SEQ ID NO:6 or SEQ ID NO:8, wherein the polypeptide is encoded by a nucleic acid molecule which hybridizes to a nucleic acid molecule comprising SEQ ID NO:5 or SEQ ID NO:7 under stringent conditions;
comprising culturing the host cell of claim 5 or claim 6 under conditions in which the nucleic acid molecule is expressed.

12. The isolated polypeptide of claim 7 comprising the amino acid sequence of SEQ ID NO:6 or SEQ ID NO: 8.

13. A method for detecting the presence of a polypeptide of any of claims 7 to 9 in a sample, comprising:
a) contacting the sample with a compound (eg an antibody) which selectively binds to said polypeptide; and
b) determining whether the compound binds to the polypeptide in the sample.

14. A kit comprising a compound which selectively binds to a polypeptide of any of claims 7 to 9 and instructions for use.

15. A method for detecting the presence of a nucleic acid molecule of any of claims 7 to 9 in a sample (eg a sample comprising RNA molecules), comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

16. A kit comprising a compound which selectively hybridizes to a nucleic acid molecule of claim 1 and instructions for use.

17. A method for identifying a compound which binds to a polypeptide of any of claims 7 to 9 comprising the steps of:
a) contacting a polypeptide, or a cell expressing said polypeptide with a test compound; and
b) determining whether the polypeptide binds to the test compound (eg wherein the binding of the test compound to the polypeptide is detected by a method selected from the group consisting of:
a) detection of binding by direct detecting of test compound/polypeptide binding; and
b) detection of binding using a competition binding assay.)

18. A method for modulating the activity of a polypeptide of any of claims 7 to 9 comprising contacting a polypeptide or a cell expressing a polypeptide of claim 12 with a compound which binds to the polypeptide in a sufficient concentration to modulate the activity of the polypeptide.

19. A method for identifying a compound which modulates the activity of a polypeptide of any of claims 7 to 9, comprising:
a) contacting a polypeptide of any of claims 7 to 9 with a test compound; and
b) determining the effect of the test compound on the activity of the polypeptide to thereby identify a compound which modulates the activity of the polypeptide.
